# EUROPEAN PATENT APPLICATION

(11) **EP 4 847 627 A2**
(43) Date of publication of application: **02.12.2026**
(21) Application number: 26205024.8
(22) Date of filing: 27.06.2023
(51) Int. Cl.: C12Q 1/00

(54) **SENSOR AND METHOD FOR MANUFACTURING SAME**

(30) Priority: 01.07.2022 JP 2022107320; 21.07.2022 JP 2022116287
(62) Document number(s) of the earlier application(s) in accordance with Art. 76 EPC: 23831465.2 / 4 549 921
(71) Applicant: PHC HOLDINGS CORPORATION, Tokyo 100-8403 (JP)
(72) Inventor: NORIKANE, Tetsuya, Ehime, 791-0395 (JP); HANEDA, Keigo, Ehime, 791-0395 (JP); NAKANO, Tarou, Ehime, 791-0395 (JP); HATAKEYAMA, Takeshi, Ehime, 791-0395 (JP)
(74) Representative: Eisenführ Speiser

(57) **Abstract**

The present invention provides a sensor, with which the inhibition of contact between a liquid sample and a detection electrode of the sensor when the sensor is immersed in the liquid sample less likely to occur. A sensor (1101) comprises an insulating substrate (1200) that is immersed in the liquid sample; detection electrodes (1300) disposed on the substrate (1200), the detection electrodes (1300) including a working electrode (1310), a reference electrode (1320), and a counter electrode (1330); wiring (1005); an insulating layer (1050); a distal end opening (1061) that is formed in the insulating layer (1050); and a flow path (1062) that is formed in the insulating layer (1050), connects the distal end opening (1061) and the upper surface (1331) of the counter electrode (1330), and guides the liquid sample from the distal end opening (1061) to the upper surface (1331) of the counter electrode (1330).

## Description

### TECHNICAL FIELD

The second disclosure relates, for example, to a sensor and a sensor unit.

### BACKGROUND ART

With the configuration of a conventional cell culture analysis device comprising a sensor for analyzing a cell culture liquid, the sensor is fixed to a through-hole portion provided in a base material, and a lead wire for taking off a signal is connected to this sensor.

For example, Patent Literature 2 (US Pat. No. 9,170,255) discloses a cell culture analysis device having a cartridge that mates with a plate on which a plurality of cell culture vessels are provided. The cell culture analysis device in Patent Literature 2 has a sensor for measuring the inside of each culture vessel, a cartridge is provided with a plurality of openings into which these sensors are inserted, and the sensors are connected to fiber cables within these openings. These fiber cables are connected to an external control unit.

### CITATION LIST

### PATENT LITERATURE

U.S. Patent No. 9,014,774
U.S. Patent No. 9,170,255

### SUMMARY

### TECHNICAL PROBLEM

The following problem is encountered with the sensor of the cell culture analysis device described in Patent Literature 2.

With the cell culture analysis device disclosed in Patent Literature 2, it is difficult to accurately position each of the sensors inserted into the plurality of culture vessels with respect to the culture vessels.

This may cause variations in the depth to which the sensor is immersed in the culture medium held in the culture vessel, which may reduce measurement accuracy. In particular, if the sensor is not immersed deep enough in the culture medium, the liquid sample may not come into sufficient contact with the detection electrodes of the sensor, and this may reduce measurement accuracy.

It is an object of the second disclosure to provide a sensor, and a sensor unit comprising this sensor, with which the inhibition of contact between a liquid sample and a detection electrode of the sensor when the sensor is immersed in the liquid sample less likely to occur.

### SOLUTION TO PROBLEM

One aspect of the second disclosure is directed to a sensor that is used in a state of being immersed in a liquid sample, comprising:
an insulating substrate including a distal end portion that is immersed in the liquid sample;
detection electrodes disposed near the distal end portion on a first surface of the substrate, the detection electrodes including a working electrode, a counter electrode, and a reference electrode;
wiring that is disposed on the first surface of the substrate and is connected to the detection electrodes;
an insulating layer that is disposed on the first surface of the substrate and is formed so as to cover at least part of the detection electrodes and the wiring;
a distal end opening that is formed in the insulating layer, communicates with an upper surface of the counter electrode, and opens to the side of the substrate with the distal end portion; and
a flow path that is formed in the insulating layer, connects the distal end opening and the upper surface of the counter electrode, and guides the liquid sample from the distal end opening to the upper surface of the counter electrode.

In a specific aspect of the sensor according to the second disclosure,
the insulating layer comprises a counter electrode opening that is formed at a position overlapping the counter electrode in plan view from the thickness direction of the substrate, and goes through in the thickness direction, and
the upper surface of the counter electrode is exposed through the counter electrode opening.

In a further specific aspect of the sensor according to the second disclosure,
the insulating layer comprises, in plan view from the thickness direction of the substrate:
a working electrode opening that is formed at a position overlapping the working electrode and goes through in the thickness direction; and
a reference electrode opening that is formed at a position overlapping the reference electrode and goes through in the thickness direction,
the working electrode includes a working electrode protective film disposed in the working electrode opening, and
the reference electrode includes a reference electrode protective film disposed in the reference electrode opening.

In a more specific aspect of the sensor according to the second disclosure,
the insulating layer includes:
a first insulating layer that is disposed on the first surface of the substrate and contains an insulating cured resin; and
a second insulating layer that is disposed on the first insulating layer and includes an insulating sheet, and
the distal end opening and the flow path are formed in the second insulating layer.

Another aspect of the second disclosure is directed to a sensor unit, comprising:
the sensor according to the second disclosure; and
a support member equipped with an engagement portion that engages with the substrate of the sensor and supports the sensor so that the distal end portion of the substrate is the lower end.

### ADVANTAGEOUS EFFECTS

With the sensor, and the sensor unit according to the second disclosure, the inhibition of contact between a liquid sample and a detection electrode of the sensor when the sensor is immersed in the liquid sample less likely to occur.

### BRIEF DESCRIPTION OF DRAWINGS

Fig. 1 is a plan view of a substrate on which wiring and conductive layers of a first working electrode, a second working electrode, a reference electrode, and a counter electrode are disposed;
Fig. 2 is a plan view of a substrate on which wiring and conductive layers of a first working electrode, a second working electrode, a reference electrode, and a counter electrode are disposed, with a first insulating layer being further disposed over these;
Fig. 3 is a plan view of a substrate on which wiring and conductive layers of a first working electrode, a second working electrode, a reference electrode, and a counter electrode are disposed, with a first insulating layer and a second insulating layer being further disposed over these;
Fig. 4 is a plan view of the substrate in Fig. 3 on which are further disposed a reagent layer of a working electrode and a silver/silver chloride layer of a reference electrode;
Fig. 5 is a plan view of the sensor according to an embodiment of the first disclosure;
Fig. 6 is a cross-sectional view along the A-A' line of the portion of the substrate in Fig. 3 corresponding to the first working electrode;
Fig. 7 is a cross-sectional view of a droplet of liquid composition A containing a reagent that participates in an oxidation-reduction reaction in water, which was formed in a first opening of a first insulating layer of a first working electrode of the substrate in Fig. 3;
Fig. 8 is a cross-sectional view along the B-B' line of the portion of the substrate in Fig. 4 corresponding to the first working electrode, which was obtained by drying the droplet of the liquid composition A in Fig. 3;
Fig. 9 is a cross-sectional view of a droplet of a first liquid composition B containing a protective film component in alcohol, which was formed in a second opening of a second insulating layer of the first working electrode of the substrate in Fig. 4;
Fig. 10 is a cross-sectional view of the portion of the protective film within the second opening of the second insulating layer of the first working electrode of the substrate in Fig. 4, which was obtained by drying the droplet of the first liquid composition B shown in Fig. 9;
Fig. 11 is a cross-sectional view of a droplet of the first liquid composition B, which was further formed within the second opening of the second insulating layer of the first working electrode, and includes a portion of the protective film in Fig. 10;
Fig. 12 is a cross-sectional view along the C-C' line of a portion of the sensor in Fig. 5 including the first working electrode, which includes the first protective film obtained by drying the droplet of the first liquid composition B in Fig. 11;
Fig. 13 is a cross-sectional view of a droplet of the second liquid composition B containing a protective film component in alcohol, which was formed in the second opening of the second insulating layer of the second working electrode of the substrate in Fig. 4;
Fig. 14 is a cross-sectional view of the second protective film in the second opening of the second insulating layer of the second working electrode of the substrate in Fig. 4, which was obtained by drying a droplet of the second liquid composition B in Fig. 9;
Fig. 15 is a cross-sectional view of a droplet of a third liquid composition B containing another protective film component in alcohol, which was formed in the second opening of the second insulating layer of the second working electrode, and includes the second protective film in Fig. 14;
Fig. 16 is a cross-sectional view along the D-D' line of the portion of the sensor in Fig. 5 that includes the second working electrode, which includes a third protective film obtained by drying a droplet of the third liquid composition B in Fig. 15;
Fig. 17 is a cross-sectional view of a comparative sensor, showing a state in which a droplet of the first liquid composition B containing a protective film component in alcohol, which was formed in the second opening of the second insulating layer of the first working electrode, has spread out and wetted the surface;
Fig. 18 is a cross-sectional view of a portion of the first protective film formed on the upper surface of the second insulating layer and in the second opening of the first working electrode, which was obtained by drying the spread-out first liquid composition B in Fig. 17;
Fig. 19 is a cross-sectional view of a state in which a droplet of the first liquid composition B, which was further formed in the second opening of the second insulating layer of the first working electrode of the sensor in a comparative example, which includes a part of the first protective film in Fig. 18, has wetted and spread out over the surface;
Fig. 20 is a cross-sectional view of the first protective film formed on the upper surface of the second insulating layer of the first working electrode and in the second opening, which was obtained by drying the spread-out first liquid composition B in Fig. 19;
Fig. 21 is a schematic diagram illustrating a method for measuring an analyte by immersing the sensor of Fig. 5 in a liquid sample;
Fig. 22 is a cross-sectional view of the portion of the sensor in Fig. 5 that includes the reference electrode, along the E-E' line;
Fig. 23 is a cross-sectional view of the portion of the sensor in Fig. 5 that includes the counter electrode, along the F-F' line;
Fig. 24 is a control block diagram of an analysis device including the sensor according to an embodiment of the first disclosure;
Fig. 25A shows the results of measuring the current value for the first working electrode (for measuring glucose) of the sensor in a working example, and Fig. 25B shows the results of measuring the current value for the first working electrode (for measuring glucose) in a comparative example;
Fig. 26A shows the results of measuring the current value for the second working electrode (for measuring lactic acid) of the sensor in a working example, and Fig. 26B shows the results of measuring the current value for the second working electrode (for measuring lactic acid) of the sensor in a comparative example;
Fig. 27 is a plan view of a substrate on which a working electrode conductive layer, a reference electrode conductive layer, a counter electrode, and wiring are disposed;
Fig. 28 is a plan view of a substrate on which a working electrode conductive layer, a reference electrode conductive layer, a counter electrode, and wiring are disposed, and a first insulating layer is further disposed over these;
Fig. 29 is a plan view of a substrate on which a working electrode conductive layer, a reference electrode conductive layer, a counter electrode, and wiring are disposed, and a first insulating layer and a second insulating layer are further disposed over these;
Fig. 30 is a plan view of a substrate on which a reagent layer of a working electrode and a silver/silver chloride layer of a reference electrode are further disposed on the substrate in Fig. 29;
Fig. 31 is a plan view of a sensor according to an embodiment of the second disclosure, in which a distal end opening and a flow channel are formed in the second insulating layer;
Fig. 32 is a plan view of a sensor according to another embodiment of the second disclosure, in which a distal end opening and a flow channel are formed in the first insulating layer and the second insulating layer;
Fig. 33 is a cross-sectional view along the J-J' line of the portion of the sensor in Fig. 31 that includes the working electrode;
Fig. 34 is a cross-sectional view along the K-K' line of the portion of the sensor in Fig. 31 that includes the reference electrode;
Fig. 35 a cross-sectional view of the portion of the sensor in Fig. 31 that includes the counter electrode, along the L-L' line;
Fig. 36 is a cross-sectional view along the M-M' line of the portion of the sensor in Fig. 31 that includes the counter electrode and the distal end opening and flow channel formed in the second insulating layer;
Fig. 37 is a cross-sectional view of a portion of a sensor according to yet another embodiment, which includes a counter electrode, a distal end opening, and a flow path, in which the distal end opening and flow path are formed in a portion of the thickness direction not including the top surface of the insulating layer, and have a structure in which the periphery is enclosed;
Fig. 38 is a cross-sectional view along the N-N' line, of the portion of the sensor in Fig. 32 that includes the counter electrode and the distal end opening and flow channel formed in the first insulating layer and the second insulating layer;
Fig. 39 is a schematic diagram of a cross section of the portion of the sensor in Figs. 31 and 36 that includes the counter electrode, the distal end opening, and the flow channel, which is immersed in a liquid sample so that the distal end portion of the substrate is at the lower end;
Fig. 40 is a schematic diagram of a cross section of the portion of a comparative sensor that includes the counter electrode having no distal end opening and no flow channel, which is immersed in a liquid sample so that the distal end portion of the substrate is at the lower end;
Fig. 41 is a plan view of a sensor according to an embodiment of the second disclosure, comprising a substrate that includes a main body, a connecting portion, and a proximal end portion, a first insulating layer disposed over the entire first surface of the substrate, and a second insulating layer that includes an insulating sheet disposed on the first surface of the substrate closer to the distal end portion than a bent portion;
Fig. 42 is a plan view of a multiple sensor in which a plurality of the sensors in Fig. 41 are coupled at the proximal end portion of the substrate (the insulating layer is not shown in Fig. 42);
Fig. 43 is a schematic diagram of the sensor in Fig. 41, in which the main body of the substrate, on which a detection electrode is disposed and which is bent at the bent portion of the connecting portion of the substrate, is immersed in a liquid sample (the insulating layer is not shown in Fig. 42);
Fig. 44 is a schematic side view of the sensor in Fig. 41, in which the main body of the substrate on which the detection electrode is disposed is bent at the bent portion of the connecting portion of the substrate and immersed in a liquid sample;
Fig. 45 is a schematic side view of a comparative sensor, in which a second insulating layer including an insulating sheet is disposed over the entire first surface of the substrate in the sensor of Fig. 41, and the main body of the substrate on which the detection electrode is disposed is bent at the bent portion of the connecting portion of the substrate and immersed in a liquid sample;

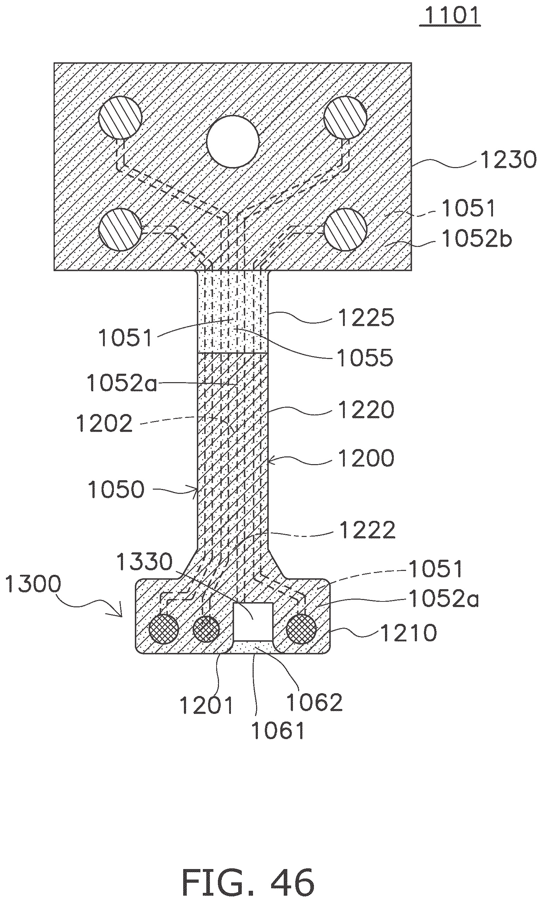

Fig. 46 is a plan view of a sensor according to yet another embodiment of the second disclosure, comprising a substrate that includes a main body, a connecting portion, and a proximal end portion, a first insulating layer that is disposed over the entire first surface of the substrate, a second insulating layer that includes a first insulating sheet disposed on the first surface of the substrate closer to the distal end side than the bent portion, and a second insulating layer that includes a second insulating sheet disposed on the first surface of the proximal end portion of the substrate, and does not include an insulating sheet on the bent portion of the substrate;
Fig. 47 is a plan view of a sensor according to yet another embodiment of the second disclosure, in which a second insulating layer that includes a plurality of third insulating sheets separated from the first insulating sheet and the second insulating sheet by cut portions, is further disposed in the region on the bent portion of the first surface of the substrate in the sensor shown in Fig. 46;
Fig. 48 is a control block diagram of an analysis device that includes the sensor according to an embodiment of the second disclosure;
Fig. 49A is a plan view of a sensor S1 in a comparative example;
Fig. 49B is a plan view of a sensor S2 in a comparative example;
Fig. 49C is a plan view of a sensor S3 in an embodiment;
Fig. 49D is a plan view of a sensor S4 in an embodiment;
Fig. 49E is a plan view of a sensor S5 in an embodiment;
Fig. 50A shows the measurement results for the current value at the working electrode of the sensor S1;
Fig. 50B shows the measurement results for the current value at the working electrode of the sensor S2;
Fig. 50C shows the measurement results for the current value at the working electrode of the sensor S3;
Fig. 50D shows the measurement results for the current value at the working electrode of the sensor S4;
Fig. 50E shows the measurement results for the current value at the working electrode of the sensor S5;
Fig. 51 is an exploded oblique view of a sensor unit according to an embodiment of the second disclosure;
Fig. 52 is a cross-sectional view of a sensor held between an upper support plate and a lower support plate;
Fig. 53 is an exploded oblique view of an adapter unit including a sensor unit;
Fig. 54 is a plan view of a sensor according to an embodiment of the third disclosure;
Fig. 55 is a cross-sectional view along the P-P' line of a portion of the sensor shown in Fig. 54, including the counter electrode;
Fig. 56 is a cross-sectional view along the Q-Q' line of the portion of the sensor shown in Fig. 54 that includes the first working electrode;
Fig. 57 is a cross-sectional view along the R-R' line of the portion of the sensor shown in Fig. 54 that includes a second working electrode;
Fig. 58 shows the results of a cyclic voltammetry test in experiment 4 of the third disclosure, in which either a carbon electrode containing no platinum particles (experiment 3-1), a carbon electrode containing 1% platinum particles (experiment 3-2), or a carbon electrode containing 5% platinum particles (experiment 3-3) was used as the working electrode;
Fig. 59 shows the results of measuring the current value over time when a potential of 0.3 V or -0.2 V was applied to either a carbon electrode containing no platinum particles (A) or a carbon electrode containing 1% platinum particles (B) as the working electrode in experiment 5 of the third disclosure;

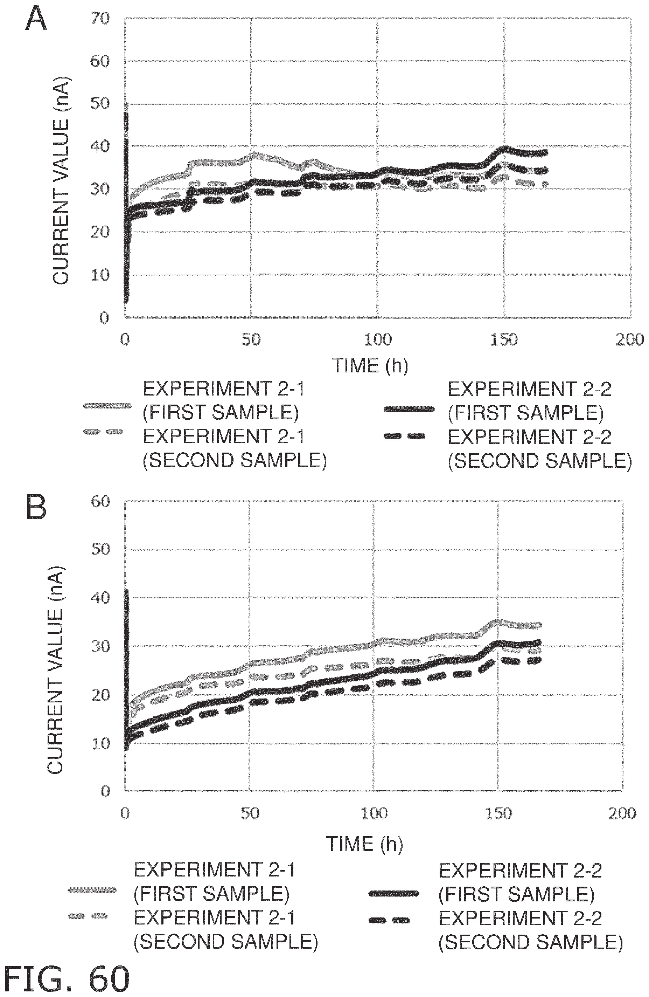

Fig. 60 shows the results of measuring a current value (A) corresponding to the glucose concentration and a current value (B) corresponding to the lactic acid concentration in a liquid sample over time (N = 2), using the sensor of experiment 2-1 (in which the working electrode conductive layer and counter electrode were carbon conductive layers containing no platinum particles) and the sensor of experiment 2-2 (in which the counter electrode was a carbon conductive layer modified with platinum nanoparticles on the surface, and the working electrode conductive layer was a carbon conductive layer containing no platinum particles) in experiment 6 of the third disclosure;

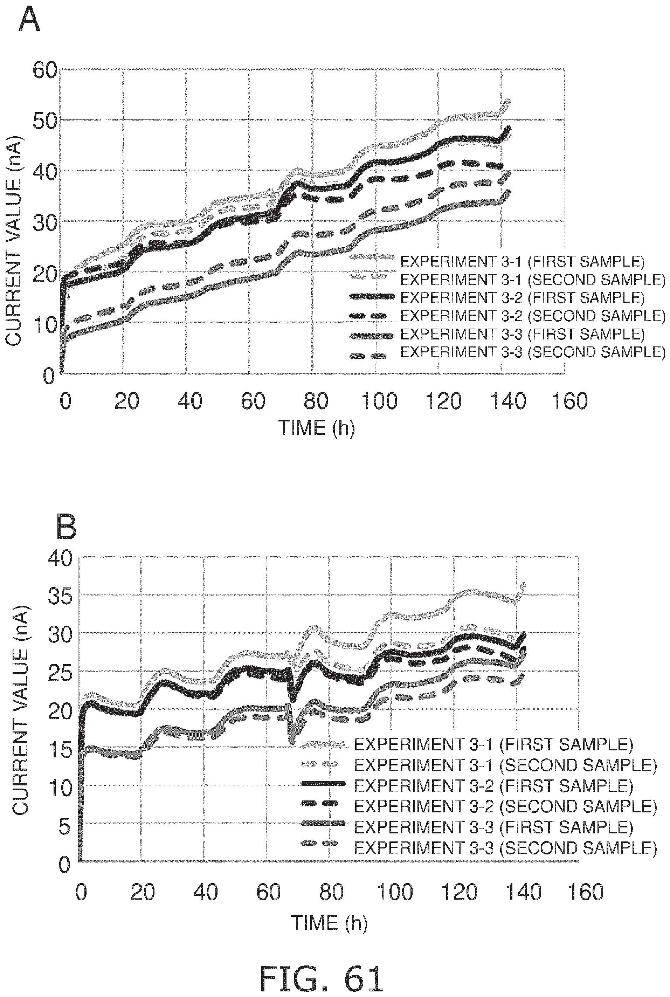

Fig. 61 shows the results of measuring a current value (A) corresponding to the glucose concentration and a current value (B) corresponding to the lactic acid concentration in a liquid sample over time (N = 2), using the sensor of experiment 3-1 (in which the working electrode conductive layer and counter electrode were carbon conductive layers containing no platinum particles), the sensor of experiment 3-2 (in which the working electrode conductive layer and counter electrode were carbon conductive layers containing 1 wt% platinum particles relative to the carbon), and the sensor of experiment 3-3 (in which the working electrode conductive layer and counter electrode were carbon conductive layers containing 5 wt% platinum particles relative to the carbon) in experiment 7 of the third disclosure;
Fig. 62 is a plan view of a sensor according to an embodiment of the fourth disclosure;
Fig. 63 is a cross-sectional view along the S-S' line of the portion of the sensor shown in Fig. 62 that includes the counter electrode;
Fig. 64 is a cross-sectional view along the T-T' line of the portion of the sensor shown in Fig. 62 that includes the working electrode;
Fig. 65 shows the results of experiment 8 of the fourth disclosure, with Fig. 65A showing the measurement results using the sensor of experiment 8-1, and Fig. 65B showing the measurement results using the sensor of experiment 8-2;
Fig. 66 shows the results of experiment 9 of the fourth disclosure, with Fig. 66A showing the measurement results using the sensor of experiment 9-1, Fig. 66B showing the measurement results using the sensor of experiment 9-2, and Fig. 66C showing the measurement results using the sensor of experiment 9-3; and
Fig. 67 shows the results of experiment 10 of the fourth disclosure.

### DESCRIPTION OF EMBODIMENTS

One or more embodiments of the first to fourth disclosures in this specification will be described. In this specification, when a temperature is not specified for a certain method or property, the method or property shall refer to a method or property at room temperature (25°C ±3°C).

This specification encompasses the disclosures of Japanese Patent Application Nos. 2022-107320 and 2022-116287, which serve as the basis for priority in this application.

Also, all publications, patents, and patent applications cited herein are hereby incorporated in their entirety into this specification by their citation.

### Embodiment of First Disclosure

Electrochemical sensors have been used to measure an analyte in a test sample, such as a cell culture fluid or a blood sample. These sensors comprise, for example, an insulating substrate and a working electrode that is disposed on the substrate surface, and further comprise a counter electrode and/or a reference electrode. The working electrode typically comprises a conductive layer and a reagent layer that is disposed on the conductive layer and contains a reagent (such as an oxidoreductase and an electron carrier) that participates in an oxidation-reduction reaction. The sensor may further comprise a protective film that covers only the working electrode, or that covers the working electrode together with the counter electrode and/or the reference electrode, in order to prevent the reagent from flowing out of the reagent layer.

For example, the electrochemical sensor described in Patent Literature 1 comprises a substrate, a first electrode (working electrode) including a sensing layer (reagent layer) and a second electrode (reference electrode) disposed on the substrate, and a flow limiting membrane (protective membrane) including a specific polymer compound that entirely covers these electrodes. Patent Literature 1 indicates that the flow limiting membrane (protective membrane) is formed by dip coating or casting.

However, the following problem is encountered with the above-mentioned conventional sensor comprising a working electrode including a reagent layer and a protective film on a conductive layer.

In Patent Literature 1, an insulating substrate comprising a working electrode including a conductive layer and a reagent layer is immersed in a solution containing a polymer compound, and is then dried to form a protective film that entirely covers the insulating substrate.

With this method, however, it can be difficult to control the characteristics of the protective film on the reagent layer of the working electrode (such as the thickness of the protective film, and the amount of the polymer compound constituting the protective film) within the designed range. Variance in the characteristics of the protective film of the working electrode can lead to a decrease in the measurement accuracy of the sensor.

Also, in Patent Literature 1, a reagent layer containing a reagent such as an enzyme is disposed on the surface of a flat electrode.

However, with a method in which a reagent layer is formed on the surface of a flat electrode, it can be difficult to control the characteristics of the reagent layer (such as the thickness of the reagent layer, and the amount of the reagent constituting the reagent layer) within the designed range. Variance in the characteristics of the reagent layer of the working electrode can also lead to a decrease in the measurement accuracy of the sensor.

It is an object of the first disclosure to provide a sensor comprising a working electrode including a reagent layer and a protective film, with which the characteristics can be easily kept within the desired range, as well as a method for manufacturing this sensor.

In view of this, this specification discloses, as a first disclosure, the sensor and the method for manufacturing of the sensor described in any of the following Supplementary Notes A1 to A15.

### Supplementary Note A1

A sensor comprising an insulating substrate and a working electrode disposed on the substrate,
wherein the working electrode comprises:
a conductive layer that is disposed on the substrate;
a first insulating layer that is at least partially disposed on the conductive layer and that has a water-repellent surface and a first opening formed at a position overlapping part of the conductive layer in a plan view in the thickness direction of the substrate, the first opening passing through in the thickness direction;
a second insulating layer that is disposed on the first insulating layer and that has a surface that is liquid-repellent to alcohol, and a second opening formed at a position overlapping the part of the first insulating layer that entirely encompasses the first opening in a plan view in the thickness direction, the second opening passing through in the thickness direction;
a reagent layer that is disposed within the first opening of the first insulating layer, and includes an outer peripheral edge defined by the inner peripheral edge of the first opening of the first insulating layer, and a reagent that participates in an oxidation-reduction reaction; and
a protective film that is disposed within the second opening of the second insulating layer, and includes an outer peripheral edge defined by the inner peripheral edge of the second opening of the second insulating layer.

### Supplementary Note A2

The sensor according to supplementary note A1,
wherein the surface of the first insulating layer contains a fluororesin.

### Supplementary Note A3

The sensor according to supplementary note A1 or A 2,
wherein the surface of the second insulating layer contains a compound containing a perfluoroalkyl group.

### Supplementary Note A4

The sensor according to any of supplementary notes A1 to A3,
wherein a plurality of the working electrodes are provided, and
a first reagent contained in a first reagent layer of a first working electrode included in the plurality of working electrodes, is different from a second reagent contained in a second reagent layer of a second working electrode different from the first working electrode.

### Supplementary Note A5

The sensor according to supplementary note A4,
wherein the first reagent contains glucose dehydrogenase or glucose oxidase, and
the second reagent contains lactate oxidase or lactate dehydrogenase.

### Supplementary Note A6

The sensor according to any of supplementary notes A1 to A5,
wherein the protective film includes a first protective film containing a polymer compound containing 4-vinylpyridine as a structural unit.

### Supplementary Note A7

The sensor according to any of supplementary notes A1 to A6,
wherein the protective film includes a second protective film disposed on the reagent layer and containing a polymer compound having a cation exchange functional group, and a third protective film disposed on the second protective film and containing a polymer compound having 4-vinylpyridine as a structural unit.

### Supplementary Note A8

The sensor according to any of supplementary notes A1 to A7,
further comprising a counter electrode and a reference electrode disposed on the substrate.

### Supplementary Note A9

A method for manufacturing the sensor according to any of supplementary notes A1 to A8, said method comprising the steps of:
forming a droplet of a liquid composition A containing the reagent in water in the first opening of the first insulating layer of the substrate on which the conductive layer, the first insulating layer, and the second insulating layer are disposed, and then drying this droplet to form the reagent layer; and
after the reagent layer is formed, forming a droplet of a liquid composition B containing a protective film component in alcohol in the second opening of the second insulating layer, and then drying this droplet to form the protective film.

### Supplementary Note A10

The method according to supplementary note A9,
wherein the surface of the first insulating layer contains a fluororesin.

### Supplementary Note A11

The method according to supplementary note A9 or A10,
wherein the surface of the second insulating layer contains a compound containing a perfluoroalkyl group.

### Supplementary Note A12

The method according to any of supplementary notes A9 to A11,
wherein the sensor comprises a plurality of the working electrodes, and
the formation of the reagent layer includes:
   forming a first reagent layer of a first working electrode included in the plurality of working electrodes by forming a droplet of a first liquid composition A containing a first reagent in water in the first opening of the first insulating layer of the first working electrode, and then drying this droplet, and
   forming a second reagent layer of a second working electrode, which is different from the first working electrode included in the plurality of working electrodes, in the first opening of the first insulating layer of the second working electrode by forming a droplet of a second liquid composition A containing a second reagent different from the first reagent in water and then drying this droplet.

### Supplementary Note A13

The method according to supplementary note A12,
wherein the first reagent contains glucose dehydrogenase or glucose oxidase, and
the second reagent contains lactate oxidase or lactate dehydrogenase.

### Supplementary Note A14

The method according to any of supplementary notes A9 to A13,
wherein the protective film includes a first protective film disposed on the reagent layer and containing a polymer compound containing 4-vinylpyridine as a structural unit, and
the formation of the protective film includes:
   after the reagent layer is formed, forming the first protective film in the second opening of the second insulating layer by forming a droplet of a first liquid composition B containing a polymer compound containing 4-vinylpyridine as a structural unit in alcohol, and then drying this droplet.

### Supplementary Note A15

The method according to any of supplementary notes A9 to A14,
wherein the protective film includes a second protective film disposed on the reagent layer and including a polymer compound containing a cation exchange functional group, and a third protective film disposed on the second protective film and containing a polymer compound containing 4-vinylpyridine as a structural unit, and
the formation of the protective film includes:
   after the reagent layer is formed, forming the second protective film in the second opening of the second insulating layer by forming a droplet of a second liquid composition B containing a polymer compound containing the cation exchange functional group in alcohol, and then drying this droplet, and
   after the second protective film is formed, forming the third protective film in the second opening of the second insulating layer of the second working electrode by forming a droplet of a third liquid composition B containing a polymer compound containing 4-vinylpyridine as a structural unit in alcohol, and then drying this droplet.

Embodiments of a sensor and a method for manufacturing a sensor according to the first disclosure will now be described. In these embodiments, some unnecessarily detailed description may be omitted. For example, detailed description of already known facts or redundant description of components that are substantially the same may be omitted. This is to avoid unnecessary repetition in the following description, and facilitate an understanding on the part of a person skilled in the art.

The applicant has provided the appended drawings and the following description so that a person skilled in the art might fully understand the first disclosure, but does not intend for these to limit what is discussed in the patent claims.

### Materials

Examples of materials that can be used in the sensor of the first disclosure of this specification, and in the method for manufacturing this sensor, will now be described.

There are no particular limitations on the material of the insulating substrate of the sensor of the first disclosure in this specification, but examples include polyethylene terephthalate, polycarbonate, polyimide, polyethylene, polypropylene, polystyrene, polyvinyl chloride, polyoxymethylene, monomer cast nylon, polybutylene terephthalate, methacrylic resin, ABS resin, and other such resin materials, and a glass material can also be used. Polyethylene terephthalate, polycarbonate, and polyimide are preferable, and polyethylene terephthalate is more preferable. There are no particular limitations on the dimensions of the substrate, such as its thickness, but the substrate can, for example, have a thickness of 0.05 mm or more and 2 mm or less, and preferably 0.1 mm or more and 1 mm or less.

The conductive layer of the working electrode of the sensor of the first disclosure in this specification is a layer containing a conductive material such as carbon, gold, platinum, palladium, or the like. The conductive layers of the working electrode, reference electrode, and counter electrode can be manufactured by forming a layer of one of the conductive materials mentioned above on the surface of the substrate by using sputtering, vapor deposition, screen printing, or another such method. The conductive layer can be worked into a specific pattern by laser trimming as necessary. The wiring of the sensor according to the embodiment (discussed below) can also be made of a similar conductive material.

The sensor of the first disclosure of this specification is immersed in a liquid sample to detect a specific analyte in the liquid sample. Examples of the analyte include glucose, lactic acid, cholesterol, bilirubin, amino acids such as glutamine and glutamic acid, glycated amino acids, glycated peptides, ketone bodies (such as 3-hydroxybutyric acid), and alcohols.

The working electrode of the sensor of the first disclosure of this specification comprises a reagent layer that contains a reagent that participates in an oxidation-reduction reaction. The reagent that participates in the oxidation-reduction reaction can be any one that will participate in an oxidation-reduction reaction of the analyte, and can be appropriately selected to suit the analyte. The reagent that participates in the oxidation-reduction reaction may include a combination of an oxidoreductase and a mediator (electron transfer substance), or an oxidoreductase. The oxidoreductase may comprise a coenzyme.

Examples of the oxidoreductase include an oxidase and a dehydrogenase. Specific examples of the oxidoreductase include glucose oxidase, lactate oxidase, cholesterol oxidase, bilirubin oxidase, glucose dehydrogenase, lactate dehydrogenase, amino acid oxidase, amino acid dehydrogenase, glutamate oxidase, glutamate dehydrogenase, fructosyl amino acid oxidase, fructosyl peptide oxidase, 3-hydroxybutyrate dehydrogenase, alcohol oxidase, and alcohol dehydrogenase. These oxidoreductases can be used to detect the analytes listed above.

Also, the mediator can be one or more selected from, but is not limited to, metal complexes (such as osmium complexes, ruthenium complexes, and iron complexes), quinone compounds (such as benzoquinone, naphthoquinone, phenanthrenequinone, phenanthrolinequinone, anthraquinone, and derivatives thereof), phenazine compounds, viologen compounds, phenothiazine compounds, and phenol compounds. Specific examples of the mediator include one or more selected from among potassium ferricyanide, hexaammineruthenium, ferrocene, poly(1-vinylimidazole)-bis(bipyridine)chloroosmium, hydroquinone, 2-methyl-1,4-benzoquinone, 1,2-naphthoquinone-4-sulfonate, 9,10-phenanthrenequinone-2-sulfonate, 9,10-phenanthrenequinone-2,7-disulfonate, 1,10-phenanthroline-5,6-dione, anthraquinone-2-sulfonate, phenazine derivatives (1-methoxy-5-methylphenazinium methyl sulfate, 1-methoxy-5-ethylphenazinium ethyl sulfate, etc.), methyl viologen, benzyl viologen, methylene blue, methylene green, 2-aminophenol, 2-amino-4-methylphenol, and 2,4-diaminophenol. Examples of the above-mentioned salts include, but are not limited to, sodium salts, potassium salts, calcium salts, magnesium salts, and lithium salts.

From the standpoint of sensor durability and suppression of outflow to the outside of the sensor, the mediator is preferably a mediator bound to a polymer compound, which is referred to as a high molecular mediator. The polymer compound bound to the mediator can be a homopolymer, a random copolymer, a block copolymer, or a polymer compound in which these are bound or mixed. The weight average molecular weight of the polymer compound is, for example, 10,000 or more, preferably 50,000 or more, and more preferably 100,000 or more, and the upper limit to the weight average molecular weight is, for example, less than 10,000,000, and preferably less than 1000,000. Also, there are no particular limitations on the polymer compound, but examples include those in which atoms of at least one type selected from among carbon atoms, nitrogen atoms, oxygen atoms, and sulfur atoms are bound in a chain shape to form a main chain. Specific examples of this include natural polymer compounds such as proteins, polypeptides, and polynucleotides, and synthetic polymer compounds such as polyamino acids, polyimines, polyallyl compounds, poly(meth)acrylates, polyalkylene oxides, and copolymers thereof. Examples of polyamino acids include poly(L-glutamic acid) and poly(L-lysine). Examples of polyimines include polyalkyleneimines such as polyethyleneimine and polypropyleneimine. Examples of polyallyl compounds include polyallylamine and polydiallylamine. Examples of polyalkylene oxides include polyethylene oxide and polypropylene oxide. The entire high molecular mediator is preferably hydrophilic, and furthermore, it is preferable for the polymer compound to which the mediator is bonded to be hydrophilic.

As the high molecular mediator, a mediator and a polymer compound bonded together via a covalent bond can be used. This covalent bond can be what is described in JP-A 2003-514924, for example, and specific examples include ether bonds, thioether bonds, ester bonds, urethane bonds, and amide bonds. The covalent bond may be formed from a reactive group originally had by the monomer forming the polymer compound, or may be formed from a reactive group had by a linker separately introduced into the mediator or the polymer compound. Specific examples of the high molecular mediator include one in which a phenazine compound is used as the mediator, poly(L-lysine) is used as the polymer, and the phenazine compound and poly(L-lysine) are bonded via an amide bond.

The reagent layer of the working electrode of the sensor in the first disclosure of this specification can be formed by drying a liquid composition A containing a reagent that participates in an oxidation-reduction reaction in water. The liquid composition A contains the reagent in water, and may further contain components such as a buffer, a hydrophilic polymer compound, a conductive carbon filler, or a crosslinking agent as needed. An example of a hydrophilic polymer compound is a cellulose derivative, and examples of cellulose derivatives include one or more selected from among methyl cellulose, carboxymethyl cellulose, hydroxyethyl cellulose, hydroxypropyl cellulose, hydroxyethyl methyl cellulose, and hydroxypropyl methyl cellulose. Examples of conductive carbon fillers include one or more selected from among carbon black, graphite powder, porous carbon, and nanocarbon.

The protective film of the working electrode of the sensor in the first disclosure of this specification can be a film that prevents or suppresses leakage of the reagent contained in the reagent layer to the outside of the protective film and is permeable to an analyte that is outside the protective film. A protective film having such properties preferably contains a polymer compound. Examples of the polymer compound contained in the protective film include a polymer compound containing 4-vinylpyridine as a structural unit, and a polymer compound containing a cation exchange functional group.

Examples of the polymer compound containing 4-vinylpyridine as a structural unit include poly(4-vinylpyridine), a copolymer (preferably a block copolymer) of 4-vinylpyridine and an alkyl methacrylate, and a copolymer (preferably a random copolymer) of styrene, 4-vinylpyridine, and an oligopropylene glycol methyl ether methacrylate. An example of an alkyl methacrylate is tert-butyl methacrylate. An example of an oligopropylene glycol methyl ether methacrylate is tripropylene glycol methyl ether methacrylate. The polymer compound containing 4-vinylpyridine as a structural unit is preferably crosslinked with a crosslinking agent containing two or more epoxy groups, such as polyethylene glycol diglycidyl ether (PEGDGE). A polymer compound containing 4-vinylpyridine as a structural unit and crosslinked with a crosslinking agent containing two or more epoxy groups includes a quaternary ammonium cation-containing functional group generated by a reaction between a pyridyl group (tertiary amine) derived from 4-vinylpyridine and an epoxy group. A preferred embodiment of the polymer compound containing 4-vinylpyridine as a structural unit and crosslinked with a crosslinking agent containing two or more epoxy groups is the specific embodiment of the "second polymer compound containing a cationic functional group" described in the fourth disclosure.

Examples of a polymer compound containing a cation exchange functional group include a polymer compound containing a structural unit having a sulfonic acid group on a side chain, with a polymer compound containing a perfluoro compound having a sulfonic acid group on a side chain as a structural unit being preferable, a copolymer compound containing a perfluoro compound having a sulfonic acid group on a side chain and a perfluoro compound having no ionic functional group on a side chain as a structural unit being better yet, a copolymer compound of tetrafluoroethylene and perfluoro-2-(2-fluorosulfonylethoxy)propyl vinyl ether being particularly favorable, and Nafion (registered trademark) being especially good. In a preferred embodiment of the polymer compound containing a cation exchange functional group, the specific embodiment of the "first polymer compound containing a cation exchange functional group" described in the fourth disclosure is preferable. The protective film containing the polymer compound containing a cation exchange functional group is preferably provided on the reagent layer in order to transport protons or other such cations between the reagent layer and the liquid sample.

The protective film of the working electrode of the sensor in the first disclosure of this specification can be a laminate of two or more protective films, such as a laminate of a protective film containing a polymer compound that contains a cation exchange functional group and is provided on the side in contact with the reagent layer, and a protective film that contains a polymer compound containing 4-vinylpyridine as a structural unit and is provided over the first film.

The protective film of the working electrode of the sensor in the first disclosure of this specification can be formed by drying a liquid composition B containing protective film components in an alcohol. Examples of the protective film components here include a polymer compound and a crosslinking agent contained in the protective film. Examples of the alcohol in the liquid composition B include C₁ to C₅ monohydric alcohols, with methanol, ethanol, or isopropyl alcohol being particularly favorable, and with ethanol being the most favorable.

The first insulating layer of the working electrode of the sensor in the first disclosure of this specification has a water-repellent surface. Here, "water-repellent surface" means a surface having a contact angle to water of at least 90°, for example, preferably at least 100°, and most preferably at least 110°. There is no particular upper limit to the contact angle to water of the water-repellent surface of the first insulating layer, but this angle can be, for example, 160° or less (the range of the contact angle to water is, for example, 90° or more and 160° or less).

The second insulating layer of the working electrode of the sensor in the first disclosure of this specification has a surface that is liquid-repellent to alcohol (alcohol-repellent). Here, "liquid-repellent surface to alcohol" means a surface having a contact angle to alcohol of, for example, at least 45°, preferably at least 50°, and most preferably at least 55°. There are no particular limitations on the upper limit of the contact angle to alcohol of the surface of the second insulating layer that is liquid-repellent to alcohol, but can be, for example, 100° or less (the range of the contact angle to alcohol is, for example, 45° or more and 100° or less). Here, the alcohol can be, for example, a C₁ to C₅ monohydric alcohol, with methanol, ethanol, or isopropyl alcohol being particularly favorable, and with ethanol being the most favorable.

The contact angle of the surface of the first insulating layer to water and the contact angle of the surface of the second insulating layer to alcohol can be the value measured at 20°C. The contact angle of the surface of the first insulating layer to water and the contact angle of the surface of the second insulating layer to alcohol can be measured using a commercially available analysis device, such as the Handy Contact Angle/Surface Free Energy Analysis Device MSA manufactured by Krüss. The contact angle of the surface to water or alcohol is preferably measured by discharging a 2-µL droplet of water or alcohol onto the surface to be measured, and measuring the contact angle between the droplet and the surface after 2 seconds.

The water-repellent surface of the first insulating layer of the working electrode of the sensor in the first disclosure of this specification preferably contains a fluororesin. This fluororesin can be a polymer compound of a fluorohydrocarbon, examples of which include a polymer compound containing one or more monomers selected from among vinylidene fluoride, tetrafluoroethylene, hexafluoropropylene, and perfluoro(alkyl vinyl ether), with a copolymer containing two or more monomers selected from among vinylidene fluoride, tetrafluoroethylene, hexafluoropropylene, and perfluoro(alkyl vinyl ether) being preferable, and a copolymer containing vinylidene fluoride and hexafluoropropylene being better yet. The first insulating layer can be made entirely of a fluororesin.

The surface of the second insulating layer that is liquid-repellent to alcohol of the working electrode of the sensor in the first disclosure of this specification preferably contains a compound containing a perfluoroalkyl group. Examples of a compound containing a perfluoroalkyl group include a fluorine-based surface modification additive. This second insulating layer can be formed by coating the first insulating layer with a composition containing an insulating matrix resin and a compound that includes a perfluoroalkyl group (fluorine-based surface modification additive) in a solvent, and drying this coating. At this time, the compound containing a perfluoroalkyl group segregates to the surface and forms a surface that is liquid-repellent to alcohol. The second insulating layer thus formed is preferably a layer of an insulating matrix resin containing a compound containing a perfluoroalkyl group on its surface. There are no particular limitations on the type of insulating matrix resin, but it may be a polyester resin, for example. There are no particular limitations on the number of carbon atoms of the perfluoroalkyl group, but it may be in the range of 2 to 20 carbon atoms, for example. The perfluoroalkyl group may contain a trifluoromethyl group at the end.

### Sensor 1 Overview

A sensor 1 according to an embodiment of the first disclosure will now be described through reference to the drawings.

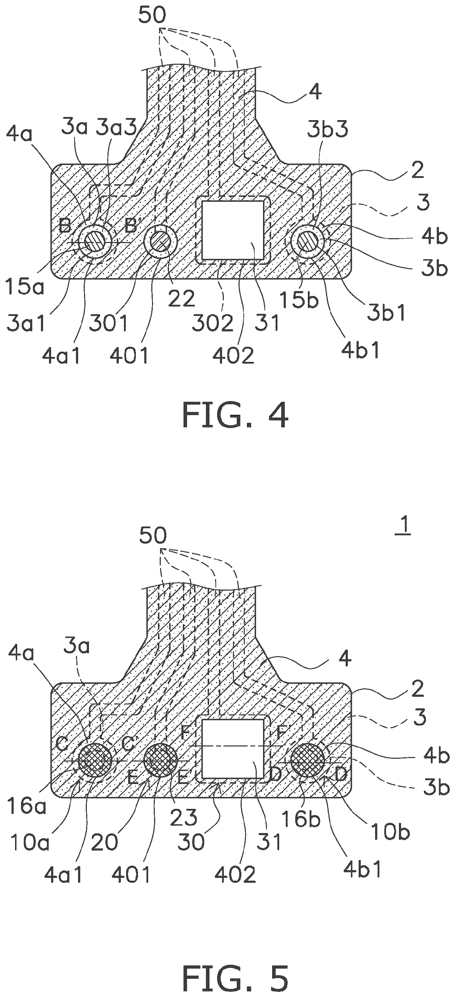

As shown in Fig. 5, the sensor 1 in this embodiment comprises an insulating substrate 2; a first working electrode 10a, a second working electrode 10b, a reference electrode 20, and a counter electrode 30 that are disposed on the substrate 2; and wiring 50 that is electrically connected to each of the first working electrode 10a, the second working electrode 10b, the reference electrode 20, and the counter electrode 30. The sensor 1 in this embodiment includes two working electrodes, but in another embodiment (not shown), the number of working electrodes may be only one, or may be three or more. In the following description, when the first working electrode 10a and the second working electrode 10b are not distinguished from one another, they will sometimes be referred to as the working electrodes 10a and 10b. Also, the first working electrode 10a, the second working electrode 10b, the reference electrode 20, and the counter electrode 30 may be collectively referred to as the electrodes. Also, the sensor 1 in this embodiment is a three-electrode sensor including a working electrode, a reference electrode, and a counter electrode as electrodes, but may be a two-electrode sensor including only a working electrode and a counter electrode, without having a reference electrode. Although not depicted in the drawings, the reference electrode and/or the counter electrode may be provided on a substrate separate from the substrate on which the working electrode is disposed.

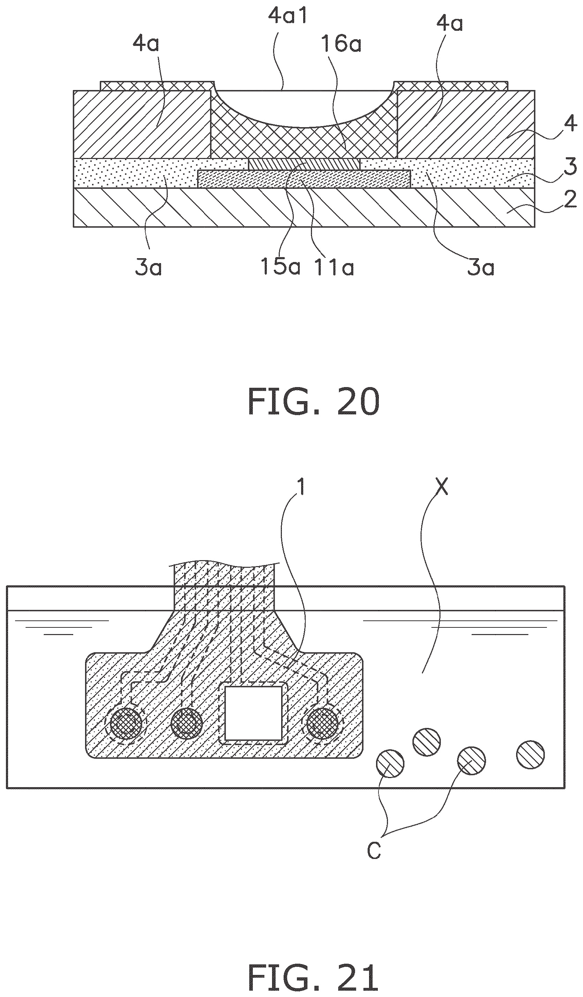

As shown in Fig. 21, the sensor 1 is immersed in a liquid sample X and used to detect a specific analyte in the liquid sample X. Examples of the liquid sample X include a culture solution containing cells C as shown in the drawings, and a liquid sample prepared using blood collected from a living organism. Specific examples of the analyte are given in the Materials section.

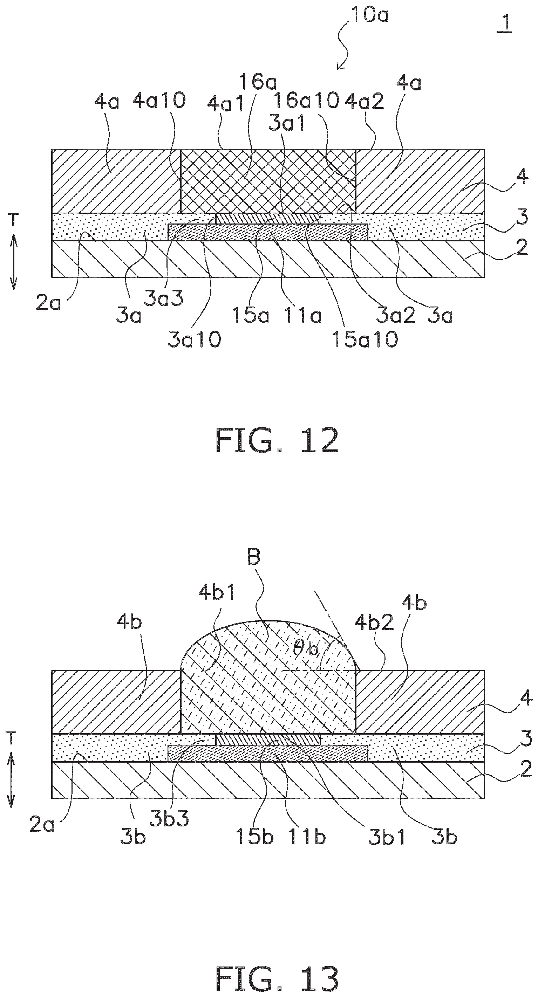

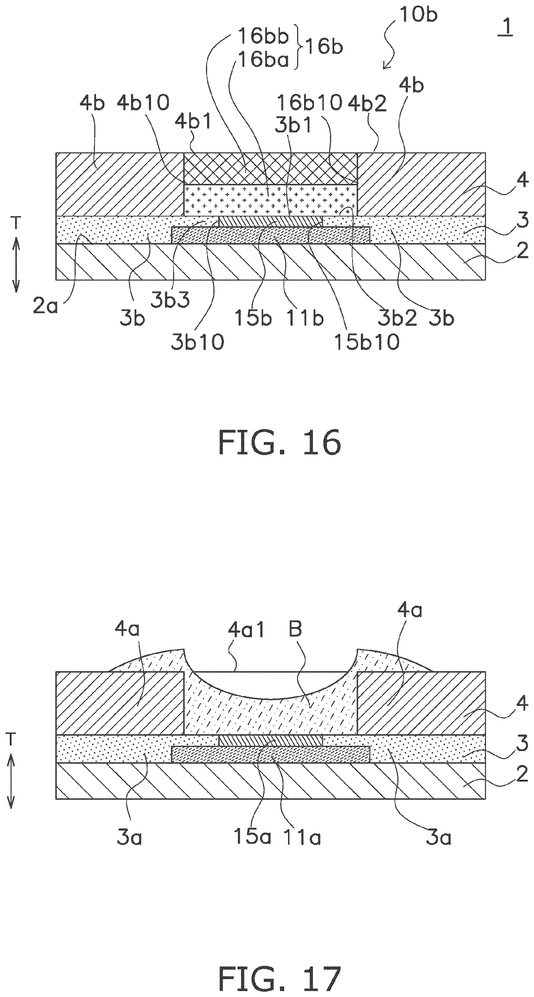

As shown in Figs. 12 and 16, the working electrodes 10a and 10b of the sensor 1 include reagent layers 15a and 15b containing a reagent that participates in the oxidation-reduction reaction of the analyte in the liquid sample X, these reagent layers being provided on conductive layers 11a and 11b. A preferred embodiment of the reagent that participates in the oxidation-reduction reaction is given in the Materials section.

If reagent layers 15a and 15b of the working electrodes 10a and 10b of the sensor 1 contain a reagent that oxidizes the analyte in the liquid sample X, electrons move from the analyte to the conductive layers 11a and 11b under conditions in which a specific voltage is applied to the electrodes of the sensor 1. Similarly, if the reagent layers 15a and 15b contain a reagent that reduces the analyte in the liquid sample X, electrons move from the conductive layers 11a and 11b to the analyte. Since the amount of electrons that move depends on the concentration of the analyte, the concentration, or a change in the concentration, of the analyte in the liquid sample X can be measured on the basis of the value of the current, or a change in the current, flowing through the working electrodes 10a and 10b of the sensor 1.

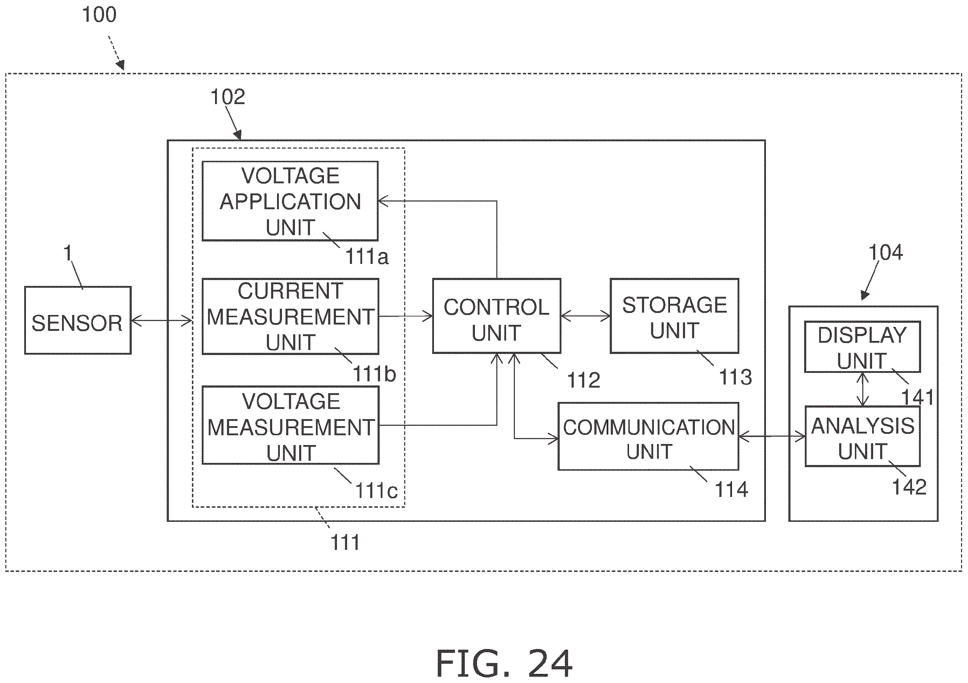

An example of an analysis device 100 for analyzing an analyte in a liquid sample, which comprises the sensor 1, will be described with reference to Fig. 24.

As shown in Fig. 24, the analysis device 100 comprises the sensor 1, an analyzer 102, and a controller 104. The working electrodes 10a and 10b, the reference electrode 20, and the counter electrode 30 of the sensor 1 are each connected to the analyzer 102 via the wiring 50. The analyzer 102 can communicate with the controller 104.

The analyzer 102 comprises an electrochemical measurement unit 111, a control unit 112, a storage unit 113, and a communication unit 114.

The electrochemical measurement unit 111 is a potentiostat that applies a specific voltage to each electrode of the sensor 1 and measures the concentration of the analyte, and includes a voltage application unit 111a and a current measurement unit 111b, and preferably also includes a voltage measurement unit (counter electrode terminal voltage measurement unit) 111c.

The voltage application unit 111a applies a specific voltage to the electrodes of the sensor 1 in order to measure the concentration of the analyte contained in the liquid sample X.

The current measurement unit 111b senses the value of, or detects a change in, the current flowing between the working electrodes 10a and 10b and the counter electrode 30 of the sensor 1, which is measured in a state in which voltage has been applied from the voltage application unit 111a to the electrodes of the sensor 1. As discussed above, the current value, or a change in the current value, sensed by the current measurement unit 111b is an index of the concentration, or change in concentration, of the analyte in the liquid sample X.

The voltage measurement unit 111c measures the terminal voltage of each electrode of the sensor 1.

The control unit 112 is connected to the voltage application unit 111a, the current measurement unit 111b, the voltage measurement unit 111c, the memory unit 113, and the communication unit 114. The control unit 112 controls the voltage application unit 111a so as to apply a specific voltage to each electrode of the sensor 1, and controls the communication unit 114 so as to transmit the measurement results from the current measurement unit 111b and the voltage measurement unit 111c to the controller 104.

The memory unit 113 is connected to the control unit 112, and stores data such as the value of the applied voltage preset for each measurement object, the measurement values at the current measurement unit 111b and the voltage measurement unit 111c, pre-measured calibration curves, and the like.

The communication unit 114 is controlled by the control unit 112 and transmits data, such as the measurement results at the current measurement unit 111b and the voltage measurement unit 111c to an analysis unit 142 of the controller 104.

The controller 104 can communicate with the analyzer 102 via the communication unit 114, and comprises a display unit 141 and the analysis unit 142.

The display unit 141 displays, as the result of the analysis performed by the analysis unit 142, the concentration of the analyte in the liquid sample X based on the current value sensed by the current measurement unit 111b, for example.

The analysis unit 142 is a PC (personal computer), for example, and calculates the concentration of the analyte on the basis of the value of the current flowing between the working electrodes 10a and 10b and the counter electrode 30, which is measured by the current measurement unit 111b.

### Working Electrodes

The features of the working electrodes 10a and 10b of the sensor 1 will now be described with reference to the drawings, and particularly Figs. 12 and 16. Fig. 12 is a cross-sectional view of the portion of the sensor 1 including the first working electrode 10a, along the C-C' line in Fig. 5. Fig. 16 is a cross-sectional view of the portion of the sensor 1 including the second working electrode 10b, along the D-D' line in Fig. 5.

The first working electrode 10a comprises a conductive layer 11a, a first insulating layer 3a, a second insulating layer 4a, a reagent layer 15a, and a protective film 16a (Fig. 12). The second working electrode 10b comprises a conductive layer 11b, a first insulating layer 3b, a second insulating layer 4b, a reagent layer 15b, and a protective film 16b (Fig. 16). In the following description, when the elements of the first working electrode 10a and the second working electrode 10b are mentioned without distinction between the two, they will be expressed as the conductive layers 11a and 11b, the first insulating layers 3a and 3b, the second insulating layers 4a and 4b, the reagent layers 15a and 15b, and the protective films 16a and 16b.

The conductive layers 11a and 11b of the working electrodes 10a and 10b are disposed on the insulating substrate 2. The main surface on the side where the conductive layers 11a and 11b are formed shall be referred to as the first surface 2a.

Preferred embodiments of the materials for the substrate 2 and the conductive layers 11a and 11b are given in the Materials section.

At least a portion of the first insulating layers 3a and 3b of the working electrodes 10a and 10b is disposed on the conductive layers 11a and 11b. As shown in Figs. 12 and 16, a portion of the first insulating layers 3a and 3b may be disposed on the conductive layers 11a and 11b, or, although not depicted in the drawings, the entire first insulating layers 3a and 3b may be disposed on the conductive layers 11a and 11b.

In the embodiment shown in the drawings, the first insulating layers 3a and 3b of the working electrodes 10a and 10b refer to the portions of the first insulating layer 3, which covers substantially the entire first surface 2a of the substrate 2, that are close to the conductive layers 11a and 11b of the working electrodes 10a and 10b. However, this embodiment is not the only option, and the first insulating layers 3a and 3b of the working electrodes 10a and 10b may be disposed only in the portions near the conductive layers 11a and 11b of the working electrodes 10a and 10b.

The first insulating layers 3a and 3b of the working electrodes 10a and 10b are characterized by having water-repellent surfaces 3a2 and 3b2 and first openings 3a1 and 3b1 that go through in the thickness direction T and are formed at positions overlapping parts of the conductive layers 11a and 11b in plan view from the thickness direction T of the substrate 2. The plan view shape of inner peripheral edges 3a10 and 3b10 of the first openings 3a1 and 3b1 from the thickness direction T is circular in the example shown in the drawings, but this is not the only option, and may instead polygonal (rectangular, triangular, etc.) or any other shape.

There are no particular limitations on the thickness of the portions of the first insulating layers 3a and 3b of the working electrodes 10a and 10b covering the conductive layers 11a and 11b, but the thickness may be, for example, 0.5 µm or more and 50 µm or less, and preferably 2 µm or more and 20 µm or less.

There are no particular limitations on the opening width of the first openings 3a1 and 3b1 of the first insulating layers 3a and 3b of the working electrodes 10a and 10b, but this width can be, for example, 0.5 mm or more and 5 mm or less, and preferably 1 mm or more and 2 mm or less.

The first insulating layers 3a and 3b of the working electrodes 10a and 10b have the water-repellent surfaces 3a2 and 3b2. As discussed below, the reagent layers 15a and 15b of the working electrodes 10a and 10b are formed in the first openings 3a1 and 3b1 of the first insulating layers 3a and 3b of the working electrodes 10a and 10b by forming droplets of a liquid composition A containing a reagent that participates in an oxidation-reduction reaction in water, and then drying the droplets. Since the surfaces 3a2 and 3b2 of the first insulating layers 3a and 3b of the working electrodes 10a and 10b are water-repellent, when the droplets are formed, the droplets of the liquid composition A tend to hold their shape, without collapsing. Therefore, it is easy to control the characteristics of the reagent layers 15a and 15b after drying (such as the thickness of the reagent layers 15a and 15b, and the amount of the reagent constituting the reagent layers 15a and 15b) within the designed range.

Preferred embodiments of the material constituting the first insulating layers 3a and 3b having the water-repellent surfaces 3a2, 3b2 are given in the Materials section.

The second insulating layers 4a and 4b of the working electrodes 10a and 10b are disposed on the first insulating layers 3a and 3b. In the embodiment depicted in the drawings, the second insulating layers 4a and 4b of the working electrodes 10a and 10b refer to the portions of the second insulating layer 4, which covers almost the entire first surface 2a of the substrate 2, that are near the conductive layers 11a and 11b of the working electrodes 10a and 10b. However, the present invention is not limited to this embodiment, and the second insulating layers 4a and 4b of the working electrodes 10a and 10b may be disposed only in the portions near the conductive layers 11a and 11b of the working electrodes 10a and 10b.

The second insulating layers 4a and 4b of the working electrodes 10a and 10b have second openings 4a1 and 4b1 that go through in the thickness direction T and are formed at positions that overlap portions 3a3 and 3b3 of the first insulating layers 3a and 3b that entirely encompass the first openings 3a1 and 3b1 in plan view in the thickness direction T of the substrate 2, and also have surfaces 4a2 and 4b2 that are liquid-repellent to alcohol.

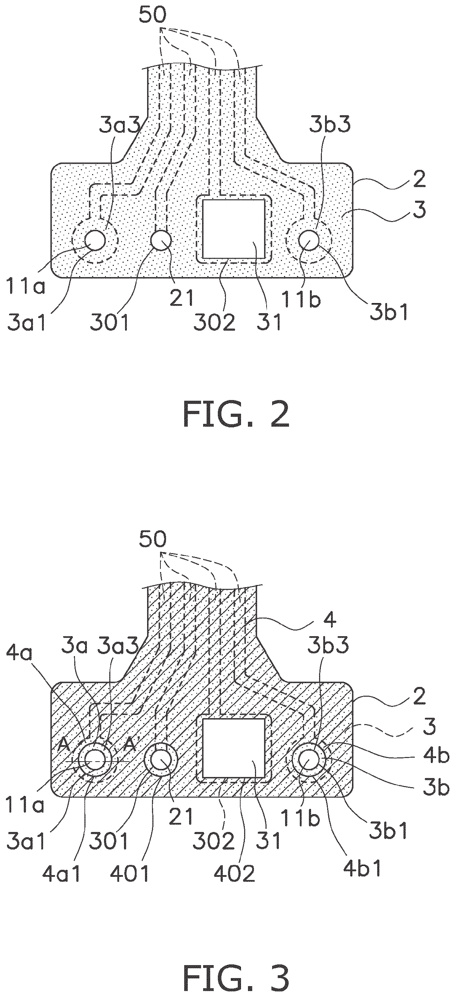

The shape of the inner peripheral edges 4a10 and 4b10 of the second openings 4a1 and 4b1 of the second insulating layers 4a and 4b of the working electrodes 10a and 10b in plan view from the thickness direction T is circular in the example depicted in the drawings, but this is not the only option, and may be polygonal (rectangular, triangular, etc.) or any other shape. As shown in Fig. 3, the inner peripheral edges 3a10 and 3b10 of the first openings 3a1 and 3b1 of the first insulating layers 3a and 3b and the inner peripheral edges 4a10 and 4b10 of the second openings 4a1 and 4b1 of the second insulating layers 4a and 4b are preferably parallel in plan view from the thickness direction T, but need not be. The second openings 4a1 and 4b1 of the second insulating layers 4a and 4b are recesses at the bottom of which include the first openings 3a1 and 3b1 of the first insulating layers 3a and 3b and the reagent layers 15a and 15b on the inside thereof, as well as the regions of the water-repellent surfaces 3a2 and 3b2 of the first insulating layers 3a and 3b that surround the first openings 3a1 and 3b1.

There are no particular limitations on the thickness of the second insulating layers 4a and 4b of the working electrodes 10a and 10b, but this thickness may be, for example, 5 µm or more and 100 µm or less, and preferably 10 µm or more and 50 µm or less.

There are no particular limitations on the opening width of the second openings 4a1 and 4b1 of the second insulating layers 4a and 4b of the working electrodes 10a and 10b, but this width can be, for example, 0.5 mm or more and 5 mm or less, and preferably 1 mm or more and 3 mm or less.

The second insulating layers 4a and 4b of the working electrodes 10a and 10b have surfaces 4a2 and 4b2 that are liquid-repellent to alcohol (alcohol-repellent). As will be discussed below, the protective films 16a and 16b of the working electrodes 10a and 10b are formed by forming droplets of a liquid composition B containing a protective film component in alcohol in the second openings 4a1 and 4b1 of the second insulating layers 4a and 4b of the working electrodes 10a and 10b, and then drying the droplets. During formation of the droplets of the liquid composition B, since the surfaces 4a2 and 4b2 of the second insulating layers 4a and 4b of the working electrodes 10a and 10b are liquid-repellent to alcohol, the droplets of the liquid composition B tend to hold their shape, without collapsing. It is therefore easy to control the characteristics of the protective films 16a and 16b after drying (such as the thickness of the protective films 16a and 16b, and the amount of the components constituting the protective films 16a and 16b) within the designed range.

Preferred embodiments of the material constituting the second insulating layers 4a and 4b having the surfaces 4a2 and 4b2 that are liquid-repellent (alcohol-repellent) to alcohol are as described in the Materials section.

The reagent layers 15a and 15b of the working electrodes 10a and 10b are disposed in the first openings 3a1 and 3b1 of the first insulating layers 3a and 3b, and include outer peripheral edges 15a10 and 15b10 defined by inner peripheral edges 3a10 and 3b10 of the first openings 3a1 and 3b1 of the first insulating layers 3a and 3b, and a reagent that participates in an oxidation-reduction reaction. There are no particular limitations on the thickness of the reagent layers 15a and 15b, but this thickness is preferably about the same as or less than the thickness of the portions of the first insulating layers 3a and 3b that cover the conductive layers 11a and 11b, as shown in the drawings.

Preferred embodiments of reagents that participate in the oxidation-reduction reaction in the reagent layers 15a and 15b are as described in the Materials section.

The protective films 16a and 16b of the working electrodes 10a and 10b are disposed in the second openings 4a1 and 4b1 of the second insulating layers 4a and 4b, and include outer peripheries 16a10, 16b10 defined by inner peripheries 4a10, 4b10 of the second openings 4a1 and 4b1 of the second insulating layers 4a and 4b. There are no particular limitations on the thickness of the protective films 16a and 16b, but this thickness is preferably about the same as or less than the thickness of the second insulating layers 4a and 4b, as shown in the drawings.

Preferred embodiments of the protective films 16a and 16b are as described in the Materials section.

In the following description, in order to distinguish between the "reagent layer 15a" of the first working electrode 10a and the "reagent layer 15b" of the second working electrode 10b, the former will sometimes be referred to as the "first reagent layer 15a" of the first working electrode 10a and the latter as the "second reagent layer 15b" of the second working electrode 10b. Also, the reagent contained in the reagent layer 15a of the first working electrode 10a will sometimes be referred to as the "first reagent," and the reagent contained in the second reagent layer 15b of the second working electrode 10b will sometimes be referred to as the "second reagent."

In a preferred embodiment, the sensor 1 comprises a plurality of working electrodes 10a and 10b, and the first reagent contained in the first reagent layer 15a of a first working electrode 10a (out of the plurality of working electrodes 10a and 10b) is different from the second reagent contained in the second reagent layer 15b of the second working electrode 10b (which is different from the first working electrode 10a). The sensor 1 according to this preferred embodiment can be used to detect a plurality of analytes, including a first analyte and a second analyte, in a liquid sample X, because the first reagent participates in an oxidation-reduction reaction of the first analyte in the liquid sample X, and the second reagent participates in an oxidation-reduction reaction of the second analyte, which is different from the first analyte, in the liquid sample X.

In the sensor 1 according to the above preferred embodiment comprising the plurality of working electrodes 10a and 10b, the first reagent preferably contains an enzyme that participates in the oxidation-reduction reaction of glucose, and more preferably glucose dehydrogenase or glucose oxidase, and the second reagent preferably contains an enzyme that participates in the oxidation-reduction reaction of lactic acid, and more preferably lactate oxidase or lactate dehydrogenase. The sensor 1 in this embodiment can detect glucose and lactic acid.

### Reference Electrode

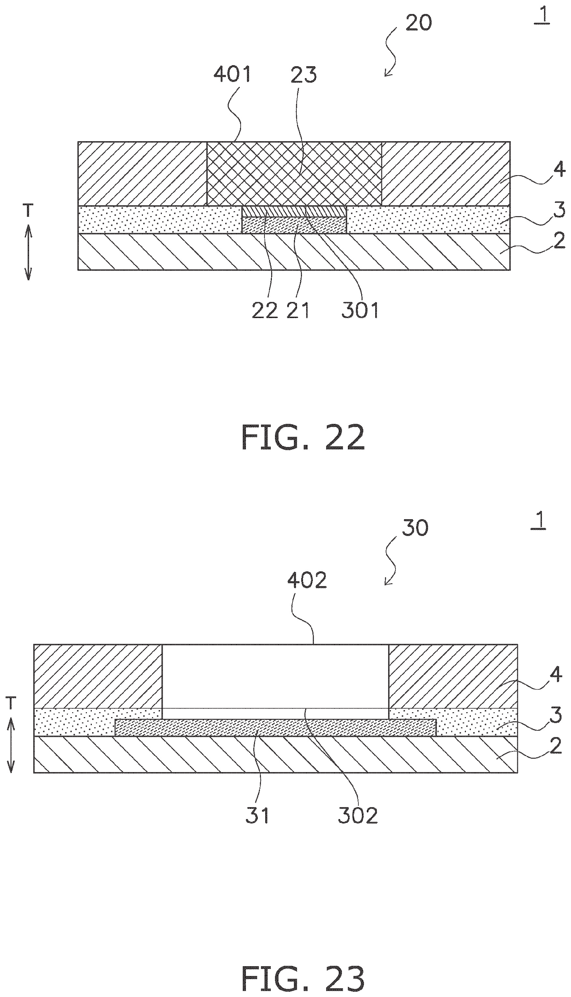

The structure of the reference electrode 20 of the sensor 1 will be described through reference to the drawings, and particularly Fig. 22. Fig. 22 is a cross-sectional view of the portion of the sensor 1 that includes the reference electrode 20, along the E-E' line in Fig. 5.

The reference electrode 20 comprises a reference electrode conductive layer 21 that is disposed on the first surface 2a of the insulating substrate 2, a silver/silver chloride layer 22 that is disposed on the reference electrode conductive layer 21, and a reference electrode protective film 23 hat is disposed on the silver/silver chloride layer 22.

The reference electrode conductive layer 21 can be formed from the materials described for the conductive layers 11a and 11b of the working electrodes 10a and 10b.

The reference electrode protective film 23 can be formed from the materials described in the Materials section in relation to the protective films 16a and 16b of the working electrodes 10a and 10b.

The first insulating layer 3 disposed on the first surface 2a of the substrate 2 includes a reference electrode first opening 301 that is partially disposed on the reference electrode conductive layer 21, goes through in the thickness direction T, and is formed at a position overlapping a portion of the reference electrode conductive layer 21 in plan view from the thickness direction T of the substrate 2. The silver/silver chloride layer 22 of the reference electrode 20 is disposed in the reference electrode first opening 301 of the first insulating layer 3.

The second insulating layer 4 disposed on the first insulating layer 3 includes a reference electrode second opening 401 that goes through in the thickness direction T and is formed at a position overlapping the portion of the first insulating layer 3 that entirely encompasses the reference electrode first opening 301 in plan view from the thickness direction T. The reference electrode protective film 23 of the reference electrode 20 is disposed in the reference electrode second opening 401 of the second insulating layer 4.

### Counter Electrode

The structure of the counter electrode 30 of the sensor 1 will be described through reference to the drawings, and particularly Fig. 23. Fig. 23 is a cross-sectional view of the portion of the sensor 1 that includes the counter electrode 30, along the F-F' line in Fig. 5.

The counter electrode 30 comprises a counter electrode conductive layer 31 that is disposed on the first surface 2a of the insulating substrate 2.

The counter electrode conductive layer 31 can be formed from the materials described in relation to the conductive layers 11a and 11b of the working electrodes 10a and 10b.

The first insulating layer 3 disposed on the first surface 2a of the substrate 2 includes a counter electrode first opening 302 that is partially disposed on the counter electrode conductive layer 31, goes through in the thickness direction T, and is formed at a position that overlaps the portion of the counter electrode conductive layer 31 in plan view from the thickness direction T of the substrate 2.

The second insulating layer 4 disposed on the first insulating layer 3 includes a counter electrode second opening 402 that goes through in the thickness direction T and is formed at the position of the first insulating layer 3 that overlaps the counter electrode first opening 302 in plan view from the thickness direction T.

The counter electrode conductive layer 31 of the counter electrode 30 is exposed to the outside through the counter electrode first opening 302 of the first insulating layer 3 and the counter electrode second opening 402 of the second insulating layer 4. Therefore, when the sensor 1 is immersed in the liquid sample X as shown in Fig. 21, the counter electrode conductive layer 31 of the counter electrode 30 comes into direct contact with the liquid sample X.

### Method for Manufacturing Sensor 1

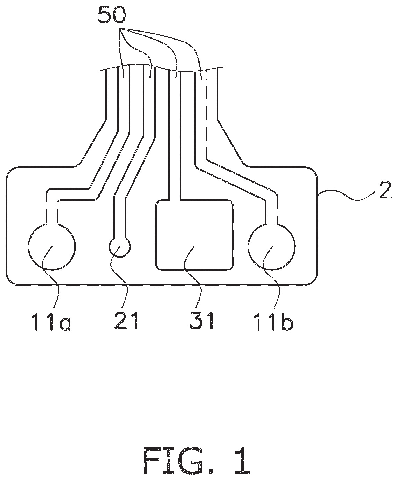

A preferred embodiment of the method for manufacturing the sensor 1 will now be described through reference to the drawings, and particularly Figs. 1 to 16.

First, as shown in Fig. 1, the conductive layers 11a and 11b of the working electrodes 10a and 10b, the reference electrode conductive layer 21 of the reference electrode 20, the counter electrode conductive layer 31 of the counter electrode 30, and the wiring 50 that is electrically connected to each of these, are disposed on the first surface 2a of the insulating substrate 2.

Then, as shown in Fig. 2, the first insulating layer 3 is further laminated on the first surface 2a of the substrate 2 on which the conductive layers 11a and 11b of the working electrodes 10a and 10b, the reference electrode conductive layer 21, the counter electrode conductive layer 31, and the wiring 50 are disposed. The portions of the first insulating layer 3 that are near the conductive layers 11a and 11b of the working electrodes 10a and 10b are the first insulating layers 3a and 3b of the working electrodes 10a and 10b. Parts of the first insulating layers 3a and 3b of the working electrodes 10a and 10b are disposed on the conductive layers 11a and 11b and cover the upper surfaces of the conductive layers 11a and 11b. The first insulating layers 3a and 3b of the working electrodes 10a and 10b have first openings 3a1 and 3b1 of the working electrodes 10a and 10b at positions overlapping parts of the conductive layers 11a and 11b of the working electrodes 10a and 10b. The first insulating layer 3 further has a reference electrode first opening 301 at a position overlapping a part of the reference electrode conductive layer 21, and a counter electrode first opening 302 at a position overlapping a part of the counter electrode conductive layer 31.

Then, as shown in Fig. 3, the second insulating layer 4 is further laminated on the first insulating layer 3. The portions of the second insulating layer 4 that are near the conductive layers 11a and 11b of the working electrodes 10a and 10b are the second insulating layers 4a and 4b of the working electrodes 10a and 10b. The second insulating layers 4a and 4b of the working electrodes 10a and 10b have second openings 4a1 and 4b1 formed at positions overlapping the portions 3a3 and 3b3 of the first insulating layers 3a and 3b of the working electrodes 10a and 10b that entirely encompass the first openings 3a1 and 3b1. The second insulating layer 4 further has a reference electrode second opening 401 at a position overlapping the reference electrode first opening 301 of the first insulating layer 3, and a counter electrode second opening 402 at a position overlapping the counter electrode first opening 302 of the first insulating layer 3. Preferably, the second insulating layer 4 can be formed by coating the first insulating layer with an ink containing an insulating matrix resin and a compound containing a perfluoroalkyl group (a fluorine-based surface modification additive) in a solvent, and drying this coating.

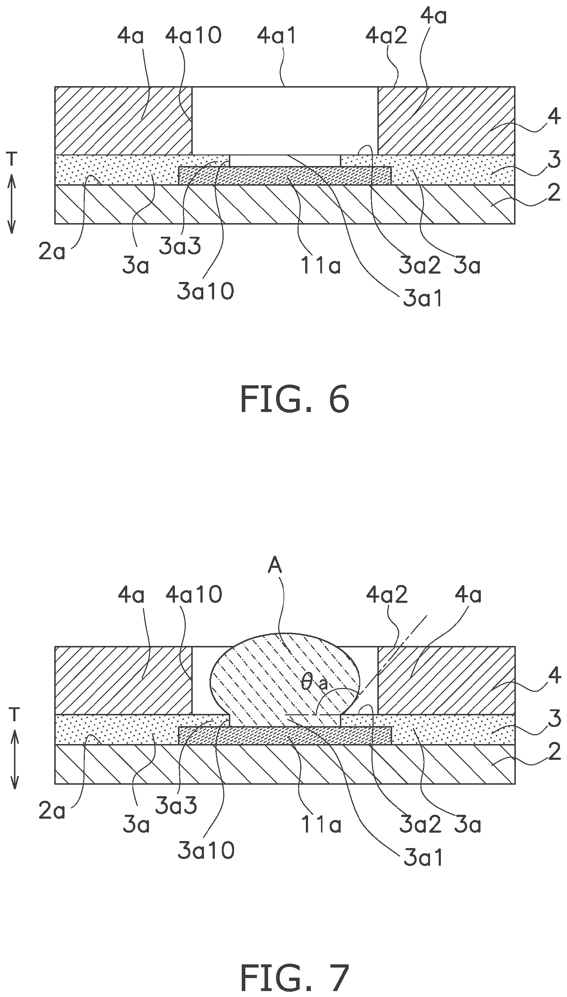

Fig. 6 shows a cross section taken along the A-A' line of the portion of the substrate 2, on which the conductive layers 11a and 11b of the working electrodes 10a and 10b, the reference electrode conductive layer 21, the counter electrode conductive layer 31, the wiring 50, the first insulating layer 3, and the second insulating layer 4 are disposed, that corresponds to the first working electrode 10a. As shown in Fig. 6, the first opening 3a1 of the first insulating layer 3a of the working electrode 10a is a recess at the bottom of which is included the conductive layer 11a of the working electrode 10a. The first insulating layer 3a of the working electrode 10a has a water-repellent surface 3a2.

Then, as shown in Fig. 7, a droplet of the liquid composition A containing a reagent that participates in an oxidation-reduction reaction in water is formed in the first opening 3a1 of the first insulating layer 3a of the working electrode 10a. At this point, since the surface 3a2 of the first insulating layer 3a of the working electrode 10a is water-repellent, the droplet of the liquid composition A holds its shape, without collapsing, until the interior angle θa (shown in Fig. 7) at the contact point with the surface 3a2 of the first insulating layer 3a reaches the water contact angle of the surface 3a2 of the first insulating layer 3a.

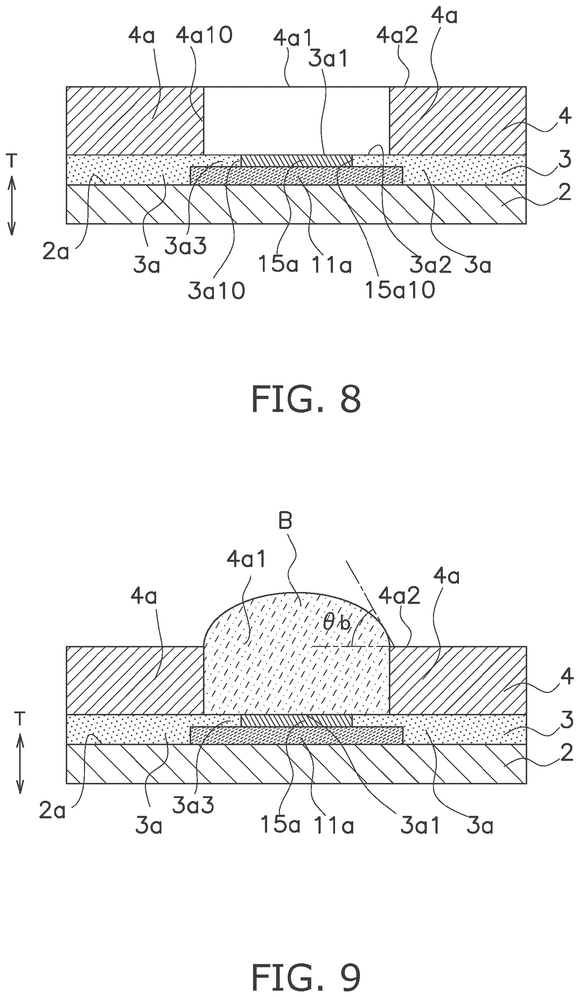

Thus, a droplet of the liquid composition A is formed in the first opening 3a1 of the first insulating layer 3a of the working electrode 10a, and is then dried to form the reagent layer 15a, as shown in Fig. 8. As discussed above, the droplet of the liquid composition A containing a reagent that participates in an oxidation-reduction reaction in water tends to hold its shape, without collapsing, in the first opening 3a1 of the first insulating layer 3a having a water-repellent surface 3a2, so it is easy to control the properties of the reagent layer 15a after drying (such as the thickness of the reagent layer 15a and the amount of the reagent constituting the reagent layer 15a) within the designed range. If the surface of the first insulating layer 3a did not have water repellency, then when droplet of the liquid composition A is formed in the first opening 3a1 of the first insulating layer 3a, the droplet would be more likely to collapse, causing the liquid composition A to wet the outside of the first opening 3a1 of the first insulating layer 3a, and it would not be easy to control the properties of the reagent layer obtained by drying to be within the designed range.

A preferred embodiment of the composition of the liquid composition A containing a reagent that participates in an oxidation-reduction reaction in water is as described in the Materials section.

After the reagent layer 15a is formed, a droplet of the liquid composition B containing a protective film component in alcohol is formed in the second opening 4a1 of the second insulating layer 4a of the working electrode 10a, as shown in Fig. 9. At this point, since the surface 4a2 of the second insulating layer 4a is liquid-repellent to alcohol, the droplet of the liquid composition B holds its shape, without collapsing, until the interior angle θb (shown in Fig. 9) at the contact point with the surface 4a2 of the second insulating layer 4a reaches the contact angle of the surface 4a2 of the second insulating layer 4a with the alcohol.

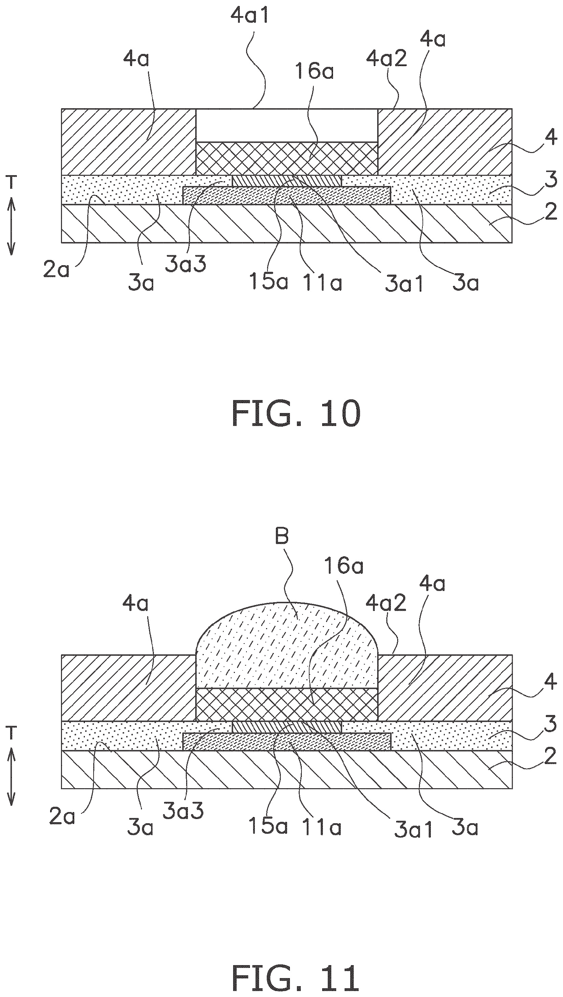

Thus, a droplet of the liquid composition B is formed in the second opening 4a1 of the second insulating layer 4a of the working electrode 10a, and is then dried to form a protective film 16a, as shown in Fig. 10. As discussed above, the droplet of the liquid composition B containing a protective film component in alcohol tends to hold its shape, without collapsing, in the second opening 4a1 of the second insulating layer 4a having the surface 4a2 that is liquid-repellent to alcohol, so it is easy to control the characteristics of the protective film 16a after drying (such as the thickness of the protective film 16a and the amount of components constituting the protective film 16a) within the designed range. If the surface of the second insulating layer 4a did not have liquid repellency to alcohol, when the droplet of the liquid composition B is formed in the second opening 4a1 of the second insulating layer 4a, the droplet would be likely to collapse and the liquid composition B to wet the outside of the second opening 4a1 of the second insulating layer 4a, and it would not be easy to control the characteristics of the protective film obtained by drying to the designed range (see the comparative example discussed below with reference to Figs. 17 to 20).

Preferred embodiments of the composition of the liquid composition B containing the protective film component in alcohol are as described in the Materials section.

Figs. 9 to 12 show an example in which the protective film 16a is formed in two stages in the second opening 4a1 of the second insulating layer 4a of the working electrode 10a. In this embodiment of the method for manufacturing the sensor 1, as shown in Fig. 9, after the reagent layer 15a is formed, a droplet of the liquid composition B containing a protective film component in alcohol is formed in the second opening 4a1 of the second insulating layer 4a, and is then dried to form a part of the protective film 16a in the thickness direction as shown in Fig. 10. Then, as shown in Fig. 11, another droplet of the liquid composition B is formed in the second opening 4a1 of the second insulating layer 4a, and is then dried to form the entire protective film 16a as shown in Fig. 12. However, this example is not the only option, and the protective film may be formed in a single stage, or in three or more stages.

As discussed above, in a preferred embodiment, the sensor 1 comprises a plurality of working electrodes 10a and 10b, and the first reagent contained in the first reagent layer 15a of the first working electrode 10a (out of the plurality of working electrodes 10a and 10b) is different from the second reagent contained in the second reagent layer 15b of the second working electrode 10b that is different from the first working electrode 10a. The method for manufacturing the sensor 1 in this preferred embodiment preferably comprises, as the steps for forming a reagent layer:
forming the first reagent layer 15a of the first working electrode 10a included in the plurality of working electrodes 10a and 10b by forming a droplet of the first liquid composition A containing the first reagent in water within the first opening 3a1 of the first insulating layer 3a of the first working electrode 10a, and then drying this droplet (see Figs. 6 to 8); and
forming the second reagent layer 15b of the second working electrode 10b that is different from the first working electrode 10a included in the plurality of working electrodes 10a and 10b, by forming a droplet of the second liquid composition A containing a second reagent that is different from the first reagent in water within the first opening 3b1 of the first insulating layer 3b of the second working electrode 10b, and then drying this droplet (although not shown, these are the same steps as those in Figs. 6 to 8).

With the method for manufacturing the sensor 1 according to this embodiment, the reagent layers 15a and 15b of the plurality of working electrodes 10a and 10b can be formed individually and independently, so the reagent layers 15a and 15b containing different reagents can be easily formed on the plurality of working electrodes 10a and 10b by adjusting the composition of the liquid composition A containing a reagent in water.

In the sensor 1 containing different reagents in the plurality of working electrodes 10a and 10b, which is manufactured by the method according to the preferred embodiment described above, the first reagent layer 15a of the first working electrode 10a contains a first reagent containing glucose dehydrogenase or glucose oxidase, for example. In this embodiment, the first working electrode 10a is provided with the first protective film 16a containing a polymer compound containing 4-vinylpyridine as a structural unit, as shown in Fig. 12. This first protective film 16a may comprise the following:
after the formation of the first reagent layer 15a of the first working electrode 10a, a droplet of the first liquid composition B containing a polymer compound containing 4-vinylpyridine as a structural unit in alcohol is formed within the second opening 4a1 of the second insulating layer 4a of the first working electrode 10a, and is then dried to form the first protective film 16a. The formation of the first protective film 16a may be performed in two stages as shown in Figs. 9 to 12, or may be performed in a single stage, or in three or more stages (not shown).

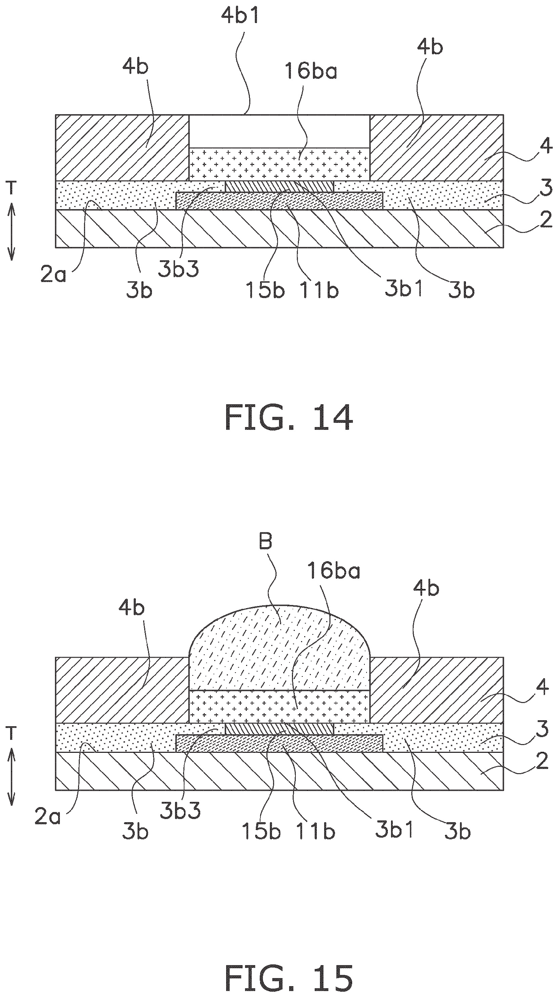

In the sensor 1 containing different reagents in the plurality of working electrodes 10a and 10b, which is manufactured by the method according to the preferred embodiment described above, the second reagent layer 15b of the second working electrode 10b contains a second reagent containing lactate oxidase or lactate dehydrogenase, for example. In this embodiment, the protective film 16b of the second working electrode 10b includes, as shown in Fig. 16, a second protective film 16ba that is disposed on the second reagent layer 15b and contains a polymer compound containing a cation exchange functional group, and a third protective film 16bb that is disposed on the second protective film 16ba and contains a polymer compound containing 4-vinylpyridine as a structural unit. This protective film 16b including the second protective film 16ba and the third protective film 16bb may be formed as follows:
after the formation of the second reagent layer 15b of the second working electrode 10b, a droplet of the second liquid composition B containing a polymer compound having a cation exchange functional group in alcohol is formed in the second opening 4b1 of the second insulating layer 4b of the second working electrode 10b, and is then dried to form the second protective film 16ba (see Figs. 13 and 14 ); and
after the second protective film 16ba is formed, a droplet of a third liquid composition B containing a polymer compound containing 4-vinylpyridine as a structural unit in alcohol is formed in the second opening 4b1 of the second insulating layer 4b of the second working electrode 10b, and is then dried to form a third protective film 16bb (see Figs. 15 and 16).

With the method according to this embodiment, the protective films 16a and 16b of the plurality of working electrodes 10a and 10b can be formed individually and independently, so the protective films 16a and 16b of different compositions optimized according to the reagent layers 15a and 15b can be easily formed on the plurality of working electrodes 10a and 10b containing different reagents, by adjusting the composition of the liquid composition B containing a protective film component in alcohol,.

The method according to this embodiment may comprise a step of forming the reference electrode 20. The step of forming the reference electrode 20 may include the following steps:
in the substrate 2 on which the reference electrode conductive layer 21, the first insulating layer 3, and the second insulating layer 4 are disposed as shown in Fig. 3, a silver/silver chloride layer 22 is disposed on the reference electrode conductive layer 21 in the reference electrode first opening 301 of the first insulating layer 3 (Fig. 4); and
after the disposition of the silver/silver chloride layer 22, the reference electrode protective film 23 is disposed on the silver/silver chloride layer 22 in the reference electrode second opening 401 of the second insulating layer 4 of the substrate 2 (Figs. 5 and 22).

### Working Example of First Disclosure

A sensor 1 having the configuration shown in Fig. 5 was manufactured as a working example of the first disclosure. The materials used for the sensor 1 having the configuration shown in Fig. 5 and the method for manufacturing this sensor are outlined below.

### Substrate

A substrate made of polyethylene terephthalate, having a thickness of 188 µm, and having the shape shown in Fig. 1, etc., was used as the insulating substrate 2.

### Conductive Layer

Carbon paste was applied to the first surface 2a of the insulating substrate 2 and heated at 140°C for 1 hour to form the conductive layers 11a and 11b of the working electrodes 10a and 10b, the reference electrode conductive layer 21, the counter electrode conductive layer 31, and the wiring 50 electrically connected to each of these, each having the shape shown in Fig. 1, from a carbon conductive layer with a thickness of 5 µm.

### First Insulating Layer

Then, as shown in Fig. 2, the first insulating layer 3 was laminated on the first surface 2a of the substrate 2 on which the carbon conductive layer was disposed, the first insulating layer 3 being made of a fluororesin containing a copolymer of vinylidene fluoride and hexafluoropropylene, covering the first surface 2a of the substrate 2 and the carbon conductive layer, and having a thickness of 5 µm in the portion that is on the carbon conductive layer. The first insulating layer 3 was formed by applying a composition that forms this fluororesin upon curing to the first surface 2a of the substrate 2 on which the carbon conductive layer was disposed, and heating at 140°C for 1 hour.

The first opening 3a1 of the first insulating layer 3a of the first working electrode 10a was circular, with a diameter of 1.2 mm. The first opening 3b1 of the first insulating layer 3b of the second working electrode 10b was circular, with a diameter of 1.4 mm. The reference electrode first opening 301 was circular, with a diameter of 1.1 mm, and the counter electrode first opening 302 was rectangular and measured 1.8 × 2.1 mm.

The water contact angle of the surface of the first insulating layer 3 (including the surfaces 3a2 and 3b2 of the first insulating layers 3a and 3b of the working electrodes 10a and 10b) was 134.2°. The water contact angle was measured using a handy contact angle/surface free energy analyzer MSA manufactured by Krüss, by discharging a 2-µL droplet of water onto the measurement surface at 20°C, and measuring the contact angle between the droplet and the surface after 2 seconds.

### Second Insulating Layer

Then, as shown in Fig. 3, a composition containing a polyester resin and a fluorine-based surface modification additive containing a perfluoroalkyl group in a solvent was applied to the first insulating layer 3, and heated at 140°C for 1 hour to laminate the second insulating layer 4 having a thickness of 40 µm.

The second opening 4a1 of the second insulating layer 4a of the first working electrode 10a and the second opening 4b1 of the second insulating layer 4b of the second working electrode 10b were each circular, with a diameter of 2 mm. The reference electrode second opening 401 was circular, with a diameter of 2 mm, and the counter electrode second opening 402 was rectangular and measured 1.8 × 2.1 mm.

The contact angle of the surface of the second insulating layer 4 (including the surfaces 4a2 and 4b2 of the second insulating layers 4a and 4b of the working electrodes 10a and 10b) to ethanol was 60.7°. The contact angle to ethanol was measured using a Handy Contact Angle/Surface Free Energy Analyzer MSA manufactured by Krüss by discharging a 2-µL droplet of ethanol onto the measurement surface at 20°C, and measuring the contact angle between the droplet and the surface after 2 seconds.

### First Working Electrode (for Glucose Measurement) Reagent Layer

A 0.4-µL droplet of a first liquid composition A containing a sodium phosphate buffer solution (pH 7.4), a carbon black dispersion, a polymer-bound mediator, glucose oxidase, and a crosslinking agent in water was formed in the first opening 3a1 of the first insulating layer 3a of the first working electrode 10a, and then dried to form the first reagent layer 15a containing the glucose oxidase and the mediator.

At this time, the droplet of the first liquid composition A did not overflow from the first opening 3a1, and the first reagent layer 15a after drying was formed only on the inside of the first opening 3a1 (see Figs. 7 and 8).

### Second Working Electrode (for Lactic Acid Measurement) Reagent Layer

A 0.6-µL droplet of a second liquid composition A containing a carbon black dispersion, hydroxypropyl cellulose, a polymer-bound mediator, lactate oxidase, polyimidazole, poly-L-lysine, and a crosslinking agent in water was formed in the first opening 3b1 of the first insulating layer 3b of the second working electrode 10b, and dried to form the second reagent layer 15b containing lactate oxidase and the mediator.

At this time, the droplet of second liquid composition A did not overflow from first opening 3b1, and second reagent layer 15b after drying was formed only on the inside of the first opening 3b1 (see Figs. 7 and 8).

### First Working Electrode Protective film (for Glucose Measurement)

The following reagents were mixed with ethanol to give the following final concentrations, and were reacted for about 1 hour to prepare a first liquid composition B (P4VP-tBuMA polymer dispersion).
- P4VP-tBuMA (Mn of poly-4-vinylpyridine: 74,000, Mn of poly-tert-butyl methacrylate: 87,000, Mw/Mn: 1.16, manufactured by NARD), final concentration 3.55 wt%
- Random copolymer of tripropylene glycol methyl ether methacrylate, styrene, and 4-vinylpyridine (manufactured by NARD), final concentration 4.45 wt%
- PEGDGE (poly(ethylene glycol) diglycidyl ether, Mn: 1000, manufactured by Sigma-Aldrich), final concentration 7 mM

A 0.65-µL droplet of the first liquid composition B was formed in the second opening 4a1 of the second insulating layer 4a of the first working electrode 10a and dried, and then another 0.65-µL droplet of the first liquid composition B was formed and dried to form the first protective film 16a (see Fig. 12).

At this time, the droplets of the first liquid composition B did not overflow from the second opening 4a1, and the first protective film 16a after drying was formed only on the inside of the second opening 4a1 (see Figs. 9, 10, 11 and 12).

### Second Working Electrode Protective Film (for Lactic Acid Measurement)

6472.79 mg of ethanol (manufactured by FUJIFILM Wako Pure Chemical Industries, Ltd.) and 1226.46 mg of 5 mol/L sodium hydroxide aqueous solution (manufactured by FUJIFILM Wako Pure Chemical Industries, Ltd.) were added to 23,093.67 mg of 21.5 wt% Nafion (registered trademark) dispersion (manufactured by Sigma-Aldrich), the pH of the dispersion was adjusted (neutralization of cation exchange groups), and the precipitate was dissolved with a vortex mixer to prepare 30,792.92 mg of 16.12 wt% Nafion (registered trademark) dispersion. The 16.12 wt% Nafion (registered trademark) dispersion thus obtained was termed the second liquid composition B. This second liquid composition B contained Nafion (registered trademark) in a solvent containing a lower alcohol.

A 0.60-µL droplet of the second liquid composition B was formed in the second opening 4b1 of the second insulating layer 4b of the second working electrode 10b and dried to form the second protective film 16ba (see Fig. 14).

At this time, the droplet of the second liquid composition B did not overflow from the second opening 4b1, and the second protective film 16ba after drying was formed only on the inside of the second opening 4b1 (see Figs. 13 and 14).

Then, the same composition as the above-mentioned first liquid composition B (P4VP-tBuMA polymer dispersion) was used in the following treatment as a third liquid composition B. That is, a 0.65-µL droplet of the third liquid composition B was formed in the second opening 4b1 of the second insulating layer 4b of the second working electrode 10b on which the second protective film 16ba was formed, and this was dried to form a third protective film 16bb (see Fig. 16).

At this time, the droplet of the third liquid composition B did not overflow from the second opening 4b1, and the third protective film 16bb after drying was formed only on the inside of the second opening 4b1 (see Figs. 15and 16).

### Reference Electrode

A silver/silver chloride paste was applied to the reference electrode conductive layer 21 within the reference electrode first opening 301 of the first insulating layer 3 and heated at 140°C for 1 hour to form the silver/silver chloride layer 22.

Then, the reference electrode protective film 23 was disposed on the silver/silver chloride layer 22 within the reference electrode second opening 401 of the second insulating layer 4 to form the reference electrode 20 (Fig. 22).

### Comparative Example

The sensor in this comparative example was produced in the same way as the sensor 1 in the working example given above, except that the second insulating layer was produced by the following procedure.

The first insulating layer 3 was coated with a composition that contained the same polyester resin as in the working example in a solvent, but did not contain a fluorine-based surface modification additive, and this was heated at 140°C for 1 hour to form a second insulating layer having a thickness of 38 µm.

The contact angle of the surface of the second insulating layer of the comparative sensor thus obtained to ethanol was 11.2°.

The following problem occurred in the formation of the protective films on the first and second working electrodes of the sensor in the comparative example.

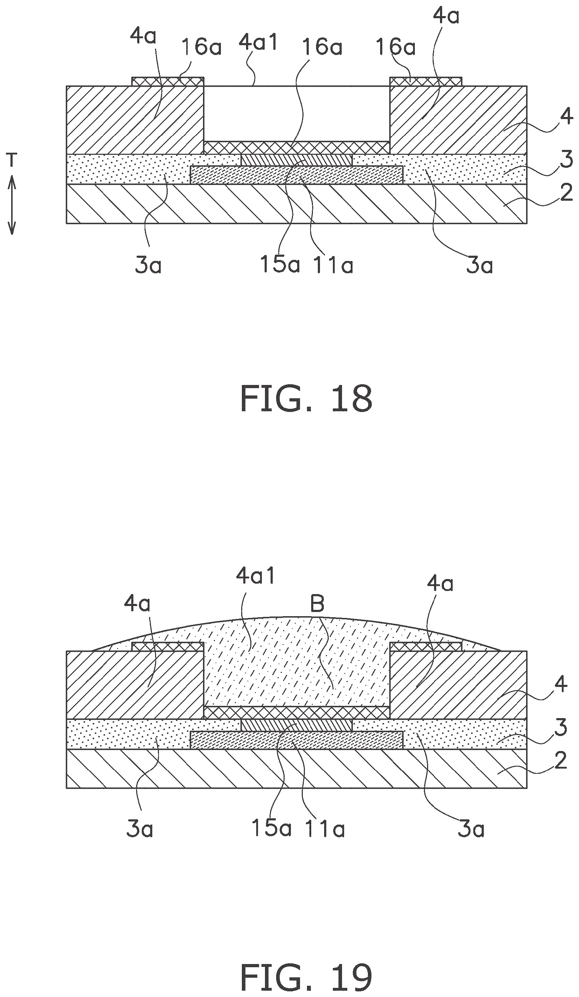

When a 0.65-µL droplet of the same first liquid composition B (P4VP-tBuMA polymer dispersion liquid) as that used in the working example was formed in the second opening 4a1 of the second insulating layer 4a of the first working electrode 10a of the sensor in the comparative example, the droplet did not remain in the second opening 4a1, and the first liquid composition B spread out and wetted the upper surface of the second insulating layer 4a as shown in Fig. 17. When this droplet was dried, as shown in Fig. 18, a part of the first protective film 16a was formed not only in the second opening 4a1, but also on the upper surface of the second insulating layer 4a. When this was dried and another 0.65-µL droplet of the first liquid composition B was formed, as shown in Fig. 19, the first liquid composition B spread out and wetted the upper surface of the second insulating layer 4a. When this was dried, as shown in Fig. 20, part of the first protective film 16a was formed not only in the second opening 4a1, but also on the upper surface of the second insulating layer 4a.

Although not depicted in the drawings, when a droplet of the same second liquid composition B (Nafion (registered trademark) dispersion) as that used in the working example was formed in the second opening 4b1 of the second insulating layer 4b of the second working electrode 10b of the sensor in the comparative example and dried, the second protective film 16ba was formed irregularly, not only in the second opening 4b1, but also on the upper surface of the second insulating layer 4b. Furthermore, when a droplet of the same third liquid composition B (P4VP-tBuMA polymer dispersion) as that used in the working example was formed and dried, the third protective film 16bb was formed irregularly, not only in the second opening 4b1, but also on the upper surface of the second insulating layer 4b.

### Glucose and Lactic Acid Measurement Experiment

### Culture Solution

A culture solution was prepared by adding MES (2-morpholinoethanesulfonic acid monohydrate) (manufactured by Dojindo Chemical Industries, Ltd.) and MOPS (3-morpholinopropanesulfonic acid) (manufactured by Dojindo Chemical Industries, Ltd.) as buffer components, each so as to reach a final concentration of 25 mM, to a solution of RPMI-1640 Medium (R1383, manufactured by Sigma-Aldrich), and further adjusting the glucose concentration to 30 mM, the lactic acid concentration to 15 mM, and the pH to 7.4.

### Current Measurement

The sensor 1 of the above working example and the sensor of the comparative example were each immersed in the above-mentioned culture solution, a voltage of 100 mV was applied to the first working electrode and the second working electrode relative to the reference electrode, and the current value between the first working electrode and the counter electrode and the current value between the second working electrode and the counter electrode were measured for about 11 days. The measurement was performed at N = 2.

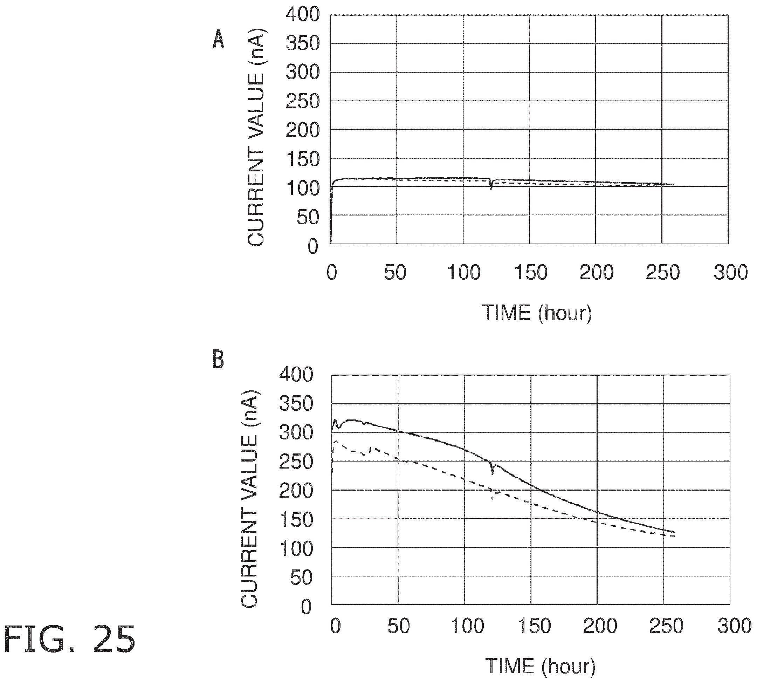

The measurement results for the current value of the first working electrode (for measuring glucose) of the sensor 1 of the working example are shown in Fig. 25A. The measurement results for the current value of the first working electrode (for measuring glucose) of the sensor of the comparative example are shown in Fig. 25B. The current value of the sensor 1 of the working example was stable, whereas the current value of the sensor of the comparative example decreased over time. With the sensor of the comparative example, it was difficult to control the thickness of the first protective film, and it was therefore surmised that the glucose permeability was high from the outset, and that the reagent including glucose oxidase leaked out.

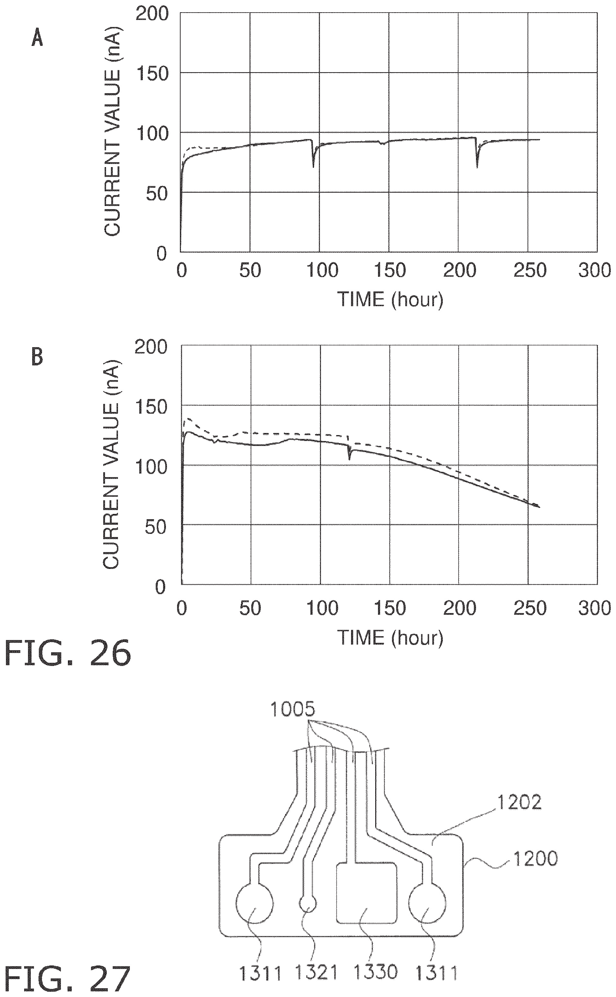

The measurement results for the current value of the second working electrode (for measuring lactic acid) of the sensor 1 of the working example are shown in Fig. 26A. The measurement results for the current value of the second working electrode (for measuring lactic acid) of the sensor of the comparative example are shown in Fig. 26B. The current value of the sensor 1 of the embodiment was stable, whereas the current value of the sensor of the comparative example decreased over time. With the sensor of the comparative example, it was difficult to control the thickness of the second protective film and the third protective film, and it was therefore surmised that the lactic acid permeability was high from the outset, and that the reagent including lactate oxidase leaked out.

### Embodiment of Second Disclosure

An embodiment of a sensor and a sensor unit according to the second disclosure will now be described. In this embodiment, some unnecessarily detailed description may be omitted. For example, detailed description of already known facts or redundant description of components that are substantially the same may be omitted. This is to avoid unnecessary repetition in the following description, and facilitate an understanding on the part of a person skilled in the art.

The applicant has provided the appended drawings and the following description so that a person skilled in the art might fully understand this disclosure, but does not intend for these to limit what is discussed in the following "Notes Regarding the Second Disclosure."

### Materials

Examples of materials that can be used in the sensor of the second disclosure of this specification are given below.

There are no particular limitations on the material of the insulating substrate of the sensor of the second disclosure of this specification, but the same material as the insulating substrate of the sensor in the first disclosure of this specification can be used, for example.

The conductive layers of the working electrode, reference electrode, and counter electrode of the sensor of the second disclosure of this specification are layers containing a conductive material such as carbon, gold, platinum, or palladium. These conductive layers can be produced by forming a layer of one of these conductive materials on the surface of the substrate by sputtering, vapor deposition, screen printing, or another such method. The conductive layers can be worked into a specific pattern by laser trimming as needed.

The wiring of the sensor of the second disclosure of this specification can be made of the same conductive material as that of the conductive layer.

The sensor of the second disclosure of this specification is used to detect a specific analyte in a liquid sample by immersing the sensor in the liquid sample. The liquid sample is preferably one that contains water as a solvent. Examples of the liquid sample include a cell culture medium and a liquid sample prepared using blood collected from a living organism. Examples of the analyte are as discussed in relation to the analyte detected with the sensor in the first disclosure of this specification.

The working electrode of the sensor of the second disclosure of this specification comprises a reagent layer containing a reagent that participates in an oxidation-reduction reaction. This reagent that participates in the oxidation-reduction reaction may be any reagent that will participate in an oxidation-reduction reaction of the analyte, and can be appropriately selected according to the analyte. The reagent that participates in the oxidation-reduction reaction may include a combination of an oxidoreductase and a mediator (electron transfer substance), or an oxidoreductase. The oxidoreductase may include a coenzyme.

Examples of oxidoreductase include oxidase and dehydrogenase. Specific examples of oxidoreductase are as discussed in relation to the sensor in the first disclosure of this specification.

Also, there are no particular limitations on the mediator, but examples are as discussed in relation to the sensor in the first disclosure of this specification.

The reagent layer of the working electrode of the sensor in the second disclosure of this specification may further contain, in addition to the reagent, a buffer, a hydrophilic polymer, a conductive carbon filler, a crosslinking agent, or other such components. Examples of the hydrophilic polymer and the conductive carbon filler are as discussed in relation to the sensor in the first disclosure of this specification.

The protective film of the working electrode of the sensor in the second disclosure of this specification can be a film that prevents or suppresses leakage of the reagent contained in the reagent layer to the outside of the protective film, and allows the analyte present outside the protective film to permeate. A protective film having such properties preferably contains a polymer compound. Examples of the polymer compound contained in the protective film include a polymer compound containing 4-vinylpyridine as a structural unit, and a polymer compound containing a cation exchange functional group.

Examples of the polymer compound containing 4-vinylpyridine as a constitutional unit and the polymer compound containing a cation exchange functional group are as discussed in relation to the sensor disclosed in the first disclosure of this specification.

The protective film of the working electrode of the sensor in the second disclosure of this specification can be a laminate of two or more protective films, examples of which are as discussed in relation to the sensor in the first disclosure of this specification.

Another preferred example of the protective film of the working electrode of the sensor in the second disclosure of this specification is a protective film that contains a polymer compound containing 4-vinylpyridine as a structural unit.

The reference electrode of the sensor in the second disclosure of this specification may have a protective film. The protective film of the reference electrode may be made of the same material as that described above for the protective film of the working electrode.

### Overview of Sensor 1101

A sensor 1101 according to an embodiment of the second disclosure will be described through reference to the drawings.

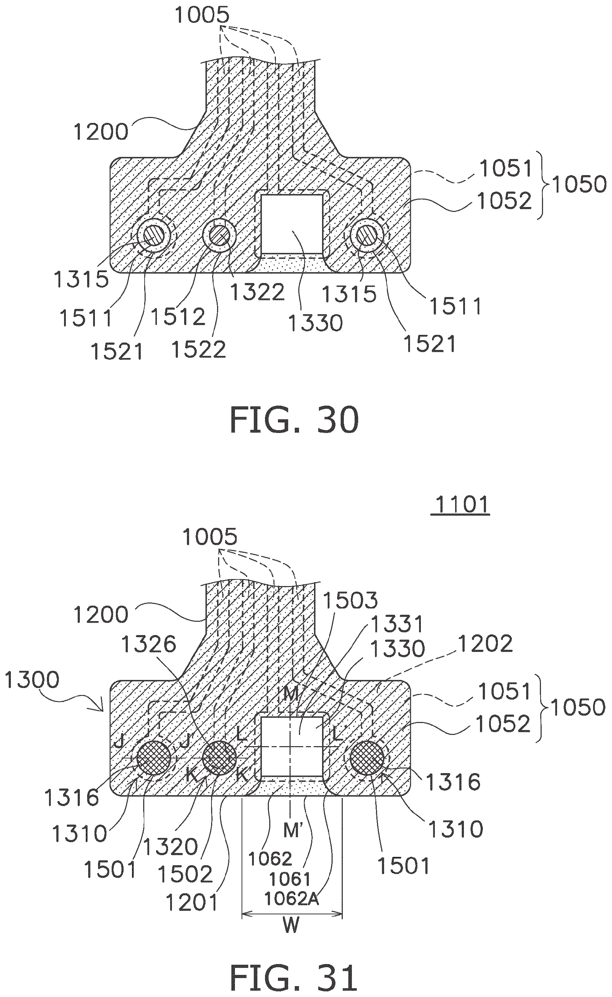

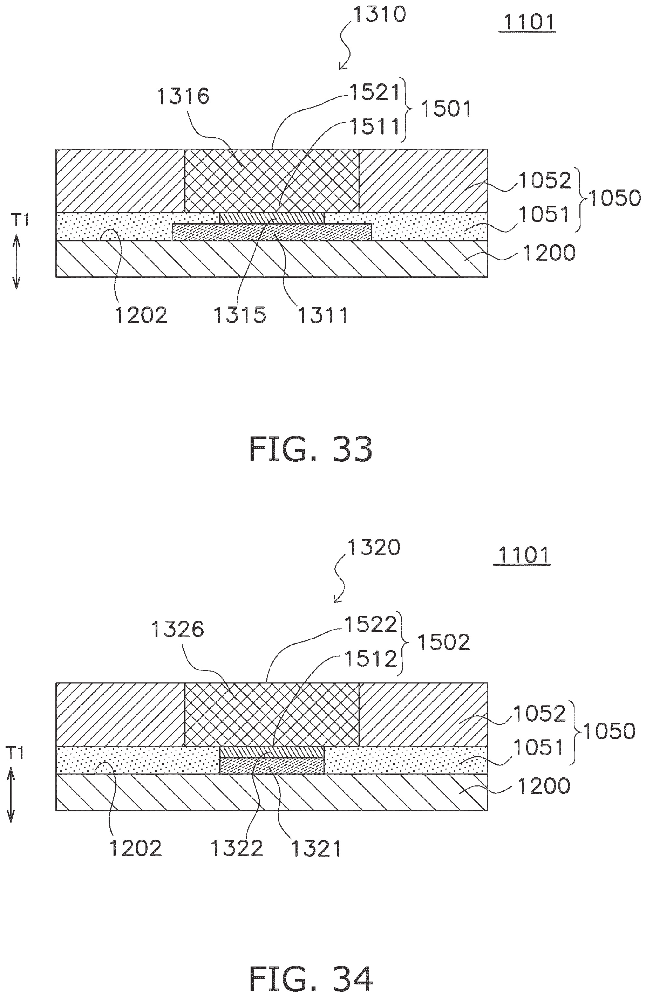

As shown in Fig. 31 and in Figs. 33, 34, 35, and 36, which are cross-sectional views of Fig. 31, the sensor 1101 in this embodiment comprises:
an insulating substrate 1200 that includes a distal end portion 1201 that is immersed in a liquid sample;
detection electrodes 1300 that are disposed near the distal end portion 1201 on a first surface 1202 of a substrate 1200, and include a working electrode 1310, a reference electrode 1320, and a counter electrode 1330;
wiring 1005 that is disposed on the first surface 1202 of the substrate 1200 and is connected to the detection electrodes 1300;
an insulating layer 1050 that is disposed on the first surface 1202 of the substrate 1200 and is formed so as to cover at least part of the detection electrodes 1300 and the wiring 1005;
a distal end opening 1061 that is formed in the insulating layer 1050, communicates with the upper surface 1331 of the counter electrode 1330, and opens on the distal end portion 1201 side of the substrate 1200; and
a flow channel 1062 that is formed in the insulating layer 1050, connects the distal end opening 1061 and the upper surface 1331 of the counter electrode 1330, and guides the liquid sample from the distal end opening 1061 to the upper surface 1331 of the counter electrode 1330.

The sensor 1101 in this embodiment includes two working electrodes 1310, but in another embodiment (not shown), there may be only one working electrode, or three or more working electrodes.

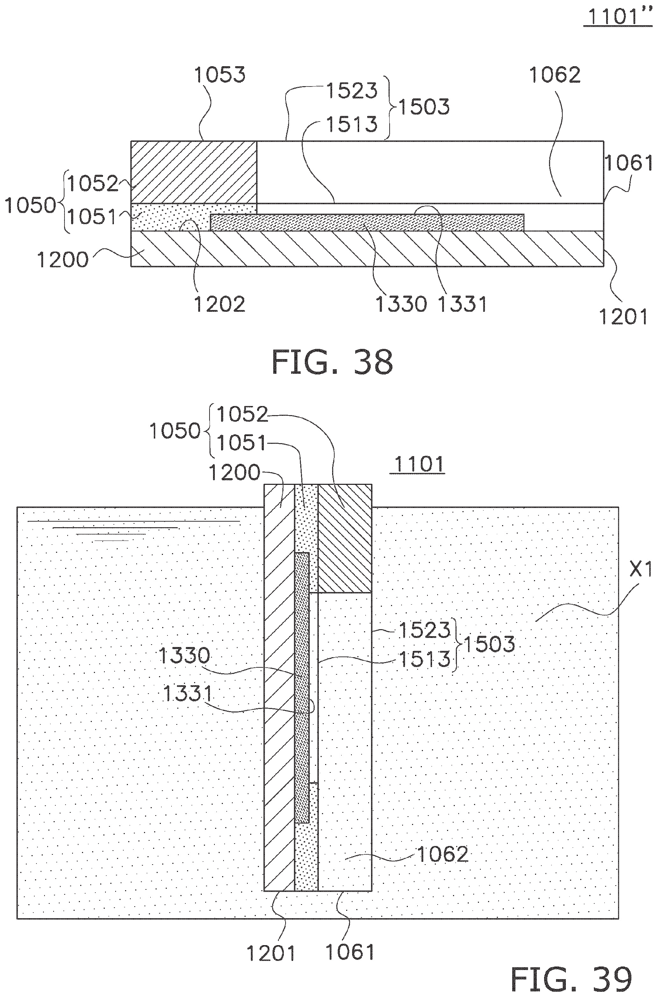

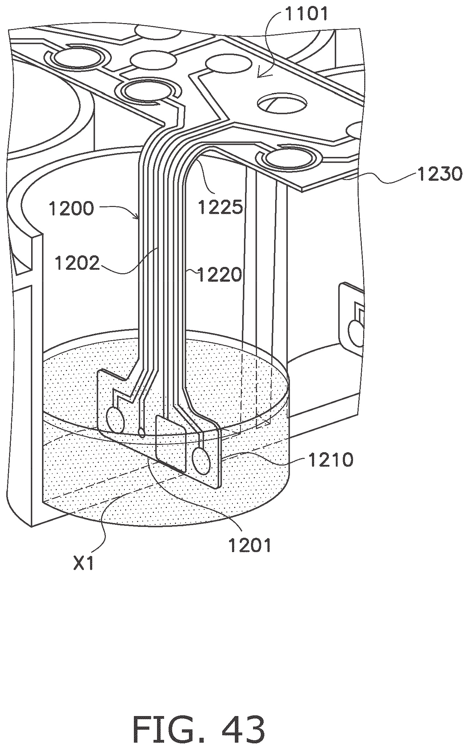

As shown in Figs. 39 and 43, the sensor 1101 is immersed in a liquid sample X1 and used to detect a specific analyte in the liquid sample X1. Specific examples of the liquid sample and the analyte are as discussed in the Materials section.

As shown in Fig. 33, the working electrode 1310 of the sensor 1101 includes a working electrode conductive layer 1311 and a reagent layer 1315. The reagent layer 1315 contains a reagent which participates in the redox reaction of the analyte in the liquid sample X1, and is disposed on the working electrode conductive layer 1311. A preferred embodiment of the reagent participating in the redox reaction is as discussed in the Materials section.

When the reagent layer 1315 of the working electrode 1310 contains a reagent that oxidizes the analyte in the liquid sample X1, electrons move from the analyte to the working electrode 1310 under conditions in which a specific voltage is applied to the detection electrodes 1300 of the sensor 1101. Similarly, when the reagent layer 1315 contains a reagent that reduces the analyte in the liquid sample X1, electrons move from the working electrode 1310 to the analyte. Since the amount of electron movement depends on the concentration of the analyte, the concentration, or change in concentration, of the analyte in the liquid sample X1 can be measured on the basis of the current value, or change in the current value, flowing through the working electrode 1310 of the sensor 1101.

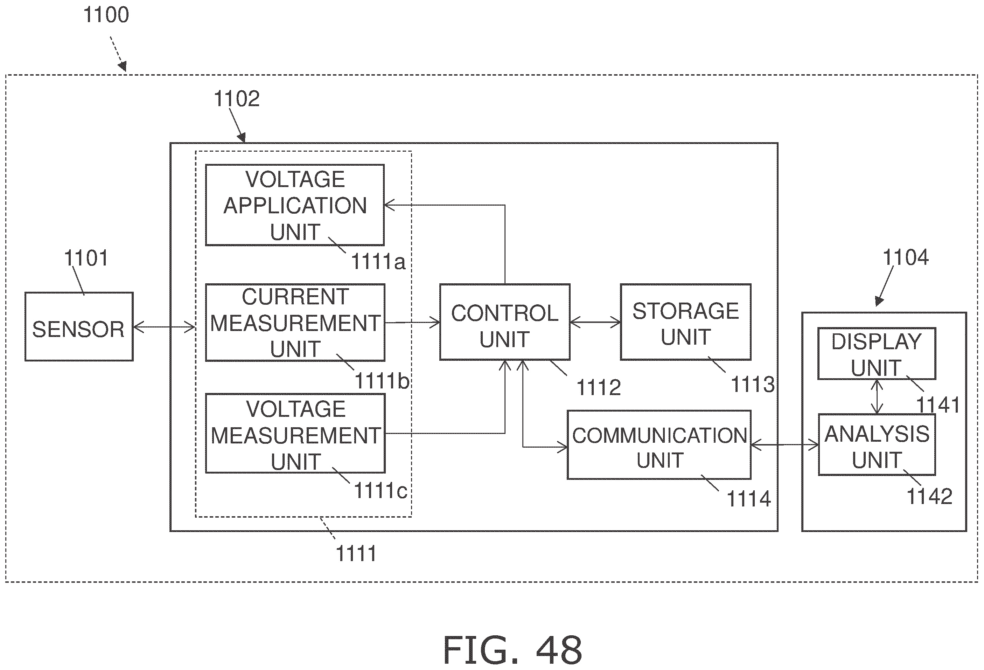

An example of an analysis device 1100 for analyzing an analyte in a liquid sample, which includes a sensor 1101, will be described with reference to Fig. 48.

As shown in Fig. 48, the analysis device 1100 comprises the sensor 1101, an analyzer 1102, and a controller 1104. The detection electrodes 1300, including the working electrode 1310, the reference electrode 1320, and the counter electrode 1330 of the sensor 1101, are each connected to the analyzer 1102 via wiring 1005. The analyzer 1102 is capable of communicating with the controller 1104.

The analyzer 1102 comprises an electrochemical measurement unit 1111, a control unit 1112, a memory unit 1113, and a communication unit 1114.

The electrochemical measurement unit 1111 is a potentiostat that applies a specific voltage to the detection electrodes 1300 of the sensor 1101 to measure the concentration of the analyte, and includes a voltage application unit 1111a and a current measurement unit 1111b, and preferably further includes a voltage measurement unit (counter electrode terminal voltage measurement unit) 1111c.

The voltage application unit 1111a applies a specific voltage to the detection electrodes 1300 of the sensor 1101 in order to measure the concentration of the analyte contained in the liquid sample X1.

The current measurement unit 1111b detects the current flowing between the working electrode 1310 and the counter electrode 1330 of the sensor 1101, or a change in the current, which is measured in a state in which a voltage is applied from the voltage application unit 1111a to the detection electrodes 1300 of the sensor 1101. As discussed above, the current value or change in the current value sensed by the current measurement unit 1111b is an index of the concentration or change in concentration of the analyte in the liquid sample X1.

The voltage measurement unit 1111c measures the terminal voltage of the counter electrode 1330 of the sensor 1101.

The control unit 1112 is connected to the voltage application unit 1111a, the current measurement unit 1111b, the voltage measurement unit 1111c, the memory unit 1113, and the communication unit 1114. The control unit 1112 controls the voltage application unit 1111a so as to apply a specific voltage to the detection electrodes 1300 of the sensor 1101, and controls the communication unit 1114 so as to transmit the measurement results from the current measurement unit 1111b and the voltage measurement unit 1111c to the controller 1104.

The memory unit 1113 is connected to the control unit 1112 and stores data such as the value of the applied voltage preset for each measurement object, the measurement values from the current measurement unit 1111b and the voltage measurement unit 1111c, and a pre-measured calibration curve.

The communication unit 1114 is controlled by the control unit 1112 and transmits the measurement results from the current measurement unit 1111b and the voltage measurement unit 1111c and other such data to the analysis unit 1142 of the controller 1104.

The controller 1104 is capable of communicating with the analyzer 1102 via a communication unit 1114, and is provided with a display unit 1141 and an analysis unit 1142.

The display unit 1141 displays the concentration of the analyte in liquid sample X1 from the current value sensed by the current measurement unit 1111b, as the result of the analysis in analysis unit 1142, for example.

The analysis unit 1142 is, for example, a PC (personal computer), and calculates the concentration of the analyte on the basis of the current flowing between the working electrode 1310 and the counter electrode 1330, as measured by the current measurement unit 1111b.

### Structure of Sensor 1101

The structure of the sensor 1101 in this embodiment will now be described, mainly with reference to Fig. 31 and to Figs. 33, 34, 35, and 36, which are cross-sectional views of Fig. 31.

The substrate 1200 of the sensor 1101 in this embodiment includes the distal end portion 1201 that is immersed in the liquid sample. The substrate 1200 may be in the form of a flat plate. The distal end portion 1201 is the portion of the peripheral edge of the substrate 1200 that becomes the distal end when the sensor 1101 is immersed in the liquid sample X1 (see Fig. 43). A preferred embodiment of the substrate 1200 is as discussed in the Materials section.

In the sensor 1101, the detection electrodes 1300 including the working electrode 1310, the reference electrode 1320, and the counter electrode 1330 are disposed near the distal end portion 1201 on the first surface 1202 of the substrate 1200.

The wiring 1005 of the sensor 1101 is disposed on the first surface 1202 of the substrate 1200, and is electrically connected to each electrode (working electrode 1310, reference electrode 1320, and counter electrode 1330) of the detection electrodes 1300. Preferred embodiments of the material of the wiring 1005 are as discussed in the Materials section.

The insulating layer 1050 of the sensor 1101 is disposed on the first surface 1202 of the substrate 1200 and is formed so as to cover at least part of the detection electrodes 1300 and the wiring 1005.

In the example depicted in the drawings, the insulating layer 1050 includes a first insulating layer 1051 disposed on the first surface 1202 of the substrate 1200, and a second insulating layer 1052 disposed on the first insulating layer 1051. However, in another embodiment (not shown), the insulating layer 1050 may be just one layer, or may include three or more layers.

The insulating layer 1050 may be any layer with insulating properties, and there are no particular limitations on the material, but it can be an insulating layer containing, for example, an insulating cured resin, or an insulating layer containing an insulating sheet, or may be a combination of plurality of insulating layers.

An insulating layer containing an insulating cured resin can be formed by curing a curable resin composition. This curable resin composition can be a composition that is polymerized and/or crosslinked by irradiation with active energy rays to produce an insulating cured resin. Examples of active energy rays include ultraviolet rays, electron beams, X-rays, infrared rays, and visible light, with ultraviolet rays or an electron beam being preferable. The curable resin composition can be, for example, a negative resist composition. The insulating cured resin that can be formed by curing a curable resin composition is preferably a fluororesin. This fluororesin can be any resin containing a polymer containing fluorine atoms on the main chain carbon and/or side chain, and examples thereof include poly(meth)acrylate resins and polyester resins containing fluorine atoms on the main chain carbon and/or a side chain. There are no particular limitations on thickness of the insulating layer containing an insulating cured resin, but this thickness can be 1 µm or more and 50 µm or less, for example.

The insulating layer including the insulating sheet may include an insulating sheet and a bonding layer including a pressure-sensitive adhesive or an adhesive agent that is disposed on one side of the insulating sheet. The insulating layer including the insulating sheet can be formed by affixing an insulating sheet including a bonding layer to the first surface 1202 of the substrate 1200, either directly or via another insulating layer. The insulating sheet can be an insulating resin sheet, examples of which include a polyethylene terephthalate sheet. The bonding layer can be, for example, a double-sided tape or a layer of a pressure-sensitive adhesive or an adhesive agent. The outer surface of the insulating sheet is preferably a water-repellent surface, and may be a water-repellent surface produced, for example, by applying a water-repellent coating or by performing surface texturing. There are no particular limitations on the thickness of the insulating layer including the insulating sheet, but the thickness of the insulating sheet may be, for example, 10 µm or more and 200 µm or less, and preferably 20 µm or more and 100 µm or less, and the thickness of the bonding layer disposed on one side of the insulating sheet may be, for example, 1 µm or more and 50 µm or less, and preferably 5 µm or more and 20 µm or less.

A preferred embodiment of the insulating layer 1050 includes a first insulating layer 1051 that is disposed on the first surface 1202 of the substrate 1200 and contains an insulating cured resin, and a second insulating layer 1052 that is disposed on the first insulating layer 1051 and contains an insulating sheet. In this embodiment, the second insulating layer 1052 can include an insulating sheet and a bonding layer that is disposed on the surface on the first insulating layer 1051 side of the insulating sheet, and is preferably affixed to the first insulating layer 1051 via the bonding layer. In this embodiment, there are no particular limitations on thickness of the first insulating layer 1051 containing an insulating cured resin, but the thickness of the portion of the detection electrodes 1300 that covers the wiring 1005 and the conductive layers (including working electrode conductive layer 1311, reference electrode conductive layer 1321, counter electrode 1330) can be 0.5 µm or more and 50 µm or less, for example, and is preferably 2 µm or more and 20 µm or less. An example of the thickness of the second insulating layer 1052 including an insulating sheet is given in the previous paragraph.

The working electrode 1310 of the sensor 1101 will be described through reference to the drawings, and particularly Fig. 33. Fig. 33 is a cross-sectional view along the J-J' line in Fig. 31 of the portion of the sensor 1101 shown in Fig. 31 that includes the working electrode 1310.

The working electrode 1310 includes a working electrode conductive layer 1311 that is disposed on the first surface 1202 of the substrate 1200, and a reagent layer 1315 that is disposed on the working electrode conductive layer 1311. The working electrode 1310 preferably further includes a working electrode protective film 1316 that is disposed on the reagent layer 1315 as shown in the drawings. Preferred embodiments of the material of the working electrode conductive layer 1311, the reagent layer 1315, and the working electrode protective film 1316 are given in the Materials section. Wiring 1005 is connected to the working electrode conductive layer 1311 of the working electrode 1310 (see Figs. 27 and 31).

Because the working electrode 1310 includes the working electrode protective film 1316, the following effect is exhibited. The working electrode protective film 1316 suppresses leakage of the reagent from the reagent layer 1315. Also, because the working electrode protective film 1316 is porous, the liquid sample X1 can permeate through it. This means that when the sensor 1101 is immersed in the liquid sample X1, the liquid sample X1 can easily reach the working electrode 1310, making it less likely that the immersion of the liquid sample X1 in the working electrode 1310 will be hindered.

The insulating layer 1050 comprises a working electrode opening 1501 that is formed at a position overlapping at least a part of the working electrode 1310 in plan view from the thickness direction T1 of the substrate 1200, and goes through in the thickness direction T1. In the example shown in the drawings, the working electrode opening 1501 is located on the working electrode conductive layer 1311, and the reagent layer 1315 and the working electrode protective film 1316 are disposed in the working electrode opening 1501. Disposing the working electrode protective film 1316 in the working electrode opening 1501 more effectively suppresses leakage of the reagent from the reagent layer 1315. The outer peripheral edges of the reagent layer 1315 and the working electrode protective film 1316 are defined by the inner peripheral edge of the working electrode opening 1501. The shape of the inner peripheral edge of the working electrode opening 1501 of the insulating layer 1050 in plan view from the thickness direction T1 is circular in the example shown in the drawings, but this is not the only option, and the shape may be polygonal (rectangular, triangular, etc.) or any other shape. There are no particular limitations on the opening width (width of the widest portion) of the working electrode opening 1501 of the insulating layer 1050, but this width can be, for example, 0.5 mm or more and 10 mm or less, and is preferably 1 mm or more and 5 mm or less.

In the example shown in the drawings, the working electrode opening 1501 of the insulating layer 1050 is made up of the working electrode first opening 1511 of the first insulating layer 1051, and the working electrode second opening 1521 of the second insulating layer 1052. The working electrode first opening 1511 is formed at a position overlapping a part of the working electrode conductive layer 1311 in plan view from the thickness direction T1 of the first insulating layer 1051, and goes through in the thickness direction T1. The working electrode second opening 1521 is formed at a position of the second insulating layer 1052 overlapping the portion of the first insulating layer 1051 that entirely encompasses the working electrode first opening 1511 in plan view from the thickness direction T1, and goes through in the thickness direction T1. The reagent layer 1315 is disposed in the working electrode first opening 1511 of the first insulating layer 1051, and includes the outer peripheral edge defined by the inner peripheral edge of the working electrode first opening 1511. The working electrode protective film 1316 is disposed in the working electrode second opening 1521 of the second insulating layer 1052, and includes the outer peripheral edge defined by the inner peripheral edge of the working electrode second opening 1521. The shapes of the inner periphery of the working electrode first opening 1511 of the first insulating layer 1051 and the working electrode second opening 1521 of the second insulating layer 1052 in plan view from the thickness direction T1 are circular in the example shown in the drawings, but this is not the only option, and each shape may be polygonal (rectangular, triangular, etc.) or any other shape. There are no particular limitations on the opening width (width of the widest part) of the working electrode first opening 1511 of the first insulating layer 1051, but this width may be, for example, 0.5 mm or more and 5 mm or less, and is preferably 1 mm or more and 2 mm or less. There are no particular limitations on the opening width (width of the widest part) of the working electrode second opening 1521 of the second insulating layer 1052, but this width may be, for example, 0.5 mm or more and 10 mm or less, and is preferably 1 mm or more and 5 mm or less.

The reference electrode 1320 of the sensor 1101 will now be described through reference to the drawings, and particularly Fig. 34. Fig. 34 is a cross-sectional view of the portion of the sensor 1101 in Fig. 31 that includes the reference electrode 1320, along the K-K' line in Fig. 31.

The reference electrode 1320 includes a reference electrode conductive layer 1321 that is disposed on the first surface 1202 of the substrate 1200, and a silver/silver chloride layer 1322 that is disposed on the reference electrode conductive layer 1321. The reference electrode 1320 preferably further includes a reference electrode protective film 1326 that is disposed on the silver/silver chloride layer 1322 as shown in the drawings. Preferred embodiments of the materials of the reference electrode conductive layer 1321 and the reference electrode protective film 1326 are as given in the Materials section. Wiring 1005 is connected to the reference electrode conductive layer 1321 of the reference electrode 1320 (see Figs. 27 and 31).

Because the reference electrode 1320 includes the reference electrode protective film 1326, the following effect is exhibited. The reference electrode protective film 1326 suppresses the leakage of silver from the silver/silver chloride layer 1322. Also, since the reference electrode protective film 1326 is porous, the liquid sample X1 can permeate through it. This means that when the sensor 1101 is immersed in the liquid sample X1, the liquid sample X1 can easily reach the reference electrode 1320, making it less likely that the immersion of the liquid sample X1 into the reference electrode 1320 will be hindered.

The insulating layer 1050 comprises a reference electrode opening 1502 that is formed at a position overlapping at least a part of the reference electrode 1320 in plan view from the thickness direction T1 of the substrate 1200, and goes through in the thickness direction T1. In the example shown in the drawings, part of the reference electrode conductive layer 1321, the silver/silver chloride layer 1322, and the reference electrode protective film 1326 are disposed in the reference electrode opening 1502. Disposing the reference electrode protective film 1326 in the reference electrode opening 1502 more effectively suppresses the leakage of silver from the silver/silver chloride layer 1322. The outer peripheral edges of the silver/silver chloride layer 1322 and the reference electrode protective film 1326 are defined by the inner peripheral edge of the reference electrode opening 1502. The shape of the inner periphery of the reference electrode opening 1502 of the insulating layer 1050 in plan view from the thickness direction T1 is circular in the example shown in the drawings, but this is not the only option, and this shape can be polygonal (rectangular, triangular, etc.) or any other shape. There are no particular limitations on the opening width (width of the widest portion) of the reference electrode opening 1502 in the insulating layer 1050, but this width can be, for example, 0.5 mm or more and 10 mm or less, and is preferably 1 mm or more and 5 mm or less.

In the example shown in the drawings, the reference electrode opening 1502 of the insulating layer 1050 is made up of a reference electrode first opening 1512 of the first insulating layer 1051 and a reference electrode second opening 1522 of the second insulating layer 1052. The reference electrode first opening 1512 is formed at a position overlapping a part of the reference electrode conductive layer 1321 in plan view from the thickness direction T1 of the first insulating layer 1051, and goes through in the thickness direction T1. The reference electrode second opening 1522 is formed at a position of the second insulating layer 1052 overlapping the part of the first insulating layer 1051 that entirely encompasses the reference electrode first opening 1512 in plan view from the thickness direction T1, and goes through in the thickness direction T1. A part of the reference electrode conductive layer 1321 and/or the silver/silver chloride layer 1322 is disposed in the reference electrode first opening 1512 of the first insulating layer 1051, and includes an outer peripheral edge that is defined by the inner peripheral edge of the reference electrode first opening 1512. The reference electrode protective film 1326 is disposed in the reference electrode second opening 1522 of the second insulating layer 1052, and includes an outer peripheral edge defined by the inner peripheral edge of the reference electrode second opening 1522. The shapes of the inner peripheral edges of the reference electrode first opening 1512 of the first insulating layer 1051 and the reference electrode second opening 1522 of the second insulating layer 1052 in plan view from the thickness direction T1 are circular in the example shown in the drawings, but this is not the only option, and may be polygonal (rectangular, triangular, etc.) or any other shape. There are no particular limitations on the opening width (width of the widest part) of the reference electrode first opening 1512 in the first insulating layer 1051, but this width can be, for example, 0.5 mm or more and 5 mm or less, and is preferably 1 mm or more and 2 mm or less. There are no particular limitations on the opening width (width of the widest part) of the reference electrode second opening 1522 in the second insulating layer 1052, but this width can be, for example, 0.5 mm or more and 10 mm or less, and is preferably 1 mm or more and 5 mm or less.

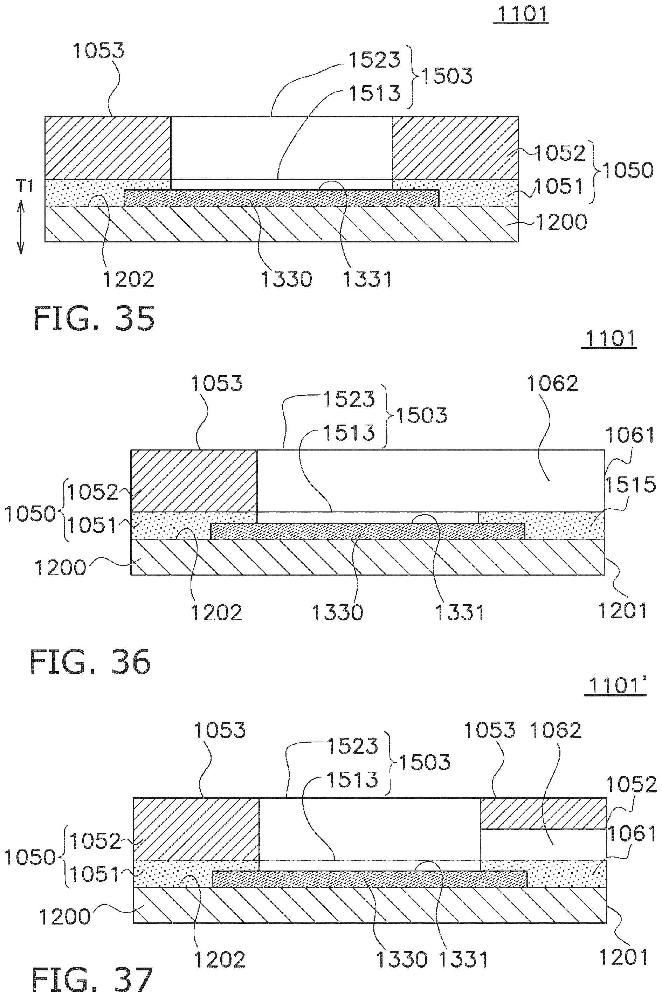

The counter electrode 1330 of the sensor 1101 will now be described through reference to the drawings, and particularly Fig. 35. Fig. 35 is a cross-sectional view of the portion of the sensor 1101 of Fig. 31 that includes the counter electrode 1330, along the L-L' line in Fig. 31.

The counter electrode 1330 is disposed on the first surface 1202 of the substrate 1200. In the embodiment shown in the drawings, the counter electrode 1330 consists entirely of a conductive layer. To specifically refer to the conductive layer portion of the counter electrode, this portion may be referred to as a "counter electrode conductive layer" or a "conductive layer of the counter electrode." Preferred embodiments of the material of the counter electrode 1330 are given in the Materials section as the material of the conductive layer of the counter electrode. Wiring 1005 is connected to the counter electrode 1330 (see Fig. 27 and Fig. 31).

With the sensor 1101, the insulating layer 1050 comprises a counter electrode opening 1503 that is formed at a position overlapping at least a part of the counter electrode 1330 in plan view from the thickness direction T1 of the substrate 1200, and goes through in the thickness direction T1, and the upper surface 1331 of the counter electrode 1330 is exposed through the counter electrode opening 1503. When the sensor 1101 thus configured is immersed in a liquid sample X1, the liquid sample X1 can come into contact with the upper surface 1331 of the counter electrode 1330 through the counter electrode opening 1503 in the insulating layer 1050. The shape of the inner peripheral edge of the counter electrode opening 1503 of the insulating layer 1050 in plan view from the thickness direction T1 is rectangular in the example in the drawings, but this is not the only option, and this shape can instead be circular, polygonal other than rectangular (such as triangular), or any other shape. There are no particular limitations on the opening width (width of the widest part) of the counter electrode opening 1503 of the insulating layer 1050, but this width can be, for example, 0.5 mm or more and 10 mm or less, and is preferably 1 mm or more and 5 mm or less. In the example shown in the drawings, a part of the upper surface 1331 of the counter electrode 1330 is exposed through the counter electrode opening 1503, but this is not the only option. For instance, the counter electrode opening 1503 may have a shape that entirely encompasses the counter electrode 1330, and the entire upper surface 1331 of the counter electrode 1330 may be exposed through the counter electrode opening 1503.

In the example shown in the drawings, the counter electrode opening 1503 of the insulating layer 1050 is made up of a counter electrode first opening 1513 of the first insulating layer 1051 and a counter electrode second opening 1523 of the second insulating layer 1052. The counter electrode first opening 1513 is formed at a position of the first insulating layer 1051 overlapping a part of the counter electrode 1330 in plan view from the thickness direction T1, and goes through in the thickness direction T1. The counter electrode second opening 1523 is formed at a position of the second insulating layer 1052 that coincides with the counter electrode first opening 1513 of the first insulating layer 1051 in plan view from the thickness direction T1, and goes through in the thickness direction T1. The counter electrode first opening 1513 of the first insulating layer 1051 and the counter electrode second opening 1523 of the second insulating layer 1052 do not necessarily have to coincide with each other, so long as they at least partially overlap. There are no particular limitations on the shape and opening width of the inner peripheral edges of the counter electrode first opening 1513 of the first insulating layer 1051 and the counter electrode second opening 1523 of the second insulating layer 1052, and these can be within the ranges given for the counter electrode opening 1503 of the insulating layer 1050, for example.

The distal end opening 1061 and the flow path 1062 of the sensor 1101 will now be described through reference to the drawings, and particularly Figs. 31 and 36. Fig. 36 is a cross-sectional view along the M-M' line in Fig. 31 of the portion of the sensor 1101 in Fig. 31 that includes the counter electrode 1330, the distal end opening 1061, and the flow path 1062.

The distal end opening 1061 is formed in the insulating layer 1050, communicates with the upper surface 1331 of the counter electrode 1330, and opens to the distal end portion 1201 side of the substrate 1200. The flow channel 1062 is formed in the insulating layer 1050, and connects the distal end opening 1061 and the upper surface 1331 of the counter electrode 1330. The flow channel 1062 guides the liquid sample X1 from the distal end opening 1061 to the upper surface 1331 of the counter electrode 1330. For example, the flow channel 1062 can guide the liquid sample X1 from the distal end opening 1061 to the upper surface 1331 of the counter electrode 1330 by capillary action.

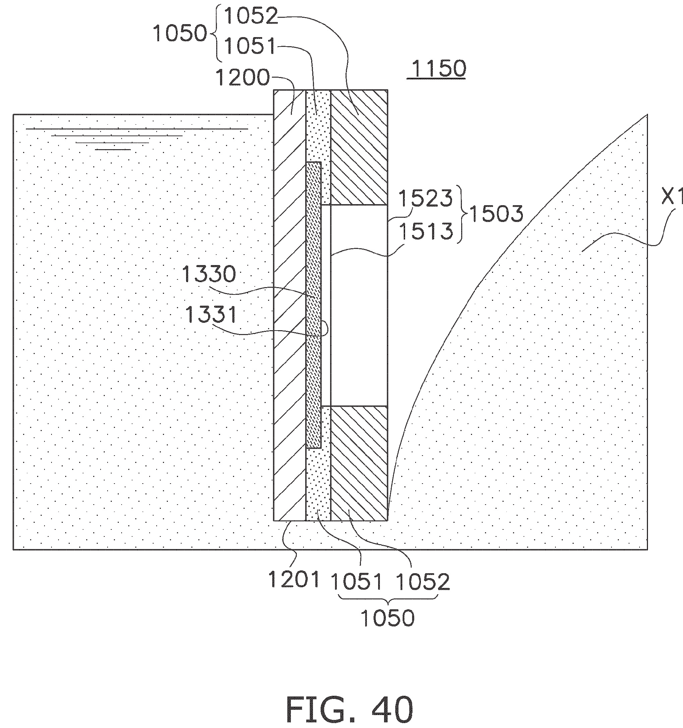

The functions of the distal end opening 1061 and the flow path 1062 of the sensor 1101 in the embodiment shown in Figs. 31 and 36 will be described through reference to Figs. 39 and 40. Fig. 39 is a schematic cross-sectional view, corresponding to Fig. 36, of the sensor 1101 in this embodiment, which is immersed in the liquid sample X1 from the distal end portion 1201 of the substrate 1200, and comprises the distal end opening 1061 and the flow path 1062. Fig. 40 is a schematic cross-sectional view of a sensor 1150 immersed in the liquid sample X1 in a comparative example, which has the same structure as the sensor 1101 of this embodiment except that there is no distal end opening 1061 or flow path 1062.

As shown in Fig. 39, when the sensor 1101 of this embodiment is immersed in the liquid sample X1 so that the distal end portion 1201 of the substrate 1200 is at the lower end, the liquid sample X1 is guided from the distal end opening 1061 through the flow path 1062 to the upper surface 1331 of the counter electrode 1330. As a result, the upper surface 1331 of the counter electrode 1330 of the sensor 1101 can be in good contact with the liquid sample X1. This is particularly favorable because the upper surface 1331 of the counter electrode 1330 of the sensor 1101 can be in good contact with the liquid sample X1 even if the liquid sample X1 is not very deep. Accordingly, the use of the sensor 1101 of this embodiment improves the measurement accuracy of the analyte in the liquid sample X1.

On the other hand, as shown in Fig. 40, with the sensor 1150 of the comparative example in which the distal end opening 1061 and the flow path 1062 are not formed in the insulating layer 1050, the region of the first surface 1202 of the substrate 1200 that is between the counter electrode 1330 and the distal end portion 1201 is covered by the insulating layer 1050. When the comparative sensor 1150 of the comparative example having this structure is immersed in the liquid sample X1 so that the distal end portion 1201 of the substrate 1200 is at the distal end, the liquid sample X1 tends to move away from the counter electrode 1330 due to the liquid repellency of the insulating layer 1050 between the counter electrode 1330 and the distal end portion 1201 of the substrate 1200. Accordingly, with the sensor 1150 of the comparative example, contact between the counter electrode 1330 and the liquid sample X1 tends to be hindered, and the analyte in the liquid sample X1 may not be measured properly. In particular, when the counter electrode 1330 is a carbon conductive layer, the upper surface 1331 of the counter electrode 1330 is highly water repellent, which makes the liquid sample X1 containing water as a solvent particularly prone to being hindered from making contact with the upper surface 1331 of the counter electrode 1330. This problem with the sensor 1150 of the comparative example can be solved by the sensor 1101 of this embodiment, in which the distal end opening 1061 and the flow path 1062 are formed in the insulating layer 1050.

The sensor 1101 of this embodiment shown in Figs. 31 and 36 comprises a distal end opening 1061 and a flow path 1062 formed in the portion of the insulating layer 1050 that is between the counter electrode 1330 and the distal end portion 1201 of the substrate 1200 in plan view from the thickness direction T1 of the substrate 1200. With the sensor 1101 of this embodiment, the flow path 1062 is connected to the counter electrode opening 1503 of the insulating layer 1050, and the upper surface 1331 of the counter electrode 1330 is exposed through the counter electrode opening 1503, so the effect whereby the upper surface 1331 of the counter electrode 1330 comes into contact with the liquid sample X1 when immersed in the liquid sample X1 is particularly pronounced.

The distal end opening 1061 and the flow channel 1062 of the sensor 1101 shown in Figs. 31 and 36 are recesses formed in the insulating layer 1050, in which the side where the substrate 1200 is present is closed and the side with the upper surface 1053 of the insulating layer 1050 is open, but this is not the only option. For example, as with a sensor 1101' according to another embodiment shown in Fig. 37, the structure may be such that the distal end opening 1061 and the flow channel 1062 are formed in a part of the thickness direction not including the upper surface 1053 of the insulating layer 1050, and the periphery of the distal end opening 1061 and the flow channel 1062 is surrounded when viewed in the direction going from the counter electrode 1330 to the distal end portion 1201 of the substrate 1200 (the M-M' direction in Fig. 31).

The flow channel 1062 of the sensor 1101 shown in Figs. 31 and 36 has an expansion section 1062A that expands the flow channel width W (see Fig. 31) toward the distal end opening 1061. Because the flow channel 1062 has the expansion section 1062A, the movement of the liquid sample X1 from the distal end opening 1061 to the upper surface 1331 of the counter electrode 1330 when the sensor 1101 is immersed in the liquid sample X1 is promoted. The flow channel width W here refers to the width of the flow channel 1062 in a direction (the L-L' direction in Fig. 31) perpendicular to the direction from the counter electrode 1330 to the distal end portion 1201 of the substrate 1200 (the M-M' direction in Fig. 31 ) in plan view of the sensor 1101 from the thickness direction T1 of the substrate 1200.

With the sensor 1101 shown in Figs. 31 and 36, the insulating layer 1050 includes a first insulating layer 1051 that is disposed on the first surface 1202 of the substrate 1200 and a second insulating layer 1052 that is disposed on the first insulating layer 1051, and the distal end opening 1061 and the flow path 1062 are formed in the second insulating layer 1052. In a particularly preferred embodiment of the sensor 1101 of this embodiment, the first insulating layer 1051 contains an insulating cured resin, and the second insulating layer 1052 includes an insulating sheet. The first insulating layer 1051 containing the insulating cured resin can be formed by coating the first surface 1202 of the substrate 1200 with a curable resin composition and then curing this coating by irradiation with active energy rays. The second insulating layer 1052 including the insulating sheet can be formed by affixing an insulating sheet via a bonding layer onto the first insulating layer 1051 containing the insulating cured resin. After the formation of the second insulating layer 1052 including an insulating sheet, the portion of the insulating sheet between the counter electrode 1330 and the distal end portion 1201 of the substrate 1200 can be peeled off to form the distal end opening 1061 and the flow path 1062 in the second insulating layer 1052. Alternatively, an insulating sheet having a notch formed in advance in the portion between the counter electrode 1330 and the distal end portion 1201 of the substrate 1200 can be attached via a bonding layer onto the first insulating layer 1051 containing an insulating cured resin to form the second insulating layer 1052 including an insulating sheet in which the distal end opening 1061 and the flow path 1062 have been formed.

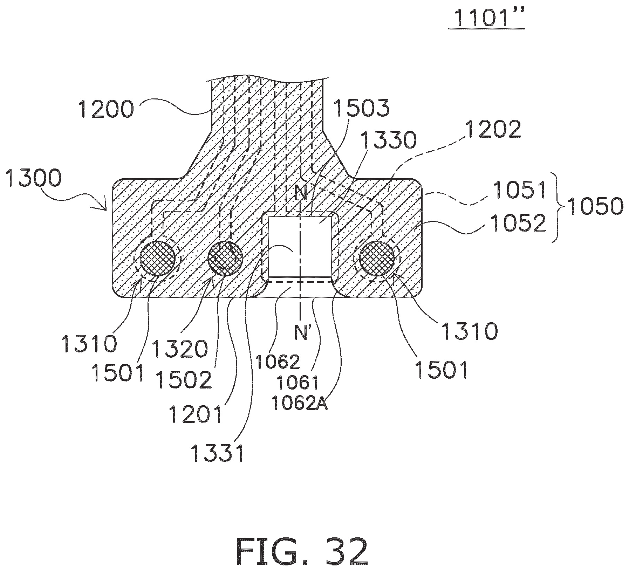

With a sensor 1101" in yet another embodiment shown in Figs. 32 and 38, a distal end opening 1061 and a flow path 1062 are further formed in the first insulating layer 1051 in the sensor 1101 shown in Figs. 31 and 36. The sensor 1101" shown in Figs. 32 and 38 is identical to the sensor 1101 shown in Figs. 31 and 36, except that a distal end opening 1061 and a flow path 1062 are further formed in the portion 1515 (see Fig. 36) of the first insulating layer 1051 that is between the counter electrode 1330 and the distal end portion 1201 of the substrate 1200. The sensor 1101" shown in Figs. 32 and 38 does not include an insulating layer between the counter electrode 1330 on the first surface 1202 of the substrate 1200 and the distal end portion 1201, and this further promotes contact between the liquid sample X1 and the counter electrode 1330 when the sensor 1101" is immersed in the liquid sample X1, which allows the analyte in the liquid sample X1 to be detected with even higher accuracy. In a particularly preferred embodiment of the sensor 1101" shown in Figs. 32 and 38, the first insulating layer 1051 contains an insulating cured resin, and the second insulating layer 1052 includes an insulating sheet. The sensor 1101" in this particularly preferred embodiment can be manufactured by first manufacturing the sensor 1101 comprising the insulating layer 1050 including the first insulating layer 1051 containing the insulating cured resin, and the second insulating layer 1052 including the insulating sheet by the procedure described above, and then peeling off the portion 1515 (see Fig. 36) of the first insulating layer 1051 containing the insulating cured resin. In another method for manufacturing the sensor 1101", when the curable resin composition is cured on the first surface 1202 of the substrate 1200 to form the first insulating layer 1051 containing the insulating cured resin, the first insulating layer 1051 lacking the portion 1515 is formed, and the second insulating layer 1052 is formed on this first insulating layer 1051.

### Method for Manufacturing Sensor 1101

An example of a method for manufacturing the sensor 1101 of the embodiment shown in Fig. 31 of the second disclosure will now be described with reference to the drawings, and particularly Figs. 27 to 31.

First, as shown in Fig. 27, working electrode conductive layers 1311, a reference electrode conductive layer 1321, and a counter electrode 1330 (counter electrode conductive layer), as well as wiring 1005 electrically connected to each of these, are disposed on the first surface 1202 of the insulating substrate 1200.

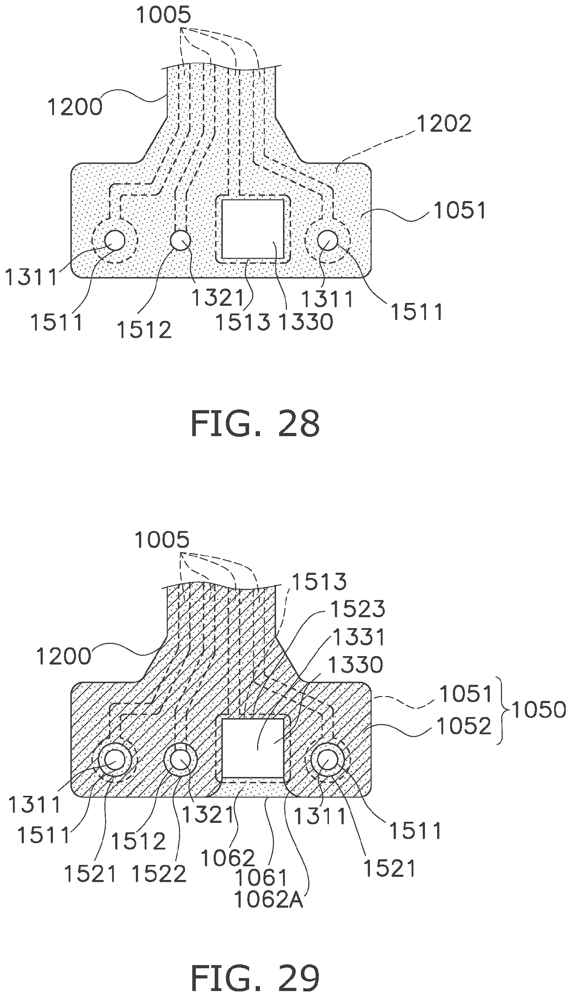

As shown in Fig. 28, a first insulating layer 1051 is laminated on the first surface 1202 of the substrate 1200 on which the working electrode conductive layers 1311, the reference electrode conductive layer 1321, the counter electrode 1330, and the wiring 1005 are disposed. The first insulating layer 1051 has working electrode first openings 1511at positions overlapping parts of the working electrode conductive layers 1311, a reference electrode first opening 1512 at a position overlapping part of the reference electrode conductive layer 1321, and a counter electrode first opening 1513 at a position overlapping part of the counter electrode 1330.

As discussed above, in a particularly preferred embodiment of the sensor 1101 of this embodiment, the first insulating layer 1051 contains an insulating cured resin. The first insulating layer 1051 containing an insulating cured resin can be formed by coating the first surface 1202 of the substrate 1200 with a curable resin composition, and then curing the coating by irradiating the coating with active energy rays.

Then, as shown in Fig. 29, the second insulating layer 1052 is further laminated on the first insulating layer 1051 of the substrate 1200 in Fig. 28. The second insulating layer 1052 has working electrode second openings 1521 having a shape that entirely encompasses the working electrode first openings 1511at positions of the first insulating layer 1051 that overlap the working electrode first openings 1511, a reference electrode second opening 1522 having a shape that entirely encompasses the reference electrode first opening 1512 at a position overlapping the reference electrode first opening 1512, and a counter electrode second opening 1523 having a shape that coincides with the counter electrode first opening 1513 at a position overlapping the counter electrode first opening 1513. The second insulating layer 1052 further has the distal end opening 1061 and the flow channel 1062 at the portion between the counter electrode 1330 and the distal end portion 1201 of the substrate 1200 in plan view from the thickness direction T1 of the substrate 1200.

As discussed above, in a particularly preferred embodiment of the sensor 1101 of this embodiment, the second insulating layer 1052 includes an insulating sheet. The second insulating layer 1052 including the insulating sheet may further include a bonding layer containing a pressure-sensitive adhesive or adhesive agent disposed on one side of the insulating sheet. The second insulating layer 1052 including the insulating sheet can be formed by affixing the insulating sheet to the first insulating layer 1051 via the bonding layer.

Then, as shown in Fig. 30, the reagent layer 1315 is disposed in the working electrode first openings 1511 of the first insulating layer 1051, and the silver/silver chloride layer 1322 is disposed in the reference electrode first opening 1512 of the first insulating layer 1051.

As shown in Fig. 31, the working electrode protective films 1316 are disposed in working electrode second openings 1521 of the second insulating layer 1052 to form the working electrodes 1310, and the reference electrode protective film 1326 is disposed in the reference electrode second opening 1522 of the second insulating layer 1052 to form the reference electrode 1320. There are no particular limitations on the order in which the layers in Figs. 30 and 31 are formed, and a different order may be used.

### Another Embodiment of Sensor 1101

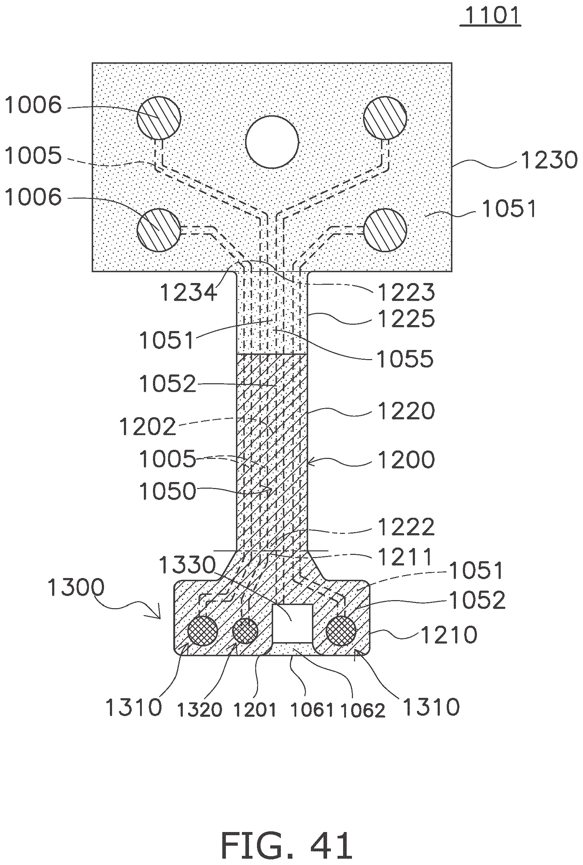

Another preferred embodiment of the sensor 1101 of the second disclosure will now be described with reference to Figs. 41 to 47.

In the sensor 1101 according to the embodiment shown in Fig. 41, the substrate 1200 comprises:
a main body portion 1210 that includes a distal end portion 1201 and a first end portion 1211 on the opposite side from the distal end portion 1201, and on which detection electrodes 1300 are disposed;
a connecting portion 1220 that includes a second end portion 1222 connected to the first end portion 1211 of the main body portion 1210, and a third end portion 1223 on the opposite side from the second end portion 1222, and that extends from the second end portion 1222 to the third end portion 1223; and
a proximal end portion 1230 that includes a fourth end 1234 connected to the third end 1223 of the connecting portion 1220, and
the connecting portion 1220 of the substrate 1200 includes a bending portion 1225 near a third end portion 1223, where the substrate 1200 is bent so that the first surface 1202 side of the substrate 1200 protrudes.

Figs. 41, 42, 46, and 47 are plan views of the sensor 1101 before the substrate 1200 has been bent at the bending portion 1225.

As shown in Fig. 41, the wiring 1005 is preferably disposed on the first surface 1202 of the substrate 1200, from the main body portion 1210 via the connecting portion 1220 to the proximal end portion 1230. As shown in Fig. 41, it is preferable if terminals 1006 are disposed on the first surface 1202 of the proximal end portion 1230, and each of the wirings 1005 electrically connects one of the detection electrodes 1300 on the first surface 1202 of the main body portion 1210 to one of the terminals 1006 on the first surface 1202 of the proximal end portion 1230.

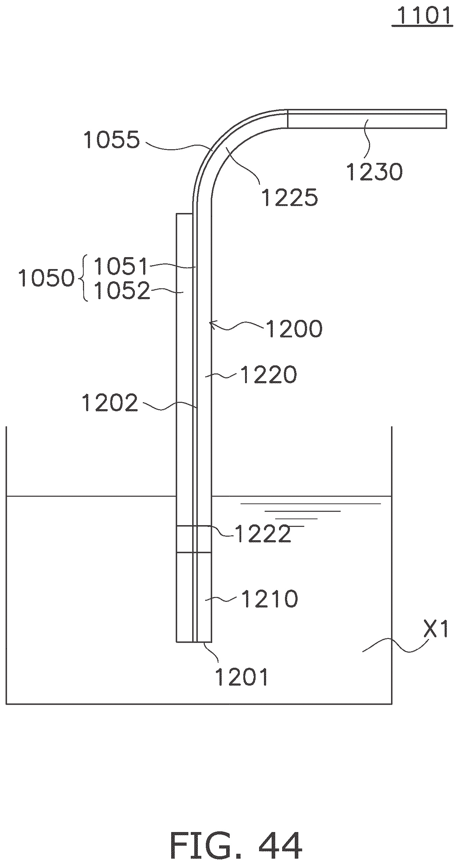

The substrate 1200 of the sensor 1101 shown in Fig. 41 is bent at the bending portion 1225 so that the side of the first surface 1202 on which the detection electrodes 1300 are disposed protrudes. The substrate 1200 bent at the bending portion 1225 has a shape in which the main body portion 1210 and the portion of the connecting portion 1220 closer to the second end 1222 side than the bending portion 1225 protrude from the proximal end portion 1230. Preferably, as shown in Figs. 43 and 44, the main body portion 1210 of the substrate 1200 and the portion of the connecting portion 1220 closer to the second end 1222 side than the bending portion 1225 protrude substantially perpendicularly with respect to the proximal end portion 1230 of the substrate 1200.

With the sensor 1101 shown in Fig. 41, which has the substrate 1200 bent at the bending portion 1225 of the connecting portion 1220, the main body portion 1210 of the substrate 1200 on which the detection electrodes 1300 are disposed can be oriented so that the distal end portion 1201 is at the bottom end, without having to tilt the proximal end portion 1230 of the substrate 1200 as shown in Figs. 43 and 44 (for example, in a state in which the proximal end portion 1230 of the substrate 1200 is held horizontally as shown in Figs. 43 and 44), allowing for immersion in the liquid sample X1 in order to measure the analyte.

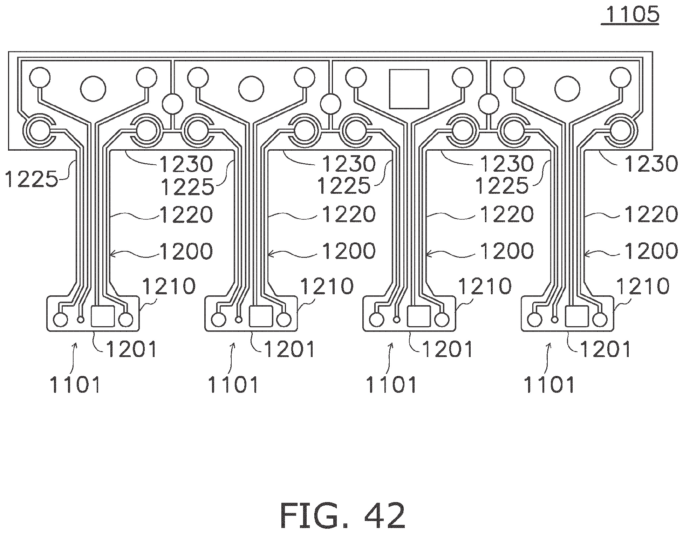

As shown in Fig. 42, a plurality of the sensors 1101 shown in Fig. 41 can be connected and integrated for use as a multiple sensor 1105. The multiple sensor 1105 shown in Fig. 42 is formed by integrating the proximal ends 1230 of the substrates 1200 of four sensors 1101.

With the sensor 1101 shown in Fig. 41, the insulating layer 1050 includes a second insulating layer 1052 that includes a first insulating sheet and is disposed in the region of the first surface 1202 of the substrate 1200 having the main body portion 1210 and the portion of the connecting portion 1220 that is closer to the second end 1222 than the bending portion 1225. Here, this portion of the connecting portion 1220 that is closer to the second end 1222 than the bending portion 1225 is the portion of the connecting portion 1220 extending from the end of the bending portion 1225 on the second end 1222 side to the second end 1222. With the sensor 1101 shown in Fig. 41, the insulating layer 1050 further includes the first insulating layer 1051 containing an insulating cured resin, which is disposed over the entire first surface 1202 of the substrate 1200. The second insulating layer 1052 including the first insulating sheet includes, in addition to the first insulating sheet, a bonding layer (not shown) containing a pressure-sensitive adhesive or adhesive agent, and is affixed to the above-mentioned region of the first surface 1202 of the substrate 1200 from the bonding layer side, via the first insulating layer 1051. The sensor 1101 shown in Fig. 41 is preferable because the portion immersed in the liquid sample X1 is integrally covered by the second insulating layer 1052 including the first insulating sheet, which affords the required insulation between the detection electrodes 1300 and the wiring 1005, and it is easy to form the distal end opening 1061 and the flow path 1062 in the first insulating sheet of the second insulating layer 1052.

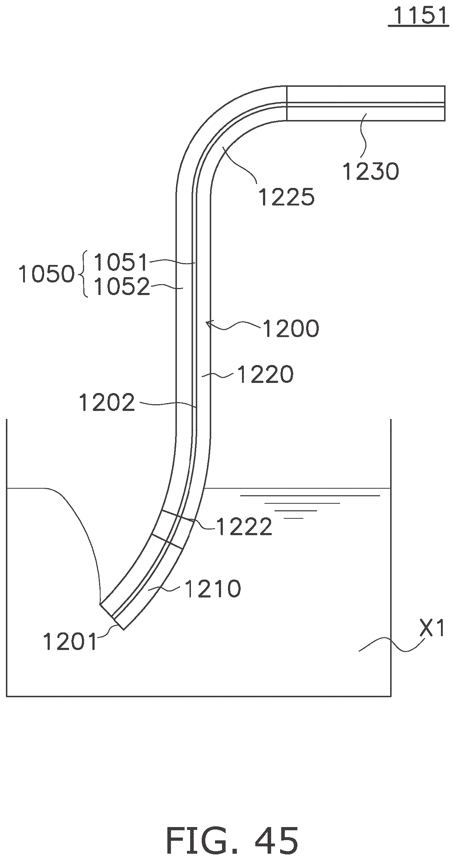

The insulating layer 1050 of the sensor 1101 shown in Fig. 41 includes a non-insulating region 1055 that does not include an insulating sheet, above the region of the bent portion 1225 of the connecting portion 1220 of the first surface 1202 of the substrate 1200. The effect of this configuration will be described with reference to Figs. 44 and 45.

Fig. 44 is a schematic side view of the sensor 1101 shown in Fig. 41, in which the main body portion 1210 including the distal end portion 1201 of the substrate 1200 is immersed in the liquid sample X1. As shown in Fig. 44, in the sensor 1101, the substrate 1200 is bent at the bending portion 1225 of the connecting portion 1220 so that the first surface 1202 side protrudes and the portions of the main body portion 1210 and the connecting portion 1220 closer to the second end 1222 than the bending portion 1225 are approximately perpendicular to the proximal end portion 1230. In the sensor 1101 shown in Figs. 41 and 44, the insulating layer 1050 includes the first insulating layer 1051 that contains an insulating cured resin and covers the entire first surface 1202 of the substrate 1200, and a second insulating layer 1052 that includes a first insulating sheet and covers the regions of the main body portion 1210 and the first surface 1202 of the substrate 1200 that are closer to the second end 1222 than the bending portion 1225 of the connecting portion 1220 via the first insulating layer 1051. The insulating layer 1050 includes a non-insulating area 1055 that does not include an insulating sheet and includes the first insulating layer 1051 containing an insulating cured resin, above the region of the bending portion 1225 of the connecting portion 1220 of the first surface 1202 of the substrate 1200. In this embodiment, the second insulating layer 1052 containing the first insulating sheet is flat and not deformed. Therefore, the substrate 1200 is unaffected by any contraction or expansion that may occur after the first insulating sheet is bent, and the portions of the main body portion 1210 and the connecting portion 1220 that are closer to the second end 1222 than the bending portion 1225 are maintained in a flat shape. As a result, as shown in Fig. 44, the main body portion 1210 of the substrate 1200 of the sensor 1101 is maintained in a vertical position with the distal end portion 1201 at the bottom in the liquid sample X1, which allows for the analyte to be properly measured by the detection electrodes 1300.

Meanwhile, Fig. 45 shows a sensor 1151 in a comparative example, which has the same structure as the sensor 1101 shown in Fig. 41 and Fig. 44 except that the second insulating layer 1052 including the first insulating sheet is formed on the entire first surface 1202 of the substrate 1200, including the region above the bending portion 1225. With this comparative example sensor 1151, the second insulating layer 1052 including the insulating sheet on the first surface 1202 of the substrate 1200 is stretched by bending the substrate 1200 at the bending portion 1225. Since the stretched second insulating layer 1052 including the insulating sheet tends to contract, warping occurs in the main body portion 1210 and the connecting portion 1220 of the substrate 1200 as shown in Fig. 45. The warped main body portion 1210 of the substrate 1200 inclines so that the side of the first surface 1202 on which the detection electrode 1300 is disposed faces upward. Therefore, as shown in Fig. 45, contact between the liquid sample X1 and the detection electrode 1300 on the first surface 1202 of the main body portion 1210 is hampered, which lowers the accuracy of measurement of the analyte by the detection electrodes 1300. With the sensor 1101 shown in Figs. 41 and 44, this problem encountered with the comparative example sensor 1151 is solved by including the non-insulating region 1055 in the insulating layer 1050 in the region of the first surface 1202 of the substrate 1200 above the bending portion 1225.

Yet another embodiment of the sensor 1101 shown in Figs. 41 and 44 will be described with reference to Fig. 46. In the sensor 1101 shown in Fig. 46, the insulating layer 1050 includes a second insulating layer 1052a, which includes a first insulating sheet and is disposed above the region of the first surface 1202 of the substrate 1200 that is closer to the second end 1222 side than the main body portion 1210 and the bending portion 1225 of the connecting portion 1220, and a second insulating layer 1052b, which includes a second insulating sheet and is disposed above the region of the proximal end portion 1230 of the first surface 1202 of the substrate 1200. Here, the first insulating sheet of the second insulating layer 1052a and the second insulating sheet of the second insulating layer 1052b are separated above the region of the bending portion 1225 of the connecting portion 1220 of the first surface 1202 of the substrate 1200. In the sensor 1101 shown in Fig. 46, the insulating layer 1050 further includes the first insulating layer 1051 containing an insulating cured resin and formed on the entire first surface 1202 of the substrate 1200. The insulating layer 1050 includes the non-insulating region 1055 that includes the first insulating layer 1051 and does not include an insulating sheet above the region of the bent portion 1225 of the connecting portion 1220 of the first surface 1202 of the substrate 1200. The second insulating layer 1052a including the first insulating sheet and the second insulating layer 1052b including the second insulating sheet are respectively disposed in the above-mentioned region of the first surface 1202 of the substrate 1200 from above the first insulating layer 1051. With this embodiment, a decrease in detection accuracy caused by warping of the substrate 1200 of the sensor 1101 can be suppressed, and insulation between the wiring 1005 and the terminal 1006 at the proximal end portion 1230 of the substrate 1200 can be increased.

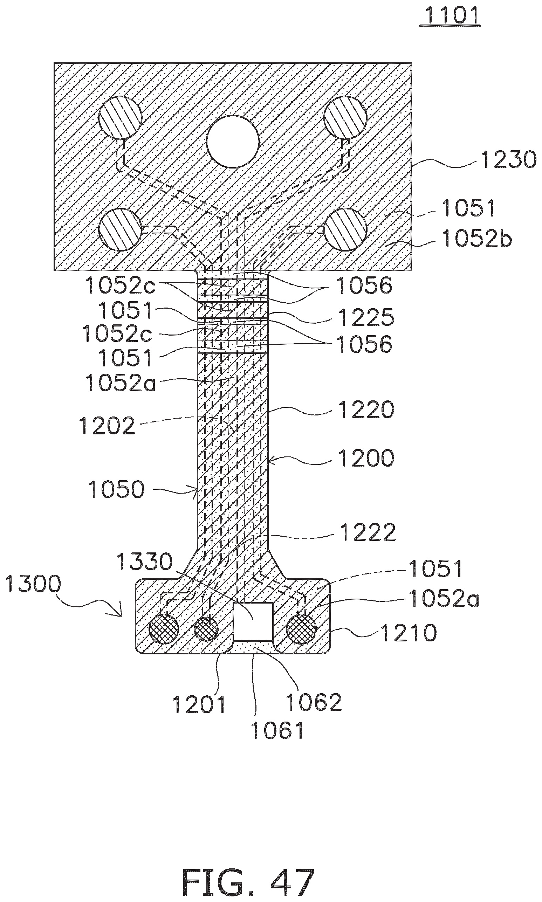

Yet another embodiment of the sensor 1101 shown in Fig. 46 will be described with reference to Fig. 47. With the sensor 1101 shown in Fig. 47, the first insulating sheet of the second insulating layer 1052a and the second insulating sheet of the second insulating layer 1052b are separated via cutouts 1056 formed in a direction intersecting the direction in which the connecting portion 1220 extends, above the region of the bending portion 1225 of the connecting portion 1220 on the first surface 1202 of the substrate 1200. In this embodiment, the insulating layer 1050 includes a second insulating layer 1052c including a plurality of third insulating sheets that are separated from the second insulating layers 1052a and 1052b via the cutouts 1056 above the region of the bending portion 1225 of the connecting portion 1220 on the first surface 1202 of the substrate 1200. With this embodiment, a decrease in detection accuracy caused by warping of the substrate 1200 of the sensor 1101 can be suppressed, and insulation between the wirings 1005 on the bending portion 1225 of the connecting portion 1220 of the substrate 1200 can be increased.

### Sensor Unit 900

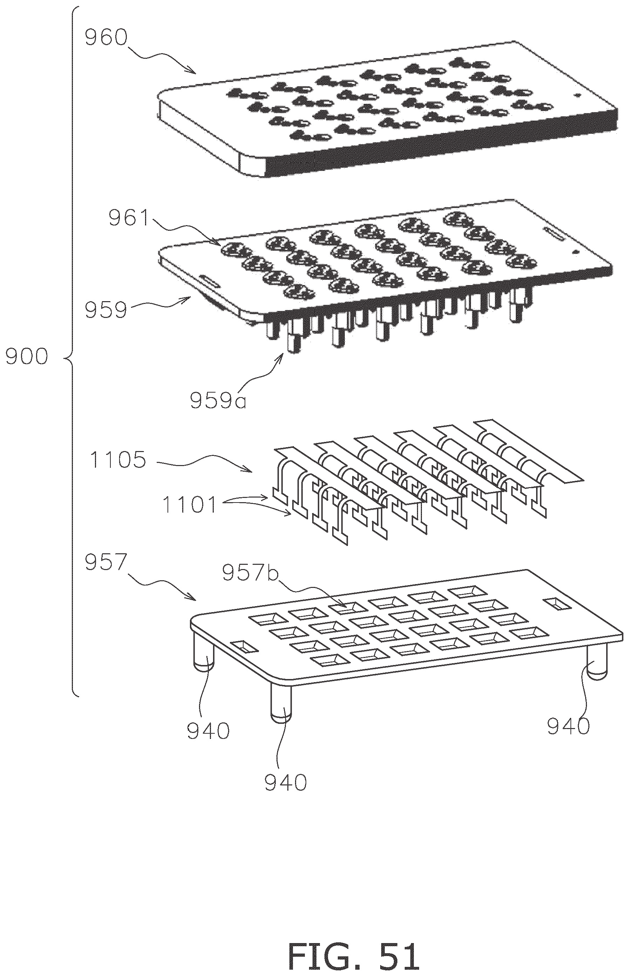

A sensor unit 900 will be described through reference to Figs. 51, 52, and 53.

The sensor unit 900 shown in the drawings is used to position the main body portion 1210 of the substrate 1200 including the detection electrodes 1300 of the sensor 1101 relative to the wells of a 24-well plate 925.

As shown in Fig. 51, the sensor unit 900 comprises a lower support plate 957, six sets of multiple sensors 1105 (see Fig. 42), an upper support plate 959 to which is attached a port 961 for supplying an additive, and a gasket sheet 960, disposed in that order starting from the bottom.

In this embodiment, the multiple sensor 1105 is formed by coupling and integrating four of the sensors 1101 shown in Fig. 42 at the proximal end portion 1230 of the substrate 1200. Each of the four sensors 1101 is bent at the bending portion 1225 of the connecting portion 1220 of the substrate 1200 so that the first surface 1202 side of the substrate 1200 protrudes, and the portions of the main body portion 1210 and the connecting portion 1220 that are closer to the second end 1222 than the bending portion 1225 are perpendicular to the proximal end portion 1230. As shown in Fig. 51, six sets of the multiple sensor 1105 including the four sensors 1101 are attached to the lower support plate 957.

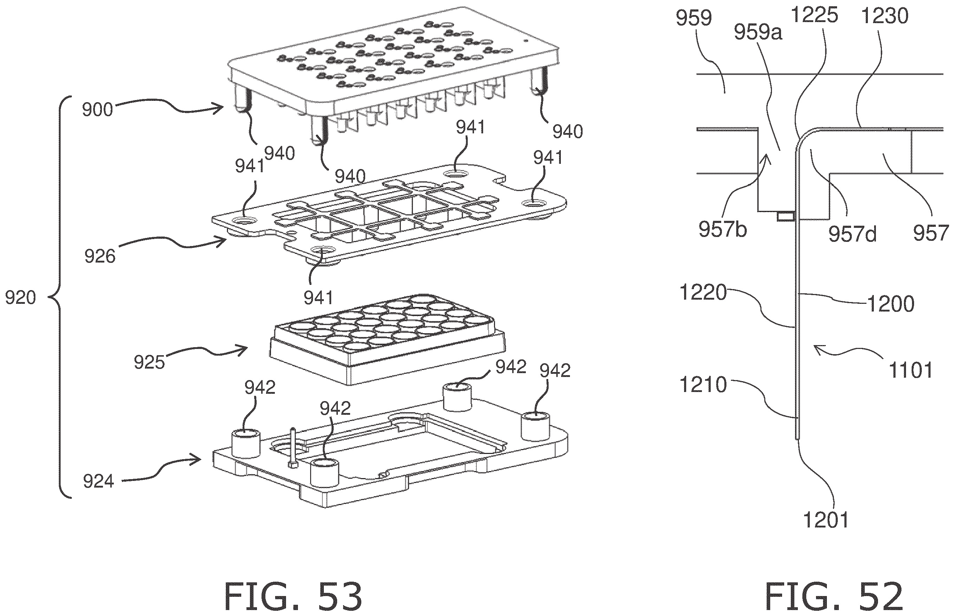

The lower support plate 957 comprises through-holes 957b at positions corresponding to the main body portion 1210 and the connecting portion 1220 of the substrate 1200 of each of the 24 sensors 1101 included in the six sets of multiple sensors 1105. The main body portions 1210 and the connecting portions 1220 of the substrates 1200 of the 24 sensors 1101 are inserted into the through-holes 957b in the lower support plate 957. Once the sensors 1101 are inserted into the through-holes 957b of the lower support plate 957, the upper surface of the lower support plate 957 and the lower surface of the proximal end portion 1230 of the substrate 1200 of the sensor 1101 are engaged with each other as shown in Fig. 52. Also, a first engagement portion 957d that supports the bending portion 1225 of the connecting portion 1220 of the substrate 1200 from below is provided on the inner peripheral walls of the through-holes 957b in the lower support plate 957, at the portion that engages with the inside of the bending portion 1225 of the connecting portion 1220 of the substrate 1200 of the sensor 1101.

Meanwhile, pin-shaped second engagement portions 959a that are inserted into the twenty-four through-holes 957b in the lower support plate 957 are provided to the lower surface of the upper support plate 959.

When the six multiple sensors 1105 are sandwiched and assembled between the lower support plate 957 and the upper support plate 959, as shown in Fig. 52, the upper surface of the first engagement portion 957d of the lower support plate 957 and the lower surface of the second engagement portion 959a of the upper support plate 959 engage and support the bending portion 1225 of the connecting portion 1220 of the substrate 1200 of the sensor 1101. As shown in Fig. 52, the first engagement portion 957d of the lower support plate 957 has an upper surface curved portion including a curved surface on the upper surface. Also, the second engagement portion 959a of the upper support plate 959 has a lower surface curved portion including a curved surface on the lower surface. With this configuration, the sensor 1101 is supported so that the distal end portion 1201 of the substrate 1200 is at the lower end, and the portion of the of the substrate 1200 including the main body portion 1210 and the portion closer to the distal end side than the bending portion 1225 of the connecting portion 1220 is vertical. As a result, the orientation of the main body portion 1210 of the substrate 1200 of the sensor 1101 relative to the liquid sample contained in the wells of the well plate can be stabilized, and the detection accuracy of the sensor 1101 can be improved.

Finally, as shown in Fig. 51, a gasket sheet 960 having through-holes is placed on the upper surface of the upper support plate 959 to complete the sensor unit 900.

The sensor unit 900 can be combined with other members to form an adaptor unit 920. As shown in Fig. 53, the adaptor unit 920 has an adaptor bottom (culture vessel installation portion) 924, a well plate (culture vessel) 925, an adaptor top 926, and the sensor unit 900 installed in that order starting from the lowest stage.

In this embodiment, the well plate 925 has 24 wells (4 × 6).

The adaptor top 926 is provided for adjusting the height of the well plate 925, and a different adaptor top 926 is used to suit the height of the well plate 925. The purpose of this is to adjust the height relationship between the sensor unit 900 and the well plate 925 when the sensor unit 900 is placed on the adaptor top 926.

There are a number of kinds of well plate 925, including general-purpose well plates, and the proper adaptor top 926 may be used according to the type.

The sensor unit 900 placed on the adapter top 926 has four legs (supports) 940 on its underside, and these are inserted through through-holes 941 in the lower adapter top 926 and into positioning holes 942 provided in the adapter bottom 924, which serves as a culture vessel installation unit.

With this adapter unit 920 comprising the sensor unit 900 and well plate 925, the main body portion 1210 of the substrate 1200 including the detection electrodes 1300 of the sensor 1101 is held in a stable orientation within each well of the well plate 925.

Although not depicted in the drawings, in the adaptor unit 920, the terminal 1006 on the proximal end portion 1230 of the substrate 1200 of the sensor 1101 included in the sensor unit 900 can be electrically connected to the electrochemical measurement unit 1111 (see Fig. 48) of an external analyzer 1102 through the through-hole in the gasket sheet 960 of the sensor unit 900. That is, the adaptor unit 920 equipped with the sensor unit 900 can be combined with the external analyzer 1102 and a controller 1104 to form the analysis device 1100 shown in Fig. 48.

### Working Example of Second Disclosure

The following five sensors were produced as working examples of the second disclosure or comparative examples. The sensors S1 to S5 used in the following experiments were not provided with the reagent layers 1315 and working electrode protective films 1316 of the working electrodes 1310, and with the reference electrode protective film 1326 of the reference electrode 1320.

### Sensor S1

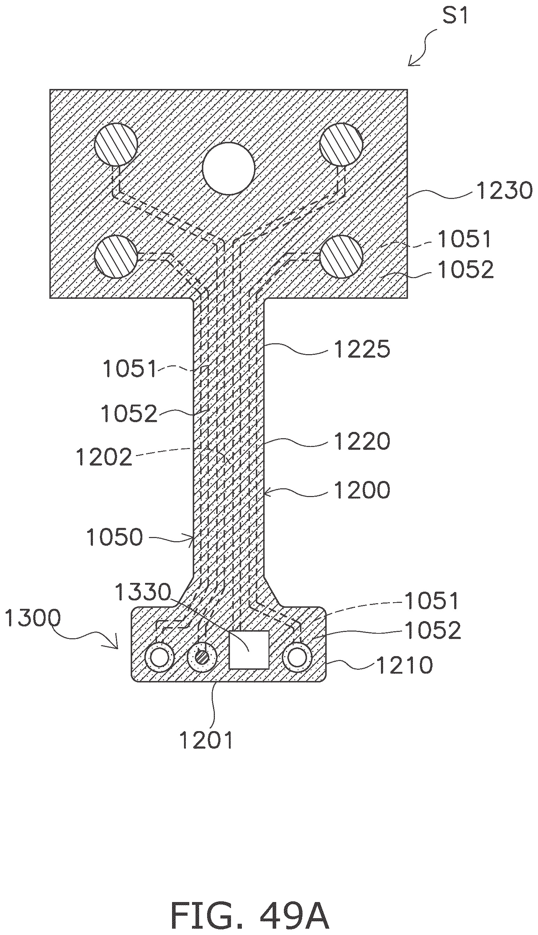

The sensor S1 is a comparative example of the structure shown in Fig. 49A. In the sensor S1, the first insulating layer 1051 and the second insulating layer 1052 including an insulating sheet are laminated over the entire first surface 1202 of the substrate 1200. With the sensor S1, the distal end opening 1061 and the flow path 1062 are not formed, and the portion of the first surface 1202 of the substrate 1200 between the counter electrode 1330 and the distal end portion 1201 is also covered by the insulating layer 1050 including the first insulating layer 1051 and the second insulating layer 1052. That is, the sensor S1 does not have the non-insulating region 1055 of the insulating layer 1050 for preventing warping of the substrate 1200, and the distal end opening 1061 and the flow path 1062 for guiding a liquid sample to the upper surface 1331 of the counter electrode 1330.

### Sensor S2

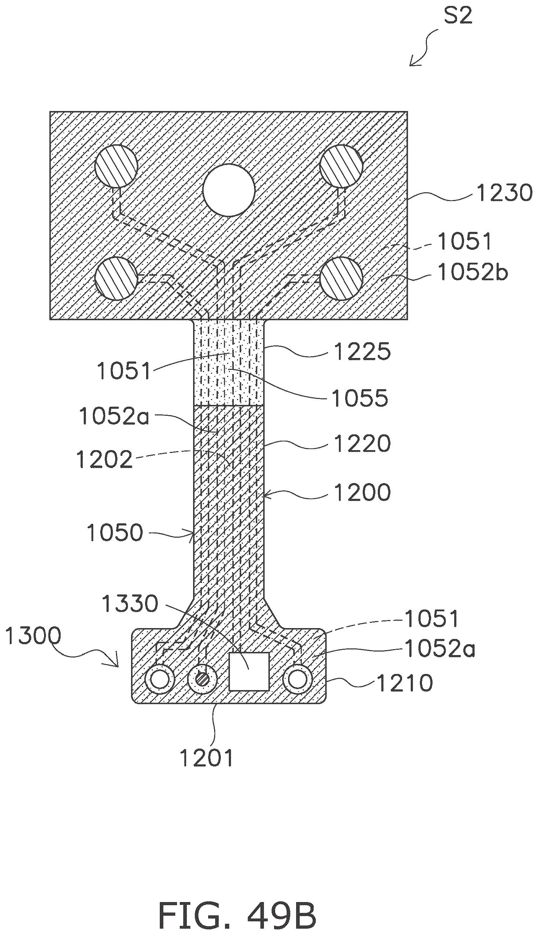

The sensor S2 is a comparative example of the structure shown in Fig. 49B. In the sensor S2, instead of the second insulating layer 1052 covering the entire sensor S1, a second insulating layer 1052a including a first insulating sheet disposed in a portion that is closer to the distal end side than the bending portion 1225 of the connecting portion 1220 of the substrate 1200, and a second insulating layer 1052b including a second insulating sheet disposed in the proximal end portion 1230 of the substrate 1200 are disposed, and a non-insulating region 1055 including the first insulating layer 1051, but not including an insulating sheet, is disposed on the bending portion 1225 of the connecting portion 1220 of the substrate 1200. That is, the sensor S2 has the non-insulating region 1055 of the insulating layer 1050 for preventing warping of the substrate 1200, but does not have the distal end opening 1061 and the flow path 1062 for guiding a liquid sample to the upper surface 1331 of the counter electrode 1330.

### Sensor S3

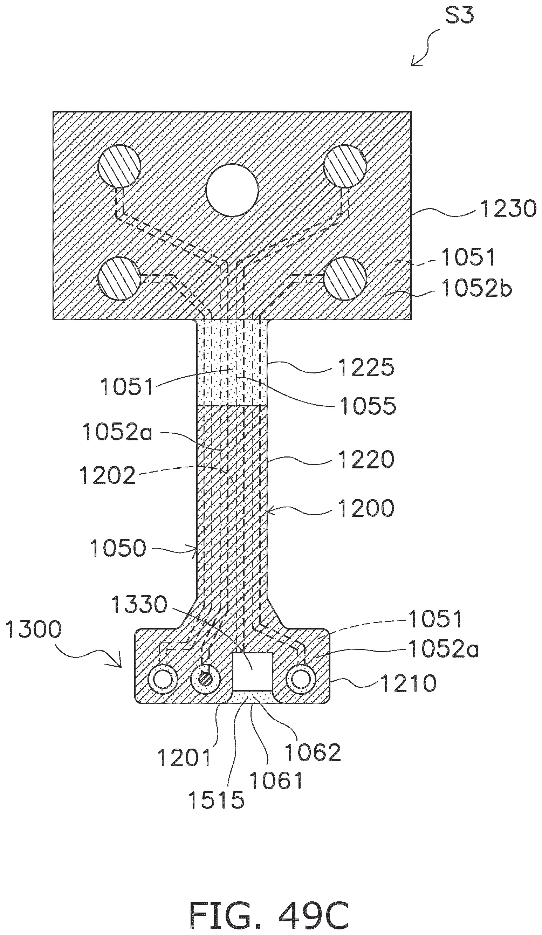

The sensor S3 is a working example of the sensor 1101 having the structure shown in Fig. 49C. Like the sensor 1101 shown in Fig. 46, the sensor S3 has the non-insulating region 1055 of the insulating layer 1050 for preventing warping of the substrate 1200, and the distal end opening 1061 and the flow channel 1062 (see Fig. 36) formed only in second insulating layer 1052.

### Sensor S4

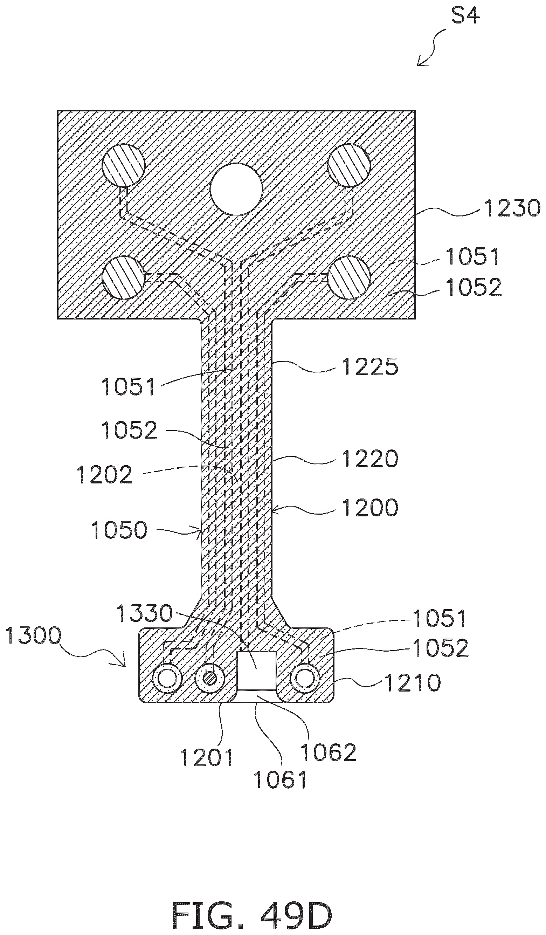

The sensor S4 is a working example of the sensor 1101 having the structure shown in Fig. 49D. The sensor S4 is obtained by replacing the second insulating layer 1052a including the first insulating sheet and the second insulating layer 1052b including the second insulating sheet of the insulating layer 1050 in the sensor S3 with a second insulating layer 1052 including an integrated insulating sheet that covers the entire first surface 1202 of the substrate 1200, and then peeling off the portion 1515 (see Figs. 36 and 49C) of the first insulating layer 1051 between the counter electrode 1330 and the distal end portion 1201 of the substrate 1200 to form the distal end opening 1061 and the flow path 1062. That is, the sensor S4 does not have the non-insulating region 1055 of the insulating layer 1050 for preventing warping of the substrate 1200, but does have the distal end opening 1061 and the flow path 1062 (see Fig. 38) formed in the first insulating layer 1051 and the second insulating layer 1052.

### Sensor S5

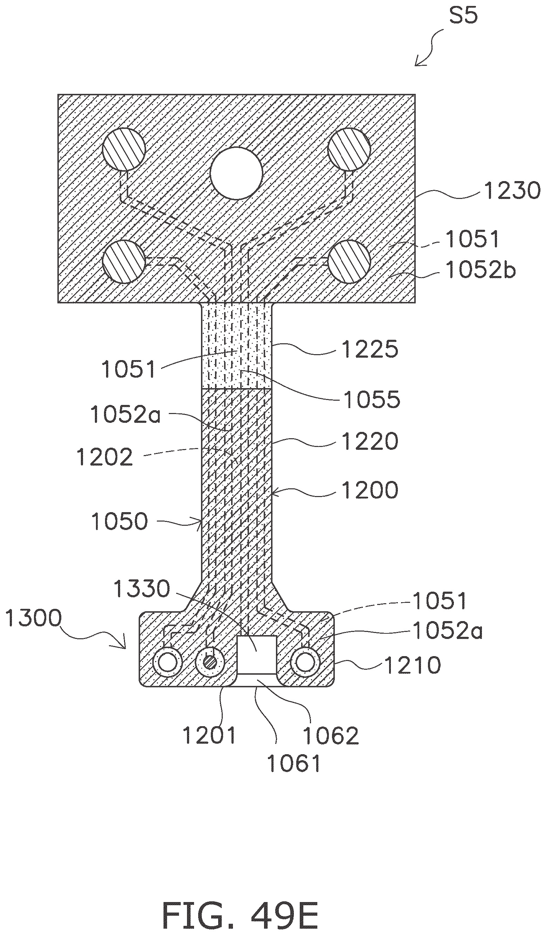

The sensor S5 is a working example of the sensor 1101 having the structure shown in Fig. 49E. The sensor S5 is obtained by peeling off the portion 1515 (see Figs. 36 and 49C) of the first insulating layer 1051 in the sensor S3 that is between the counter electrode 1330 and the distal end portion 1201 of the substrate 1200 to form the distal end opening 1061 and the flow channel 1062. That is, the sensor S5 has the non-insulating region 1055 of the insulating layer 1050 for preventing warping of the substrate 1200, and the distal end opening 1061 and the flow channel 1062 (see Fig. 38) formed in the first insulating layer 1051 and second insulating layer 1052.

The materials used in the manufacture of the five sensors above are given below.

### Substrate

A substrate 1200 made of polyethylene terephthalate and having a thickness of 188 µm, which included the main body portion 1210, the connecting portion 1220, and the proximal end portion 1230, and had the shape shown in Figs. 49A to 49E was used as an insulating substrate.

The main body portion 1210 of the substrate 1200 was rectangular, with a width of 11 mm and a length of 4.2 mm.

After the insulating layer 1050 was laminated to complete the sensor, the substrate 1200 was bent at the bending portion 1225 of the connecting portion 1220 , so that the substrate 1200 protruded toward the first surface 1202 and that the portions of the main body portion 1210 and the connecting portion 1220 that is closer to the distal end side than the bending portion 1225 were perpendicular to the proximal end portion 1230.

### Conductive Layer

On the first surface 1202 of the substrate 1200, the working electrode conductive layers 1311, the reference electrode conductive layer 1321, the counter electrode (counter electrode conductive layer) 1330, the wiring 1005, and the terminal 1006 were formed from a carbon conductive layer having a thickness of 5 µm and obtained by applying and heating a carbon paste, in the shapes shown in Figs. 27 and 46.

### First Insulating Layer

On the first surface 1202 of the substrate 1200 on which the carbon conductive layer was disposed, the first insulating layer 1051 made of a fluororesin was laminated in a thickness of 5 µm on the carbon conductive layer, covering the first surface 1202 of the substrate 1200 and the carbon conductive layer, as shown in Fig. 28. The first insulating layer 1051 was formed by coating with a curable resin composition (negative resist composition) that is cured by irradiation with active energy rays to produce an insulating cured resin, and then irradiating this coating with active energy rays. This was masked off appropriately, and the working electrode first openings 1511, the reference electrode first opening 1512, and the counter electrode first opening 1513 were formed in the first insulating layer 1051.

The silver/silver chloride layer 1322 was disposed in the reference electrode first opening 1512 of the first insulating layer 1051.

### Second Insulating Layer

The second insulating layer 1052 was disposed in a specific region on the first surface 1202 of the substrate 1200 on which the carbon conductive layer and the first insulating layer 1051 were disposed. The second insulating layer 1052, which was a laminate of a polyethylene terephthalate sheet (insulating sheet) having a thickness of 50 µm and whose surface had undergone a water repellency treatment, and a bonding layer having a thickness of 10 µm and containing a pressure-sensitive adhesive, was affixed to a specific region on the first insulating layer 1051 disposed on the first surface 1202 of the substrate 1200 via the bonding layer.

The working electrode second openings 1521 and the reference electrode second opening 1522 were each a circle with a diameter of 2.0 mm. The counter electrode second opening 1523 was a rectangle with a width of 2.1 mm in the lateral direction of the main body portion 1210 and a width of 1.8 mm in the longitudinal direction.

The following experiments involved the use of the sensors S1 to S5 which were not provided with the reagent layers 1315 and the working electrode protective films 1316 of the working electrodes 1310, and the reference electrode protective film 1326 of the reference electrode 1320.

A 24-well plate was prepared, each cylindrical well having a depth of 17.40 mm, a bottom diameter of 16.26 mm, and a well diameter of 15.62 mm. RPMI medium to which 500 µM of ascorbic acid had been added (hereinafter referred to as "liquid sample X1") was added in an amount of 0.85 mL, 0.94 mL, 1.0 mL, or 1.2 mL to the wells of the 24-well plate.

As shown in Figs. 43 to 45, the sensors S1 to S5 were held horizontally so that the proximal end portion 1230 of the substrate 1200 faced upward, and the main body portion 1210 of the substrate 1200 was immersed in each amount of liquid sample X1 in the well so that the distal end portion 1201 was the lower end.

The voltage difference between the working electrodes 1310 (not including the reagent layer and protective film) of the sensors S1 to S5 and the reference electrode 1320 (not including the protective film) was controlled to 500 mV by a potentiostat, and the current value between each of the two working electrodes 1310 and the counter electrode 1330 was measured over time until 0.7 hour later. Also, the voltage of the counter electrode 1330 was measured over time to check whether or not the liquid sample X1 was in contact with the counter electrode 1330.

In order to promote contact between each of the detection electrodes 1300 and the liquid sample X1, measurement was performed while vibrating the 24-well plate after a certain time had elapsed since the start of the measurement.

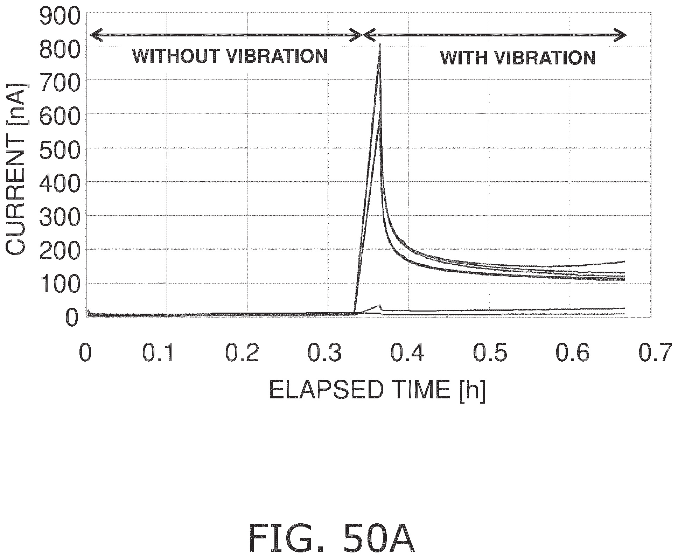

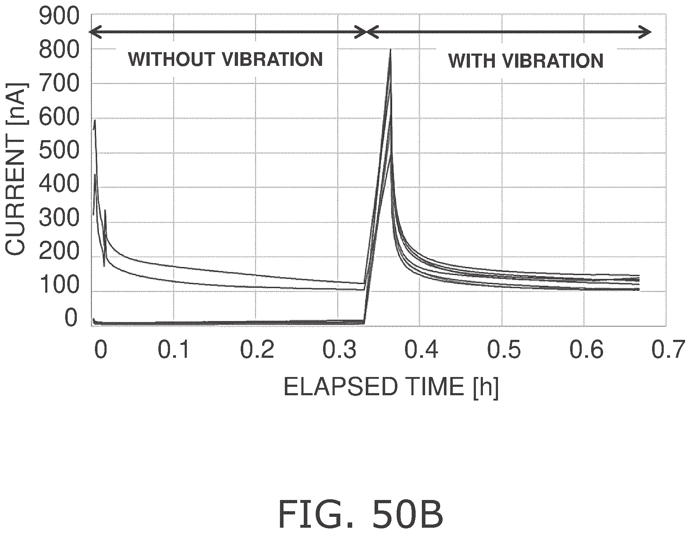

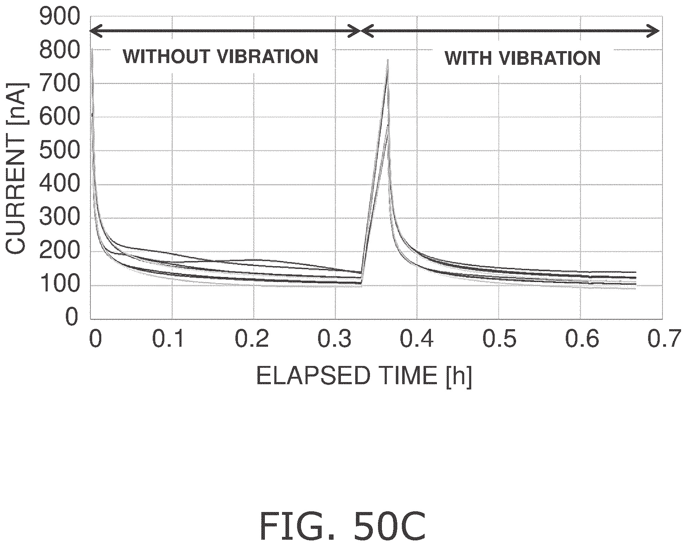

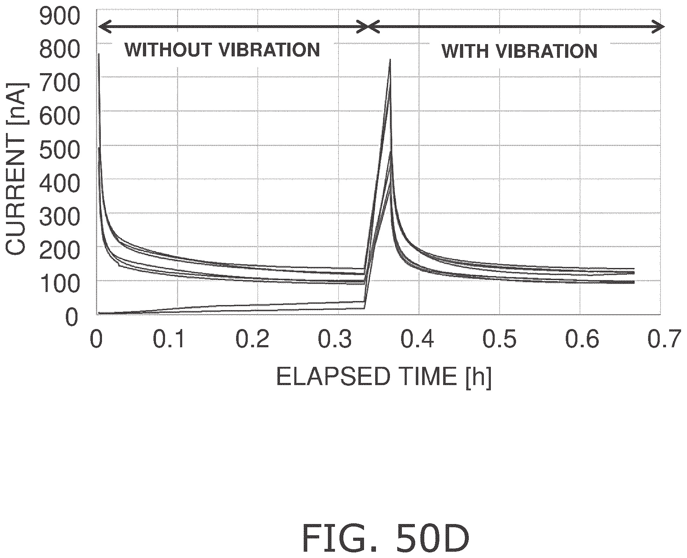

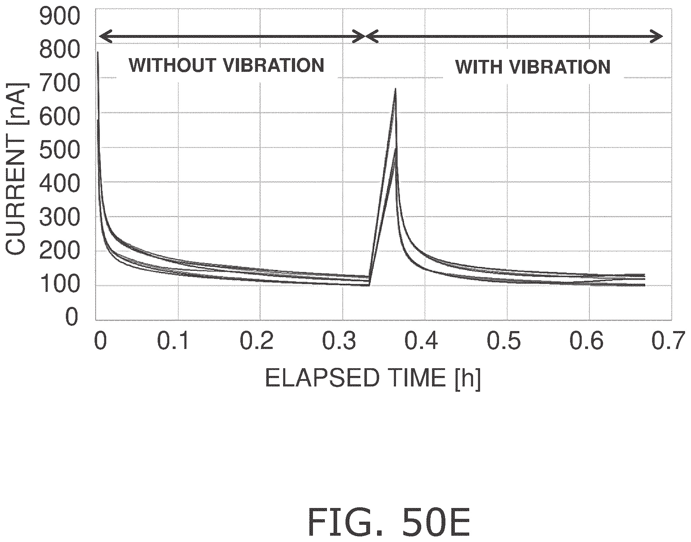

Fig. 50 shows the measurement results for the current values of the two working electrodes 1310 when each of four volumes of liquid sample were used for each of the sensors S1 to S5. Fig. 50A shows the results for sensor S1, Fig. 50B shows the results for sensor S2, Fig. 50C shows the results for sensor S3, Fig. 50D shows the results for sensor S4, and Fig. 50E shows the results for sensor S5.

In the comparative example sensor S1 (see Fig. 49A), when no vibration was applied, the current value of the working electrode bottomed out in all volumes of the liquid sample X1, and a normal current value was not measured. When vibration was applied, a normal current value was measured at the working electrode in 1.2 mL, 1.0 mL, and 0.94 mL of liquid sample X1, but in 0.85 mL of liquid sample X1, the current value of the working electrode bottomed out and normal measurement was not possible.

In the comparative example sensor S2 (see Fig. 49B), when vibration was not applied, the current value of the working electrode bottomed out and no normal current value was measured in 1.2 mL, 1.0 mL, and 0.85 mL of liquid sample X1, but a normal current value was measured in 0.94 mL of liquid sample X1. When vibration was applied, a normal current value was measured in the working electrode for all volumes of liquid sample X1.

In the working example sensor S3 (see Fig. 49C), a normal current value was measured at the working electrode for all volumes of the liquid sample X1, both when vibration was and was not applied.

In the working example sensor S4 (see Fig. 49D), when no vibration was applied, a normal current value was measured at the working electrode in 1.2 mL, 1.0 mL, and 0.94 mL of liquid sample X1, but in the 0.85 mL liquid sample, the current value at the working electrode bottomed out and normal measurement was not possible. When vibration was applied, normal current values were measured at the working electrode in all volumes of liquid sample X1.

In the working example sensor S5 (see Fig. 49E), a normal current value was measured at the working electrode in all volumes of the liquid sample X1, both when vibration was and was not applied.

The above results are collected in the following table.

**Table 1**

| Sensor | Liquid volume 1.2 mL | Liquid volume 1.0 mL | Liquid volume 0.94mL | Liquid volume 0.85mL |
|---|---|---|---|---|
| S1 | F (A) | F (A) | F (A) | F (F) |
| S2 | F (A) | F (A) | A (A) | F (A) |
| S3 | A (A) | A (A) | A (A) | A (A) |
| S4 | A (A) | A (A) | A (A) | F (A) |
| S5 | A (A) | A (A) | A (A) | A (A) |

| | | | | |
|---|---|---|---|---|
| F: working electrode current value could not be measured A: working electrode current measurement successful Outside of parentheses are results when vibration was not applied Inside of parentheses are results when vibration was applied | | | | |

It was confirmed that regardless of which sensor was used, when the current value of the working electrode bottomed out and normal measurement was not possible, the voltage value of the counter electrode also bottomed out, and when the current value of the working electrode was normal, the voltage value of the counter electrode was also normal (data not shown in the drawings). This indicates that the counter electrode and the liquid sample need to be in good contact in order to achieve normal measurement by the sensor. It was also confirmed from the measurement results of the current value that forming a distal end opening and a flow path in the insulating layer allows the liquid sample to be guided to the upper surface of the counter electrode, and makes it less likely that contact will be inhibited between the counter electrode and the liquid sample, and that suppressing the warping of the base material further suppresses contact inhibition.

### Supplementary Notes Regarding Second Disclosure

With the configuration of a conventional cell culture analysis device comprising a sensor for analyzing a cell culture liquid, the sensor is fixed to a through-hole portion provided in a base material, and a lead wire for taking off a signal is connected to this sensor.

For example, Patent Literature 2 (US Pat. No. 9,170,255) discloses a cell culture analysis device having a cartridge that mates with a plate on which a plurality of cell culture vessels are provided.

The cell culture analysis device in Patent Literature 2 has a sensor for measuring the inside of each culture vessel, a cartridge is provided with a plurality of openings into which these sensors are inserted, and the sensors are connected to fiber cables within these openings. These fiber cables are connected to an external control unit.

The following problem is encountered with the sensor of the cell culture analysis device described in Patent Literature 2.

With the cell culture analysis device disclosed in Patent Literature 2, it is difficult to accurately position each of the sensors inserted into the plurality of culture vessels with respect to the culture vessels.

This may cause variations in the depth to which the sensor is immersed in the culture medium held in the culture vessel, which may reduce measurement accuracy. In particular, if the sensor is not immersed deep enough in the culture medium, the liquid sample may not come into sufficient contact with the detection electrodes of the sensor, and this may reduce measurement accuracy.

It is an object of the second disclosure to provide a sensor, and a sensor unit comprising this sensor, with which the inhibition of contact between a liquid sample and a detection electrode of the sensor when the sensor is immersed in the liquid sample less likely to occur.

In view of this, this specification discloses, as a second disclosure, the sensor and the sensor unit described in any of the following Supplementary Notes 1 to 11.

### Supplementary Note 1

A sensor that is used in a state of being immersed in a liquid sample, comprising:
an insulating substrate including a distal end portion that is immersed in the liquid sample;
detection electrodes disposed near the distal end portion on a first surface of the substrate, the detection electrodes including a working electrode, a counter electrode, and a reference electrode;
wiring that is disposed on the first surface of the substrate and is connected to the detection electrodes;
an insulating layer that is disposed on the first surface of the substrate and is formed so as to cover at least part of the detection electrodes and the wiring;
a distal end opening that is formed in the insulating layer, communicates with an upper surface of the counter electrode, and opens to the side of the substrate with the distal end portion; and
a flow path that is formed in the insulating layer, connects the distal end opening and the upper surface of the counter electrode, and guides the liquid sample from the distal end opening to the upper surface of the counter electrode.

### Supplementary Note 2

The sensor according to Supplementary Note 1, wherein:
the insulating layer comprises a counter electrode opening that is formed at a position overlapping the counter electrode in plan view from the thickness direction of the substrate, and goes through in the thickness direction, and
the upper surface of the counter electrode is exposed through the counter electrode opening.

### Supplementary Note 3

The sensor according to Supplementary Note 1 or 2, wherein:
the insulating layer comprises, in plan view from the thickness direction of the substrate:
a working electrode opening that is formed at a position overlapping the working electrode and goes through in the thickness direction; and
a reference electrode opening that is formed at a position overlapping the reference electrode and goes through in the thickness direction,
the working electrode includes a working electrode protective film disposed in the working electrode opening, and
the reference electrode includes a reference electrode protective film disposed in the reference electrode opening.

### Supplementary Note 4

The sensor according to any of Supplementary Notes 1 to 3, wherein:
the insulating layer includes:
a first insulating layer that is disposed on the first surface of the substrate and contains an insulating cured resin; and
a second insulating layer that is disposed on the first insulating layer and includes an insulating sheet, and
the distal end opening and the flow path are formed in the second insulating layer.

### Supplementary Note 5

The sensor according to Supplementary Note 4,
wherein the distal end opening and the flow path are further formed in the first insulating layer.

### Supplementary Note 6

The sensor according to any of Supplementary Notes 1 to 5, wherein the flow path has an expansion section where the width of the flow path expands toward the distal end opening.

### Supplementary Note 7

The sensor according to any of Supplementary Notes 1 to 6, wherein:
the substrate includes:
a main body portion that includes the distal end portion and a first end portion on the opposite side from the distal end portion, and on which the detection electrodes are disposed;
a connecting portion that includes a second end connected to the first end of the main body portion, and a third end on the opposite side from the second end, and that extends from the second end to the third end; and
a proximal end portion that includes a fourth end connected to the third end of the connecting portion, and
the connecting portion of the substrate includes a bending portion near the third end portion, at which |the substrate is bent so that the first surface side protrudes.

### Supplementary Note 8

The sensor according to Supplementary Note 7,
wherein the insulating layer includes a first insulating sheet that is disposed above the region of the first surface of the substrate having the portions of the main body portion and the connecting portion that are closer to the second end portion than the bending portion.

### Supplementary Note 9

The sensor according to Supplementary Note 8,
wherein the insulating layer has a non-insulating region that does not include an insulating sheet, above the region of the first surface of the substrate having the bending portion of the connecting portion.

### Supplementary Note 10

The sensor according to Supplementary Note 8 or 9, wherein:
the insulating layer further includes a second insulating sheet that is disposed above the region of the first surface of the substrate having the proximal end portion, and
the first insulating sheet and the second insulating sheet are separated from each other above the region of the first surface of the substrate having the bending portion of the connecting portion.

### Supplementary Note 11

A sensor unit, comprising:
the sensor according to any of Supplementary Notes 1 to 10; and
a support member equipped with an engagement portion that engages with the substrate of the sensor and supports the sensor so that the distal end portion of the substrate is the lower end.

### Embodiment of Third Disclosure

An embodiment of the sensor according to the third disclosure will now be described. In this embodiment, some unnecessarily detailed description may be omitted. For example, detailed description of already known facts or redundant description of components that are substantially the same may be omitted. This is to avoid unnecessary repetition in the following description, and facilitate an understanding on the part of a person skilled in the art. The components in the sensor according to the third disclosure that are the same as in the sensor according to the first disclosure will be numbered the same. See the description of the corresponding components in the sensor according to the first disclosure.

Also, the applicant has provided the appended drawings and the following description so that a person skilled in the art might fully understand this third disclosure, but does not intend for these to limit what is discussed in the following "Supplementary Notes Regarding Third Disclosure."

### Materials

Examples of materials that can be used in the sensor of the third disclosure in this specification are described below.

When the sensor of the third disclosure of this specification has an insulating substrate, there are no particular limitations on the material thereof, but the same material as that of the insulating substrate of the sensor in the first disclosure of this specification can be used, for example.

The conductive layer of the working electrode, reference electrode, and counter electrode of the sensor of the third disclosure of this specification is a layer containing a conductive material such as carbon or gold. The conductive layer can be produced by forming a layer of this conductive material on the surface of the substrate by sputtering, vapor deposition, screen printing, or another such method. The conductive layer can be worked into a specific pattern using laser trimming as necessary. In the third disclosure, the conductive layer of the working electrode will sometimes be referred to as the working electrode conductive layer, and the conductive layer of the reference electrode as the reference electrode conductive layer. In the third disclosure, the conductive layer of the counter electrode is simply referred to as the counter electrode because the conductive layer itself constitutes the counter electrode. More preferred embodiments of the conductive material constituting the working electrode conductive layer and the counter electrode shall be given below.

The wiring of the sensor of the third disclosure of this specification may also be made of the same conductive material similar as that of the conductive layer.

The sensor of the third disclosure of this specification is used to detect a specific analyte in a liquid sample by immersing the sensor in the liquid sample. The liquid sample is preferably one that contains water as a solvent. Examples of the liquid sample include a cell culture medium and a liquid sample prepared using blood collected from a living organism. Examples of the analyte are as given for the analyte detected with the sensor in the first disclosure of this specification, and it is particularly favorable for this substance to be glucose and/or lactic acid. The liquid sample is preferably one that contains cells, particularly living cells, and a cell culture medium is especially favorable.

The working electrode of the sensor of the third disclosure of this specification comprises a reagent layer that is disposed on the working electrode conductive layer and contains a reagent that will participate in the oxidation-reduction reaction of the analyte. This reagent can be appropriately selected as dictated by the analyte. The reagent can include a combination of an oxidoreductase and a mediator (electron transfer substance), or just an oxidoreductase. The oxidoreductase can include a coenzyme.

The oxidoreductase may be an oxidase or a dehydrogenase. Specific examples of the oxidoreductase are as given for the sensor in the first disclosure of this specification, and it is especially favorable for the oxidoreductase to be glucose oxidase, lactate oxidase, glucose dehydrogenase, or lactate dehydrogenase.

Also, there are no particular limitations on the mediator, and examples thereof are as given for the sensor in the first disclosure of this specification.

The reagent layer provided to the working electrode of the sensor of the third disclosure of this specification may further contain, in addition to the reagent, a buffer, a hydrophilic polymer compound, a conductive carbon filler, a crosslinking agent, or other such components. Examples of the hydrophilic polymer compound and the conductive carbon filler are as given for the sensor in the first disclosure of this specification.

The working electrode of the sensor of the third disclosure of this specification may further comprise a protective film. The protective film on the working electrode may be a film that prevents or suppresses leakage of the reagent contained in the reagent layer out of the protective film, and that allows the analyte on the outside of the protective film to permeate. A protective film having such properties preferably contains a polymer compound. Examples of the polymer compound contained in the protective film include a polymer compound containing 4-vinylpyridine as a structural unit, and a polymer compound containing a cation exchange functional group. Examples of the polymer compound containing a cation exchange functional group include a polymer compound having proton conductivity. Examples of the cation exchange functional group include an anionic functional group.

Examples of the polymer compound containing 4-vinylpyridine as a structural unit and the polymer compound having proton conductivity are as given for the sensor disclosed in the first disclosure of this specification.

The protective film provided on the working electrode of the sensor of the third disclosure of this specification can be a laminate of two or more protective films, examples of which are as given for the sensor in the first disclosure of this specification.

Another favorable example of the protective film provided on the working electrode of the sensor according to the third disclosure of this specification is a protective film containing a polymer compound containing 4-vinylpyridine as a structural unit.

The reference electrode of the sensor disclosed in the third specification may have a protective film. The protective film of the reference electrode may be made of the material described above for the protective film of the working electrode.

The counter electrode of the sensor of the third disclosure of this specification may be covered with a protective film. Preferred embodiments of the protective film that covers the counter electrode are as discussed below.

In the sensor of the third disclosure of this specification, the electrodes including the working electrode and the counter electrode disposed on the insulating substrate are preferably covered with an insulating layer, except for the portion in contact with the liquid sample. The insulating layer may include an insulating resin. The insulating layer may have a multilayer structure consisting of two or more layers. The insulating layers of the multilayer structure may include, for example, a first insulating layer disposed on the insulating substrate and the conductive layer, and a second insulating layer disposed on the first insulating layer. Preferred embodiments of the materials constituting the first insulating layer and the second insulating layer are as discussed for the sensor of the first disclosure.

### Overview of Sensor 300

A sensor 300 according to an embodiment of the third disclosure will be described through reference to the drawings.

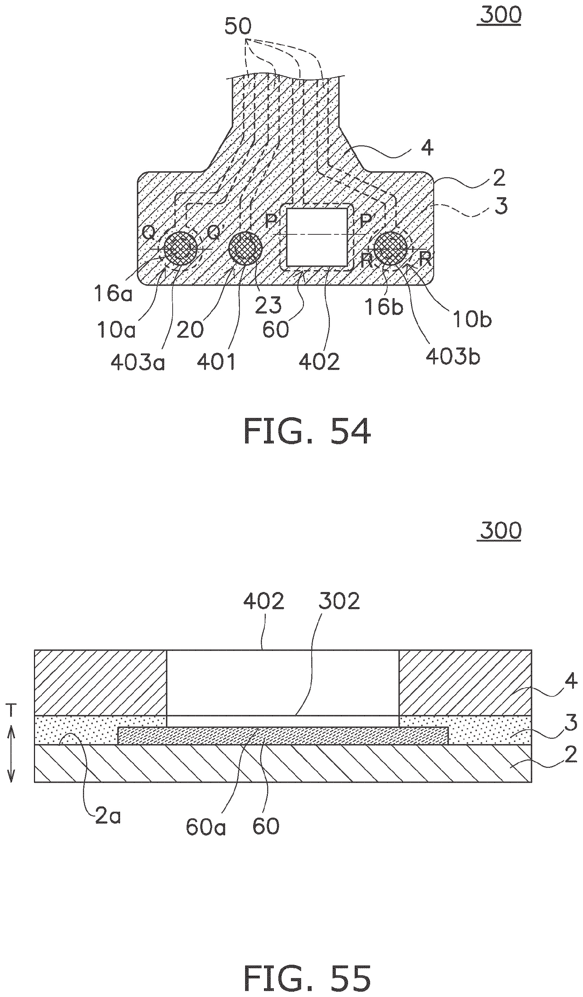

As shown in Figs. 54 to 57, the sensor 300 of this embodiment comprises an insulating substrate 2; a first working electrode 10a, a second working electrode 10b, a reference electrode 20, and a counter electrode 60 that are disposed on a first surface 2a of the substrate 2; and wiring 50 that is electrically connected to each of the first working electrode 10a, the second working electrode 10b, the reference electrode 20, and the counter electrode 60. The sensor 300 of this embodiment includes two working electrodes, but in another embodiment (not shown), the number of working electrodes may be just one, or may be three or more. In the following description, when the first working electrode 10a and the second working electrode 10b are not distinguished from each other, they will sometimes be referred to as the working electrodes 10a and 10b. Also, the first working electrode 10a, the second working electrode 10b, the reference electrode 20, and the counter electrode 60 will sometimes be collectively referred to as the electrodes. The sensor 300 of this embodiment is a three-electrode sensor including a working electrode, a reference electrode, and a counter electrode as electrodes, but it may instead be a two-electrode sensor including only a working electrode and a counter electrode, without including a reference electrode. Although not depicted in the drawings, the reference electrode and/or the counter electrode may be provided on a different substrate from the substrate on which the working electrode is disposed.

Just as with the sensor 1 of the first disclosure shown in Fig. 21, the sensor 300 is immersed in a liquid sample X and used to detect a specific analyte in the liquid sample X. Specific examples of the liquid sample and the analyte are as given in the Materials section.

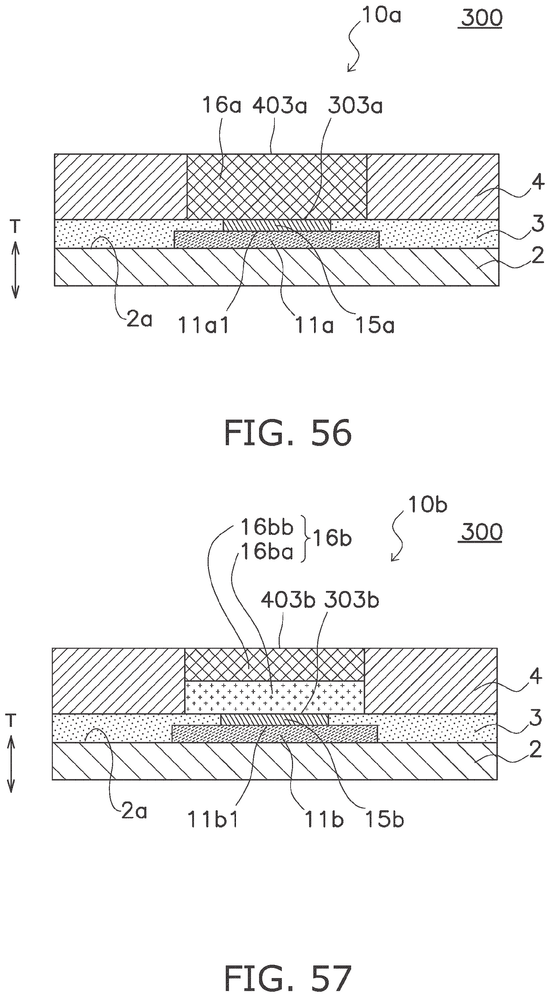

The working electrodes 10a and 10b of the sensor 300 include working electrode conductive layers 11a and 11b and reagent layers 15a and 15b containing a reagent that participates in the oxidation-reduction reaction of the analyte in the liquid sample, as shown in Figs. 56 and 57. Preferred embodiments of the reagent are as discussed in the Materials section.

When the reagent layers 15a and 15b of the working electrodes 10a and 10b of the sensor 300 contain a reagent that oxidizes the analyte in the liquid sample, electrons move from the analyte to the working electrode conductive layers 11a and 11b under conditions in which a specific voltage is applied to the electrodes of the sensor 300. Similarly, if the reagent layers 15a and 15b contain a reagent that reduces the analyte in the liquid sample, electrons move from the working electrode conductive layers 11a and 11b to the analyte. Since the amount of electrons that move depends on the concentration of the analyte, the concentration, or a change in the concentration, of the analyte in the liquid sample can be measured on the basis of the value of the current, or a change in the current, flowing through the working electrodes 10a and 10b of the sensor 300.

The sensor 300 can be part of an analysis device for analyzing an analyte in a liquid sample. An example of the analysis device is one that comprises the sensor 300, an analyzer 102, and a controller 104, in which the sensor 1 according to the first disclosure in the analysis device 100 shown in Fig. 24 is replaced with the sensor 300 of the third disclosure. The function of this analysis device is as described for the analysis device 100 shown in Fig. 24.

### Structure of Sensor 300

The structure of the sensor 300 according to the third disclosure will be described through reference to Fig. 54 and to the cross-sectional views thereof in Figs. 55, 56 and 57. The structure of the reference electrode 20 of the sensor 300 of the third disclosure is the same as that of the reference electrode 20 of the sensor 1 of the first disclosure. Accordingly, the description of the reference electrode 20 of the sensor 1 of the first disclosure with reference to Fig. 22 is cited as the description of the reference electrode 20 of the sensor 300 of the third disclosure.

The sensor 300 comprises the insulating substrate 2, and the working electrode conductive layers 11a and 11b, the counter electrode 60, the reference electrode conductive layer 21, and the wiring 50 disposed on the first surface 2a of the substrate 2. In the embodiment of the sensor 300 according to the third disclosure shown in the drawings, the counter electrode 60 consists entirely of a conductive layer. In order to specifically refer to the conductive layer portion of the counter electrode, this can also be called the "counter electrode conductive layer" or the "conductive layer of the counter electrode." A first insulating layer 3 is further disposed on the first surface 2a of the substrate 2, and a second insulating layer 4 is further disposed on the first insulating layer 3. The height of the upper surface of the first insulating layer 3 from the first surface 2a of the substrate 2 is greater than the height of the working electrode conductive layers 11a and 11b, the counter electrode 60, the reference electrode conductive layer 21, and the wiring 50 from the first surface 2a of the substrate 2. Accordingly, part of the first insulating layer 3 is disposed on the working electrode conductive layers 11a and 11b, the counter electrode 60, the reference electrode conductive layer 21, and the wiring 50.

As shown in Fig. 55, the first insulating layer 3 comprises a counter electrode first opening 302 that is formed at a position overlapping part of the counter electrode 60 and that goes through in the thickness direction T of the substrate 2. The second insulating layer 4 comprises a counter electrode second opening 402 that is formed at a position overlapping the entire counter electrode first opening 302 of the first insulating layer 3 and that goes through in the thickness direction T of the substrate 2. The counter electrode 60 is exposed to the outside through the counter electrode first opening 302 and the counter electrode second opening 402. The portion 60a of the counter electrode 60 that comes into contact with the liquid sample when the sensor 300 is immersed in the liquid sample is located on the bottom surface of a recess formed by the counter electrode first opening 302.

As shown in Figs. 56 and 57, the working electrodes 10a and 10b include the working electrode conductive layers 11a and 11b, and the reagent layers 15a and 15b that are disposed on the working electrode conductive layers 11a and 11b and contain a reagent that participates in the oxidation-reduction reaction of the analyte. Preferred embodiments of the reagent in the reagent layers 15a and 15b are as discussed in the Materials section.

As in the first disclosure, in order to distinguish between the "reagent layer 15a" of the first working electrode 10a and the "reagent layer 15b" of the second working electrode 10b, the former may be referred to as the "first reagent layer 15a" of the first working electrode 10a, and the latter as the "second reagent layer 15b" of the second working electrode 10b. Also, the reagent contained in the reagent layer 15a of the first working electrode 10a may be referred to as the "first reagent," and the reagent contained in the second reagent layer 15b of the second working electrode 10b as the "second reagent." In a preferred embodiment, the sensor 300 comprises the plurality of working electrodes 10a and 10b, and the first reagent contained in the first reagent layer 15a of the first working electrode 10a (out of the plurality of working electrodes 10a and 10b) is different from the second reagent contained in the second reagent layer 15b of the second working electrode 10b, which is different from the first working electrode 10a. The sensor 300 in this preferred embodiment can be used to detect a plurality of analytes, including a first analyte and a second analyte, in a liquid sample, since the first reagent participates in an oxidation-reduction reaction of the first analyte in the liquid sample, and the second reagent participates in an oxidation-reduction reaction of a second analyte different from the first analyte in the liquid sample.

As shown in Figs. 56 and 57, the working electrodes 10a and 10b can include protective films 16a and 16b that are disposed on the reagent layers 15a and 15b. Preferred embodiments of the protective films 16a and 16b are as discussed in the Materials section. In the example shown in the drawings, the first protective film 16a, which consists of a single layer, is disposed on the first reagent layer 15a, and the protective film 16b, which consists of a second protective film 16ba and a third protective film 16bb, is disposed on the second reagent layer 15b, but this is not the only option, and the protective films 16a and 16b may have the same structure, or may have some other structure that is not depicted in the drawings.

As shown in Figs. 56 and 57, the first insulating layer 3 comprises working electrode first openings 303a and 303b that are formed at positions overlapping parts of the working electrode conductive layers 11a and 11b, respectively, and go through in the thickness direction T of the substrate 2. Furthermore, the second insulating layer 4 comprises working electrode second openings 403a and 403b that are formed at positions overlapping the portion of the first insulating layer 3 that entirely encompasses the working electrode first openings 303a and 303b, and go through in the thickness direction T of the substrate 2. The working electrodes 10a and 10b are exposed to the outside through the working electrode first openings 303a and 303b and the working electrode second openings 403a and 403b. The portions 11a1 and 11b1 of the working electrode conductive layers 11a and 11b that come into contact with the liquid sample when the sensor 300 is immersed in the liquid sample are located on the bottom surfaces of the recesses formed by the working electrode first openings 303a and 303b. Also, the reagent layers 15a and 15b are encompassed by the working electrode first openings 303a and 303b, and the outer peripheral edges of the reagent layers 15a and 15b are defined by the inner peripheral edges of the working electrode first openings 303a and 303b. The protective films 16a and 16b are encompassed by the working electrode second openings 403a and 403b, and the outer peripheral edges of the protective films 16a and 16b are defined by the inner peripheral edges of the working electrode second openings 403a and 403b.

### Suppression of Generation of Hydrogen Peroxide by Sensor 300

The sensor 300 according to the third disclosure is characterized in that, when immersed in a liquid sample and to electrochemically measure an analyte in the liquid sample, the generation of hydrogen peroxide during measurement is suppressed and/or the concentration of generated hydrogen peroxide is reduced, which keeps the hydrogen peroxide concentration in the liquid sample during the measurement below 15 µM. Hydrogen peroxide is known to be toxic to cells. Accordingly, when using an electrochemical sensor to continuously measure an analyte in a liquid sample containing cells, such as a cell culture medium, for an extended period of time of several days or more, it is preferable to suppress any increase in hydrogen peroxide concentration during the measurement.

With the sensor 300, the hydrogen peroxide concentration in the liquid sample can be maintained at a low concentration of less than 15 µM during measurement without causing a marked decrease in the detection sensitivity for the analyte.

Here, "electrochemically measuring an analyte in a liquid sample" refers, for example, to connecting the sensor 300 immersed in the liquid sample to a potentiostat, applying a specific potential to the working electrodes 10a and 10b with the reference electrode 20 serving as a reference, sensing the current between the working electrodes 10a and 10b and the counter electrode 60, and measuring the analyte on the basis of the sensed current. Electrochemical measurement using the sensor 300 is typically performed continuously for a period of at least several hours or several days, such as for at least 1 hour, and preferably for at least 72 hours (3 days), more preferably at least 96 hours (4 days), even more preferably at least 168 hours (7 days), and most preferably at least 240 hours (10 days). There are no particular limitations on the upper limit of the period, but the period is no longer than 336 hours (14 days), for example. With the sensor 300, the hydrogen peroxide concentration in the liquid sample during this period is kept at less than 15 µM. The hydrogen peroxide concentration in the liquid sample during the period is more preferably kept at 10 µM or less.

In a preferred embodiment of the sensor 300 characterized as above, the counter electrode 60 contains a catalyst that breaks down hydrogen peroxide in the portion 60a that comes into contact with the liquid sample. It is believed that hydrogen peroxide is generated mainly through a reaction on the counter electrode 60 in which oxygen (O₂) in the liquid sample is reduced to hydrogen peroxide (H₂O₂). The generation of hydrogen peroxide on the counter electrode 60 can be particularly problematic in the measurement of an analyte that undergoes an oxidation reaction on the working electrodes 10a and 10b. An example of the measurement of an analyte that undergoes an oxidation reaction on the working electrodes 10a and 10b is measurement using the working electrodes 10a and 10b equipped with the reagent layers 15a and 15b that contain an oxidase, dehydrogenase, or the like that will participate in the oxidation of the analyte. With the sensor 300 having the counter electrode 60 containing a catalyst that breaks down hydrogen peroxide in the portion 60a that comes into contact with the liquid sample, since oxygen (O₂) in the liquid sample is reduced to water (H₂O) on the counter electrode 60 during measurement, the generation of hydrogen peroxide is suppressed. Also, some of the reagents that may be contained in the reagent layers 15a and 15b of the working electrodes 10a and 10b involve a reaction that generates hydrogen peroxide during measurement (such as oxidase or dehydrogenase). With this embodiment, since the hydrogen peroxide generated on the working electrodes 10a and 10b is also broken down into water by the catalyst contained in the counter electrode 60, an increase in the hydrogen peroxide concentration in the liquid sample during measurement can be suppressed.

In the third disclosure, the catalyst can be one that contains a metal, for example. A catalyst containing a metal is preferable because its stability is better than that of an organic catalyst such as catalase. The catalyst containing a metal is preferably one that contains one or more elements selected from among platinum, nickel, palladium, iron, manganese, and tungsten, with a catalyst containing platinum being especially favorable. These catalysts containing a metal are preferred because they have high activity for breaking down hydrogen peroxide, and in particular, a catalyst containing platinum is especially favorable in terms of catalytic activity and stability.

In the third disclosure, the catalyst may be contained in the portion of the counter electrode that comes into contact with the liquid sample, and may be contained only in the portion that comes into contact with the liquid sample, or may be contained in the entire counter electrode. Also, the catalyst may be contained in the counter electrode as a film that covers at least the surface of the portion that comes into contact with the liquid sample, or may be contained in a state of being dispersed in a conductive material that constitutes the entire counter electrode, or at least the portion that comes into contact with the liquid sample.

In the third disclosure, examples of the counter electrode in which the catalyst is contained as a film covering the surface of the portion in contact with the liquid sample include a counter electrode obtained by forming a film of a metal that can be used as the catalyst on the surface of the portion in contact with the liquid sample by sputtering, plating, or the like, and a counter electrode obtained by coating the surface of the portion in contact with the liquid sample with a dispersion containing particles of the catalyst in a liquid medium, and then drying this coating. Of these, a counter electrode obtained by using a dispersion containing particles of the catalyst in a liquid medium is better because it can be produced by a simple method such as coating or printing. Examples of the catalyst particles include particles of the metals mentioned above, with platinum particles being especially favorable. The platinum particles are preferably platinum nanoparticles having an average particle size of less than 1 µm, such as 800 nm or less, and preferably 600 nm or less.

In the third disclosure, an example of a counter electrode in which the catalyst is contained in a state of being dispersed in the conductive material constituting the entire counter electrode, or at least the portion that comes into contact with the liquid sample, is a counter electrode obtained by using a paste or liquid composition containing particles of the catalyst and particles of a conductive material to form the entire counter electrode or at least the portion that comes into contact with the liquid sample. The counter electrode in this embodiment can be produced in just a few steps because the catalyst can be added at the same time as the counter electrode is formed on the substrate. Examples of the catalyst particles include particles of the above-mentioned metals, with platinum particles being especially favorable. A preferred embodiment of the platinum particles is as described above.

In the third disclosure, the counter electrode may contain carbon, gold, or the like as a conductive material, and it is particularly favorable for carbon to be contained as the conductive material. This carbon can be glassy carbon, carbon black, graphite, diamond-like carbon, graphene, carbon nanotubes, fullerene, or another such conductive carbon material.

In the third disclosure, the amount of catalyst contained in the counter electrode can be suitably set according to the type and activity of the catalyst. For example, if the entire counter electrode, or at least the part in contact with the liquid sample, contains carbon as the conductive material and platinum particles as the catalyst, the platinum particles can be contained in an amount of, for example, 0.5 wt% or more, preferably 1 wt% or more, more preferably 3 wt% or more, and particularly preferably 5 wt% or more, and while there are no particular limitations on the upper limit, from a cost standpoint the amount is preferably 50 wt% or less (the ratio of the platinum particles to the carbon is, for example, 0.5 wt% or more and 50 wt% or less), more preferably 30 wt% or less, and most preferably 10 wt% or less. Setting the ratio of carbon to platinum particles within this range suppresses an increase in the hydrogen peroxide concentration in the liquid sample during the measurement period.

Although not shown in the drawings, in the third disclosure, the surface of the counter electrode may be further covered with a protective film. The protective film covering the counter electrode preferably contains a polymer compound having a cation exchange functional group, and particularly a polymer compound having a cation exchange functional group in a side chain. An example of this polymer compound having a cation exchange functional group is a polymer compound containing a structural unit having a sulfonic acid group, preferably a polymer compound containing a perfluoro compound having a sulfonic acid group on a side chain as a structural unit, particularly preferably a copolymer of tetrafluoroethylene and perfluoro-2-(2-fluorosulfonylethoxy)propylvinylether, and most preferably Nafion (registered trademark). Anions such as chloride ions and organic acid ions that may be contained in the liquid sample have the potential to adversely affect the activity of the platinum catalyst or other such catalyst that breaks down the hydrogen peroxide. If the counter electrode is covered with a protective film containing a polymer compound having a cation exchange functional group, anions can be prevented from coming into contact with the counter electrode, and this suppresses a decrease in the activity of the catalyst.

On the other hand, in the sensor 300 according to the present disclosure, it is preferable for at least the portions 11a1 and 11b1 of the working electrode conductive layers 11a and 11b of the working electrodes 10a and 10b that come into contact with the liquid sample, and preferably the entire working electrode conductive layers 11a and 11b, not to contain a catalyst that decomposes hydrogen peroxide. If the working electrode conductive layers 11a and 11b of the working electrodes 10a and 10b of the sensor 300 contain this catalyst, and particularly a catalyst containing platinum, this can lead to an increase in background noise during measurement and a decrease in the current response value. Examples of the working electrode conductive layers 11a and 11b or the portions 11a1 and 11b1 that do not contain a catalyst that breaks down hydrogen peroxide include layers, or portions thereof, of conductive materials such as carbon and gold that do not contain a catalyst that breaks down hydrogen peroxide.

More preferably, the working electrode conductive layers 11a and 11b of the working electrodes 10a and 10b satisfy the following condition: when a three-electrode electrolytic cell comprising the working electrode conductive layers 11a and 11b as working electrodes, a silver-silver chloride (saturated KCl) electrode as the reference electrode, and a platinum electrode as the counter electrode is used, and a potential within the range of -0.2 V to 0.3 V (versus the silver-silver chloride (saturated KCl) electrode) is applied to the working electrode conductive layers in phosphate buffered saline, the measured current value is ±5.5 nA/mm² or less. The sensor 300 having the working electrode conductive layers 11a and 11b that satisfy this condition is preferable because the background current when measuring the analyte is sufficiently low, and the responsiveness of the sensor is high. Examples of these working electrode conductive layers 11a and 11b include carbon conductive layers containing carbon, and more preferably, carbon conductive layers that do not contain a catalyst that breaks down hydrogen peroxide, such as a catalyst containing platinum. The carbon here may be any of the carbon materials listed for the conductive material of the counter electrode 60. The fact that the current value is ±5.5 nA/mm² or less means that the absolute value of the current value per square millimeter of the projected surface area in the vertical direction of the portion of working electrode conductive layers 11a and 11b used as the working electrodes that is in contact with phosphate buffered saline is 5.5 nA or less. The measurement of the current value using the above-mentioned three-electrode electrolytic cell can be accomplished by the procedure outlined in Third Disclosure / Experiment 5 above.

The inventors further found an unanticipated effect that in the sensor 300 according to this disclosure, which is that in an embodiment in which the portion 60a of the counter electrode 60 in contact with the liquid sample contains a catalyst containing platinum, negative current with a large absolute value can flow at a low potential. Therefore, the counter electrode 60 containing a catalyst containing platinum in the portion 60a in contact with the liquid sample can efficiently function as a counter electrode with a smaller surface area than a counter electrode that does not contain a catalyst containing platinum, and this helps make the sensor more compact. Accordingly, with the sensor 300 according to this disclosure, the projected area of the counter electrode 60 is preferably 250% or less, more preferably 100% or less, and preferably 3% or more (the projected area of the counter electrode is, for example, at least 3% and no more than 250% with respect to the projected area of the working electrode conductive layer), and more preferably 10% or more with respect to the projected area of the working electrode conductive layers 11a and 11b. As shown in the drawings, the sensor 300 equipped with the counter electrode 60 containing a platinum-containing catalyst in the portion 60a in contact with the liquid sample is also suited to when a plurality of working electrodes 10a and 10b are provided. The projected area of the counter electrode 60 refers to the projected area in the vertical direction of the portion 60a of the counter electrode 60 in contact with the liquid sample (corresponding to the bottom surface of the recess formed by the counter electrode first opening 302). The projected area of the working electrode conductive layers 11a and 11b refers to the projected area in the vertical direction of the portions 11a1 and 11b1 of the working electrode conductive layers 11a and 11b in contact with the liquid sample (corresponding to the bottom surface of the recess formed by the working electrode first openings 303a and 303b), and refers to the total surface area when a plurality of working electrodes 10a and 10b are used.

### Working Example of Third Disclosure

In experiments 1 to 7 on the sensor according to the third disclosure, a sensor 300 having the configuration shown in Fig. 54 was used in both a working example and a comparative example of the sensor of the third disclosure.

The characteristics of the sensor used in each experiment will be described individually for experiments 1 to 7. An overview of the materials and manufacturing methods common to all the sensors used in the experiments will be given.

### Substrate

Just as in the working example of the first disclosure, a substrate having a thickness of 188 µm and made of polyethylene terephthalate having the shape shown in Fig. 54 was used as the insulating substrate 2.

### Conductive Layers

The specific carbon paste described in experiments 1 to 7 was applied to the first surface 2a of the insulating substrate 2 and heated at 140°C for 1 hour to form the working electrode conductive layers 11a and 11b, the reference electrode conductive layer 21, and the counter electrode (counter electrode conductive layer) 60, each having the same shape as that shown in Fig. 1 relating to the first disclosure, as well as wiring 50 electrically connected to each of these, using a carbon conductive layer with a thickness of 5 µm.

### First Insulating Layer

A first insulating layer 3, which was made of a fluororesin containing a copolymer containing vinylidene fluoride and hexafluoropropylene, which covered the first surface 2a of the substrate 2 and the carbon conductive layer, and in which the portion on the carbon conductive layer had a thickness of 5 µm, was then laminated on the first surface 2a of the substrate 2 on which the carbon conductive layer was disposed. The first insulating layer 3 was manufactured by the same method as that described in the working example of the first disclosure.

The working electrode first opening 303a in the first working electrode conductive layer 11a of the first insulating layer 3 was a circle with a diameter of 1.2 mm, and the working electrode first opening 303b in the second working electrode conductive layer 11b was a circle with a diameter of 1.4 mm. The reference electrode first opening 301 was a circle with a diameter of 1.1 mm, and the counter electrode first opening 302 was a rectangle that measured 1.8 × 2.1 mm.

### Second Insulating Layer

The first insulating layer 3 was then coated with a composition containing a polyester resin and a fluorine-based surface modification additive containing a perfluoroalkyl group in a solvent, and this coating was heated at 140°C for 1 hour to form a second insulating layer 4 having a thickness of 40 µm.

The working electrode second openings 403a and 403b of the second insulating layer 4 on the working electrode conductive layers 11a and 11b were each a circle having a diameter of 2 mm. The reference electrode second opening 401 was a circle having a diameter of 2 mm, and the counter electrode second opening 402 was a rectangle that measured 1.8 × 2.1 mm.

### First Working Electrode (for Glucose Measurement) Reagent Layer

A 0.4-µL droplet of a first liquid composition A containing a sodium phosphate buffer solution (pH 7.4), a carbon black dispersion, a polymer-bound mediator, glucose oxidase, and a crosslinking agent in water was formed in the working electrode first opening 303a of the first insulating layer 3 on the first working electrode conductive layer 11a, and then dried to form a first reagent layer 15a containing glucose oxidase and a mediator.

### Second Working Electrode (for Lactic Acid Measurement) Reagent Layer

A 0.6-µL droplet of a second liquid composition A containing a carbon black dispersion, hydroxypropyl cellulose, a polymer-bound mediator, lactate oxidase, polyimidazole, poly-L-lysine, and a crosslinking agent in water was formed in the working electrode first opening 303b of the first insulating layer 3 of the second working electrode conductive layer 11b, and dried to form a second reagent layer 15b containing lactate oxidase and a mediator.

### First Working Electrode (for Glucose Measurement) Protective Film

The following reagents were mixed with ethanol to give the following final concentrations, and the mixture was allowed to react for about 1 hour to prepare a first liquid composition B (P4VP-tBuMA polymer dispersion).
- P4VP-tBuMA (poly-4-vinylpyridine Mn: 74,000, poly-tert-butyl methacrylate Mn: 87,000, Mw/Mn: 1.16, manufactured by NARD), final concentration 5.72 wt%
- PEGDGE (poly(ethylene glycol) diglycidyl ether, Mn: 1000, manufactured by Sigma-Aldrich), final concentration 0.9 wt%

A 0.65-µL droplet of the first liquid composition B was formed in the working electrode second opening 403a of the second insulating layer 4 on the first working electrode 10a and dried, and then a 0.65-µL droplet of the first liquid composition B was further formed and dried to form a first protective film 16a.

### Second Working Electrode (for Lactic Acid Measurement) Protective Film

6472.79 mg of ethanol (manufactured by FUJIFILM Wako Pure Chemical Industries and 1,226.46 mg of a 5 mol/L aqueous sodium hydroxide solution (manufactured by FUJIFILM Wako Pure Chemical Industries) were added to 23,093.67 mg of a 21.5 wt% Nafion (registered trademark) dispersion (manufactured by Sigma-Aldrich), the pH of the dispersion was adjusted (neutralization of cation exchange groups), and the precipitate was dissolved with a vortex mixer to prepare 30,702 mg of a Nafion (registered trademark) dispersion with a concentration of 16.12 wt%. The Nafion (registered trademark) dispersion with a concentration of 16.12 wt% thus obtained was designated the second liquid composition B. This second liquid composition B contained Nafion (registered trademark) in a solvent containing a lower alcohol.

A 0.60-µL droplet of the second liquid composition B was formed in the second working electrode opening 403 b of the second insulating layer 4 on the second working electrode 10b, and then dried to form a second protective film 16ba.

A composition the same as the above-mentioned first liquid composition B (P4VP-tBuMA polymer dispersion liquid) was then used in the following treatment as a third liquid composition B. That is, a 0.65-µL droplet of the third liquid composition B was formed in the working electrode second opening 403b of the second insulating layer 4 on which the second protective film 16ba was formed, and then dried to form a third protective film 16bb. In this way, a protective film 16b in which the third protective film 16bb was laminated on the second protective film 16ba was formed on the second working electrode 10b.

### Reference Electrode

The cross-sectional structure of the reference electrode 20 is the same as that of the sensor of the first disclosure in Fig. 22, so the method for manufacturing the reference electrode 20 will be briefly described below with reference to Fig. 22. The reference electrode conductive layer 21 in the reference electrode first opening 301 of the first insulating layer 3 was coated with a silver-silver chloride paste, and this coating was heated at 140°C for 1 hour to form a silver-silver chloride layer 22. A reference electrode protective film 23 was then disposed on the silver-silver chloride layer 22 in the reference electrode second opening 401 of the second insulating layer 4 to form the reference electrode 20.

### Third Disclosure / Experiment 1

Experiment 1-1: In the above method, a carbon conductive layer was formed using an ordinary carbon paste that did not contain a catalyst such as platinum, to produce the sensor 300 in experiment 1-1. With the sensor of experiment 1-1, the working electrode conductive layers 11a and 11b, the reference electrode conductive layer 21, and the counter electrode (counter electrode conductive layer) 60 were all made of a carbon conductive layer that did not contain a catalyst that breaks down hydrogen peroxide.

Experiment 1-2: The sensor 300 of experiment 1-2 was produced by the above procedure, except that after the carbon conductive layer was formed using an ordinary carbon paste that did not contain a catalyst such as platinum, platinum was vapor-deposited only on the counter electrode 60 prior to the formation of the first insulating layer 3. In the sensor of experiment 1-2, the counter electrode 60 has the portion 60a that is exposed through the counter electrode first opening 302 of the first insulating layer 3 and the counter electrode second opening 402 of the second insulating layer 4, that is in contact with the liquid sample, and that includes a carbon conductive layer and a platinum vapor-deposited film formed over this layer, and the working electrode conductive layers 11a and 11b and the reference electrode conductive layer 21 are made of a carbon conductive layer that does not contain a catalyst such as platinum.

Phosphate buffered saline (PBS) (Takara Bio PBS Tablets T9181), glucose (manufactured by FUJIFILM Wako Pure Chemical Industries), and sodium lactic acid (manufactured by Sigma-Aldrich) were used to prepare PBS containing 30 mM glucose and 15.6 mM lactic acid, with the pH adjusted to 7.0, as a liquid sample containing glucose and lactic acid (the analytes).

The sensor of experiment 1-1 or experiment 1-2 was immersed in the liquid sample, a voltage of 100 mV was applied to the first working electrode 10a and the second working electrode 10b relative to the reference electrode 20 (Ag/AgCl), and the current value between the first working electrode 10a and the counter electrode 60 and the current value between the second working electrode 10b and the counter electrode 60 were measured for seven days in a row. The measurement was performed at N = 4.

Using the liquid sample after 7 days of measurement, a hydrogen peroxide concentration measurement sample was prepared according to the formulation shown in the following table. The absorbance at 666 nm of the hydrogen peroxide concentration measurement sample was measured for 5 minutes with a plate reader (at 37°C).

**Table 2**

| | Stock Concentration | Addition volume (µL) | Final concentration |
|---|---|---|---|
| Sodium phosphate buffer (pH 7.0) | 200 mM | 40 | 40 mM |
| DA-67 (Color reagent, FUJIFILM Wako Pure Chemical) | 0.5 mM | 50 | 0.125 mM |
| Horseradish peroxidase (Sigma-Aldrich) | 235 U/mL (ABTS conversion) | 10 | 11.75 U/mL |
| Liquid sample | | 100 | |
| Total | | 200 | |

Also, a hydrogen peroxide concentration measurement sample was prepared by compounding a solution containing a known concentration of hydrogen peroxide (manufactured by FUJIFILM Wako Pure Chemical Industries) instead of the liquid sample in the above table, and the absorbance at 666 nm was similarly measured to create a calibration curve indicating the relation between hydrogen peroxide concentration and absorbance.

The hydrogen peroxide concentration in the liquid sample was determined from the calibration curve. The hydrogen peroxide concentration thus determined was corrected for the amount of water evaporated from the liquid sample during the 7-day continuous measurement. The results are given in the following table.

**Table 3**

| | Experiment 1-1 | Experiment 1-2 |
|---|---|---|
| Counter electrode | Carbon conductive layer | Carbon conductive layer with platinum vapor deposition film |
| Hydrogen peroxide concentration (average of N = 4) | 49.94 µM | Outside detection limits |

From the above results, it was confirmed that with the sensor in experiment 1-2, which had a counter electrode made of a carbon conductive layer with a platinum vapor deposition film formed on the portion in contact with the liquid sample, the generation of hydrogen peroxide in the liquid sample during measurement of the analyte in the liquid sample was suppressed or reduced compared to the sensor in experiment 1-1, which had a counter electrode consisting of only a carbon conductive layer.

### Third Disclosure / Experiment 2

Experiment 2-1: The sensor 300 in experiment 2-1 was the same as that in experiment 1-1, in which all of the conductive layers, including the counter electrode 60, were carbon conductive layers.

Experiment 2-2: In the sensor of experiment 2-1, a platinum nanoparticle dispersion (manufactured by Sigma-Aldrich, platinum particle diameter: 70 ±6 nm) was dropped onto the surface of the counter electrode 60 exposed through the counter electrode first opening 302 and the counter electrode second opening 402, and this dispersion was dried, and then a 21.5 wt% concentration Nafion (registered trademark) dispersion (manufactured by Sigma-Aldrich) was dropped onto the surface and dried to form a protective film (not shown), thereby producing the sensor 300 of experiment 2-2. With the sensor 300 of experiment 2-2 thus produced, the portion 60a of the counter electrode 60 in contact with the liquid sample was a carbon conductive layer whose surface was modified with platinum nanoparticles, and the surface of this portion 60a was covered with a protective film (not shown) containing Nafion (registered trademark), which is a polymer compound having cation exchange functional groups.

The sensor 300 of experiment 2-1 or experiment 2-2 was immersed in a liquid sample having the composition described in experiment 1, a voltage of 100 mV was applied to the first working electrode 10a and the second working electrode 10b relative to the reference electrode 20 (Ag/AgCl), and the current value between the first working electrode 10a and the counter electrode 60 and the current value between the second working electrode 10b and the counter electrode 60 were measured for 7 days in a row. The measurement was performed at N = 2.

Using the liquid sample after 7 days of measurement, a hydrogen peroxide concentration measurement sample was prepared according to the formulation shown in the following table. The absorbance at 666 nm of the hydrogen peroxide concentration measurement sample was measured for 5 minutes with a plate reader (at 37°C).

**Table 4**

| | Stock Concentration | Addition volume (µL) | Final concentration |
|---|---|---|---|
| Sodium phosphate buffer (pH 7.0) | 200 mM | 24.5 | 24.5 mM |
| DA-67 (Color reagent, FUJIFILM Wako Pure Chemical) | 0.5 mM | 50 | 0.125 mM |
| Horseradish peroxidase (Sigma-Aldrich) | 23.5 U/mL (ABTS conversion) | 25.5 | 3 U/mL |
| Liquid sample | | 100 | |
| Total | | 200 | |

Also, a hydrogen peroxide concentration measurement sample was prepared by compounding a solution containing a known concentration of hydrogen peroxide (manufactured by FUJIFILM Wako Pure Chemical Industries, Ltd.) instead of the liquid sample in the above table, and the absorbance at 666 nm was similarly measured to create a calibration curve of hydrogen peroxide concentration and absorbance.

The hydrogen peroxide concentration in the liquid sample was determined from the calibration curve. The hydrogen peroxide concentration thus determined was corrected for the amount of water evaporated from the liquid sample during the continuous measurement for 7 days. The results are shown in the following table.

**Table 5**

| | Experiment 2-1 | 2-2 | |
|---|---|---|---|
| Counter electrode | Carbon conductive layer | Carbon conductive layer modified with platinum nanoparticle | |
| Hydrogen peroxide concentration | 47.59 µM (Average of N=2) | Outside detection limits (first sample) | 0.99µM (second sample) |

It was confirmed from the above results that with the sensor 300 of experiment 2-2, which had a counter electrode 60 consisting of a carbon conductive layer modified with platinum nanoparticles at the portion 60a in contact with the liquid sample, the generation of hydrogen peroxide in the liquid sample during measurement of the analyte in the liquid sample can be suppressed or reduced as compared to the sensor 300 of experiment 2-1, which had a counter electrode 60 consisting of only a carbon conductive layer.

### Third Disclosure / Experiment 3

Experiment 2-1: The sensor 300 in experiment 3-1 was the same as the sensors in experiments 1-1 and 2-1, in which all of the conductive layers including the counter electrode 60 were made of a carbon conductive layer.

Experiment 3-2: Sensor 300 of experiment 3-2 was produced by the procedure discussed above, except that the working electrode conductive layers 11a and 11b, the reference electrode conductive layer 21, the counter electrode (counter electrode conductive layer) 30, and the wiring 50 were formed using a carbon paste containing carbon (containing graphite and carbon black) and 1 wt% platinum particles (average particle diameter of 390 nm) relative to the carbon.

Experiment 3-3: Sensor 300 of experiment 3-3 was produced by the sensor and procedure of experiment 3-2, except that a carbon paste containing the carbon and 5 wt% platinum particles relative to the carbon was used.

The sensor of experiment 3-1, experiment 3-2, or experiment 3-3 was immersed in a liquid sample having the composition described in experiment 1, a voltage of 100 mV was applied to the first working electrode 10a and the second working electrode 10b relative to the reference electrode 20 (Ag/AgCl), and the current value between the first working electrode 10a and the counter electrode 60 and the current value between the second working electrode 10b and the counter electrode 60 were measured for 7 days in a row. The measurement was performed at N = 4.

The liquid sample after 7 days of measurement was used to prepare a hydrogen peroxide concentration measurement sample according to the formulation shown in the following table. The absorbance at 666 nm of the hydrogen peroxide concentration measurement sample was measured for 5 minutes with a plate reader (at 37°C).

**Table 6**

| | Stock Concentration | Added volume (µL) | Final concentration |
|---|---|---|---|
| Sodium phosphate buffer (pH 7.0) | 200 mM | 85 | 106.25 mM |
| DA-67 (Color reagent, FUJIFILM Wako Pure Chemical) | 2 mM | 10 | 0.1 mM |
| Horseradish peroxidase (Sigma-Aldrich) | 120 U/mL (ABTS conversion) | 5 | 3 U/mL |
| Liquid sample | | 100 | |
| Total | | 200 | |

Also, a hydrogen peroxide concentration measurement sample was prepared by compounding a solution containing a known concentration of hydrogen peroxide (manufactured by FUJIFILM Wako Pure Chemical Industries) instead of the liquid sample in the above table, and the absorbance at 666 nm was similarly measured to create a calibration curve of hydrogen peroxide concentration and absorbance.

The hydrogen peroxide concentration in the liquid sample was determined from the calibration curve. The hydrogen peroxide concentration thus determined was corrected for the amount of water evaporated from the liquid sample during the measurement for 7 days in a row. The results are shown in the following table.

**Table 7**

| | Experiment 3-1 | Experiment 3-2 | Experiment 3-3 |
|---|---|---|---|
| Counter electrode | Carbon conductive layer | Carbon conductive layer containing 1 wt% of platinum particles | Carbon conductive layer containing 5 wt% of platinum particles |
| Hydrogen peroxide concentration (average of N = 4) | 81.0 µM | 6.21 µM | 1.99 µM |

It was confirmed from the above results that with the sensors 300 in experiments 3-2 and 3-3, which had a counter electrode 60 consisting of a carbon conductive layer containing platinum particles, the generation of hydrogen peroxide in the liquid sample during measurement of the analyte in the liquid sample can be suppressed or reduced as compared to the sensor 300 in experiment 3-1, which had a counter electrode 60 consisting of only a carbon conductive layer.

### Third Disclosure / Experiment 4

The following experiments were conducted to examine the electrochemical properties of a carbon conductive layer containing no platinum (hereinafter call a "carbon electrode without platinum particles"), a carbon conductive layer containing 1 wt% platinum particles relative to the carbon ("carbon electrode containing 1% platinum particles"), and a carbon conductive layer containing 5 wt% platinum particles relative to the carbon ("carbon electrode containing 5% platinum particles"), which were used as the counter electrode 60 in experiments 3-1, 3-2, and 3-3.

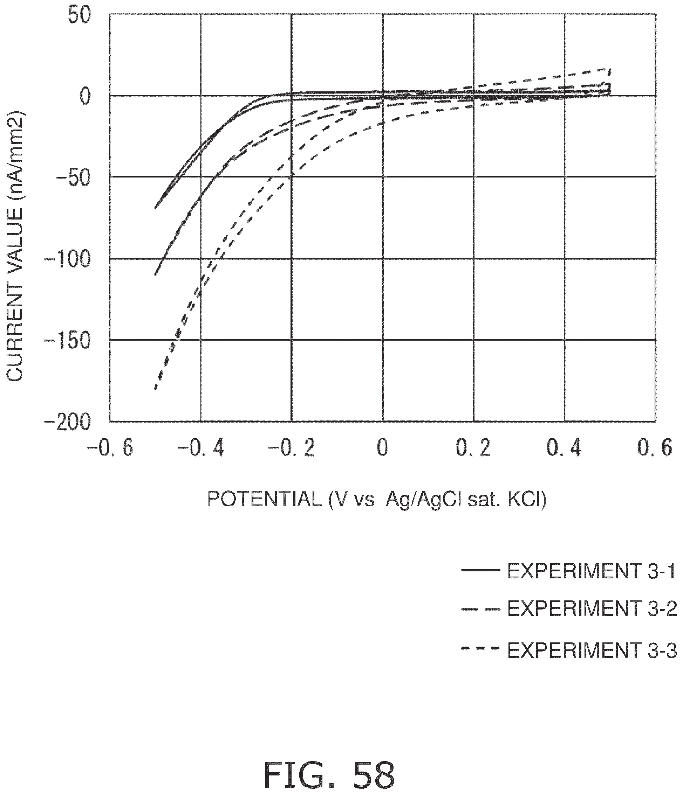

A three-electrode electrolysis cell was constructed using the carbon electrode without platinum particles, the carbon electrode containing 1% platinum particles, or the carbon electrode containing 5% platinum particles as the working electrode, a silver-silver chloride (saturated KCl) electrode (manufactured by BAS) as the reference electrode, and a platinum electrode as the counter electrode, and these electrodes were connected to a potentiostat. The working electrode, counter electrode, and reference electrode were immersed in phosphate buffered saline (PBS) (Takara Bio PBS Tablets T9181; 0.14 M NaCl, 0.0027 M KCl, 0.010 M PO₄³⁺ ) adjusted to pH 7.4 ± 0.05 (25°C), and a potential within the range of ±0.5 V in relation to the reference electrode was applied to the working electrode at a scan rate of 10 mV/s to perform cyclic voltammetry. The cyclic voltammogram thus obtained is shown in Fig. 58.

The higher is the platinum particle content of an electrode, the greater is the absolute value of the positive current value on the high potential side and the greater is the absolute value of the negative current value on the low potential side. The negative current value on the low potential side is thought to be attributable to the reduction of oxygen and the like in the PBS. This suggests that when an electrode containing platinum particles is used as a working electrode, there is a possibility that background noise will increase or the current response value will decrease, and the influence of environmental disturbances such as oxygen concentration will be more pronounced, resulting in a drop in sensitivity.

On the other hand, when an electrode containing platinum particles is used as a counter electrode, a negative current with a large absolute value can flow at a low potential (a potential with a small absolute value) in the negative potential region due to an increase in current caused by the reduction of oxygen, etc. Accordingly, an electrode containing platinum particles can function as a counter electrode efficiently as a counter electrode with a smaller surface area than a carbon electrode that does not contain any platinum, and this affords a sensor that is more compact. Also, driving the sensor of this disclosure using an electrode containing platinum particles as the counter electrode at a low potential in the negative potential region also reduces the burden on the potentiostat used for the measurement.

### Third Disclosure / Experiment 5

The following experiment was conducted to further examine the electrochemical characteristics of the carbon electrode without platinum particles and the carbon electrode containing 1% platinum particles used in experiment 4.

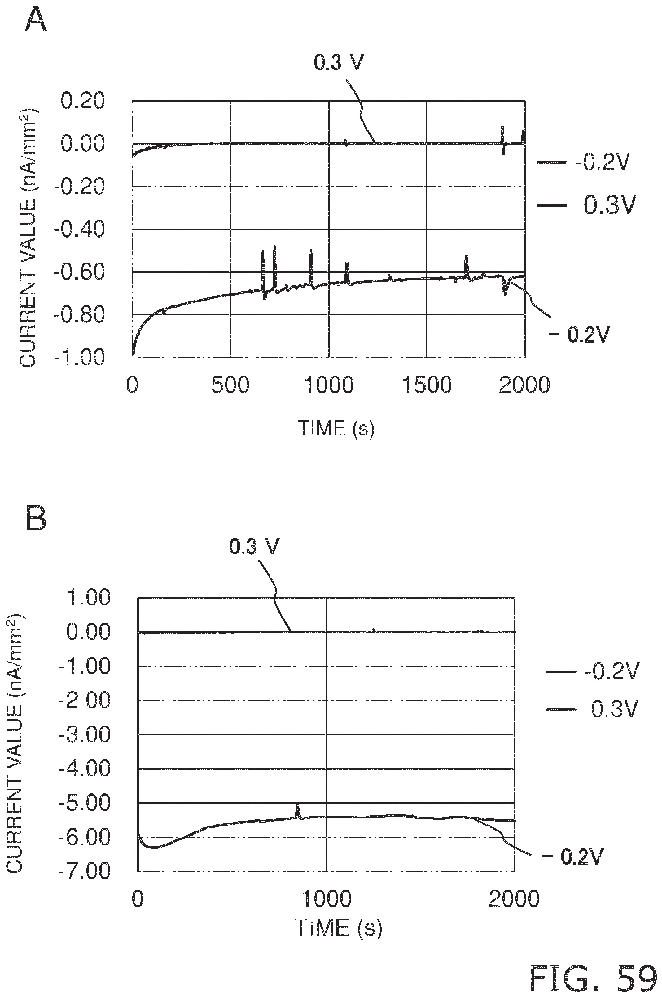

A three-electrode electrolysis cell was constructed with either a carbon electrode without platinum particles or a carbon electrode containing 1% platinum particles as the working electrode, a silver-silver chloride (saturated KCl) electrode (manufactured by BAS) as the reference electrode, and a platinum electrode as the counter electrode, and these electrodes were connected to a potentiostat. The working electrode, counter electrode, and reference electrode were immersed in phosphate buffered saline (PBS) (Takara Bio PBS Tablets T9181; 0.14 M NaCl, 0.0027 M KCl, 0.010 M PO₄³⁺) adjusted to a pH of 7.4 ±0.05 (25°C), and amperometric measurement was performed in which a potential of 0.3 V or -0.2 V was applied to the working electrode relative to the reference electrode to measure the current value over time. The measurement results of the current value per unit of surface area of the working electrode are shown in Fig. 59.

Fig. 59A shows the measurement results when a carbon electrode without platinum particles was used as the working electrode. Fig. 59B shows the measurement results when a carbon electrode containing 1% platinum particles was used as the working electrode. It was confirmed that the current value (absolute value) when a carbon electrode without platinum particles was used as the working electrode was significantly lower than the current value (absolute value) when a carbon electrode containing 1% platinum particles was used as the working electrode (the scales on the vertical axis are different for A and B). This result indicates that a conductive layer without platinum particles is suitable as the working electrode conductive layers 11a and 11b because of its low background current.

### Third Disclosure / Experiment 6

In the sensor of experiment 2-1 described above in experiment 2, all of the conductive layers including the working electrode conductive layers 11a and 11b and the counter electrode 60 are carbon conductive layers without platinum particles.

On the other hand, in the sensor of experiment 2-2, the portion 60a of the counter electrode 60 in the sensor of experiment 2-1 that comes into contact with the liquid sample to be measured is a carbon conductive layer having a surface modified with platinum nanoparticles, while the working electrode conductive layers 11a and 11b are carbon conductive layers without platinum particles.

In this experiment 6, the sensor of experiment 2-1 or experiment 2-2 was immersed in a liquid sample having the composition described in experiment 1, a voltage of 100 mV was applied to the first working electrode 10a and the second working electrode 10b relative to the reference electrode 20 (Ag/AgCl), and the current value between the first working electrode 10a and the counter electrode 60 and the current value between the second working electrode 10b and the counter electrode 60 were continuously measured for a specific length of time. The measurement was performed at N = 2.

The measurement results for the current values of the first working electrode 10a and the second working electrode 10b are shown in A and B of Fig. 60, respectively. The current value of the first working electrode 10a shown in Fig. 60A corresponds to the glucose concentration. The current value of the second working electrode 10b shown in Fig. 60B corresponds to the lactic acid concentration. No significant difference was observed in responsiveness between the sensor of experiment 2-2 in which the working electrode conductive layers 11a and 11b did not contain platinum particles and only the counter electrode 60 contained platinum particles, and the sensor of experiment 2-1 in which neither the working electrode conductive layers 11a and 11b nor the counter electrode 60 contained platinum particles.

### Third Disclosure / Experiment 7

In the sensor of experiment 3-1 described in experiment 3 above, all of the conductive layers including the working electrode conductive layers 11a and 11b and the counter electrode 60 are carbon conductive layers without platinum particles.

In the sensor of experiment 3-2, all of the conductive layers including the working electrode conductive layers 11a and 11b and the counter electrode 60 are carbon conductive layers containing platinum particles in an amount of 1 wt% relative to the carbon.

In the sensor of experiment 3-3, all of the conductive layers including the working electrode conductive layers 11a and 11b and the counter electrode 60 are carbon conductive layers containing platinum particles in an amount of 5 wt% relative to the carbon.

In this experiment 7, the sensor of experiment 3-1, experiment 3-2, or experiment 3-3 was immersed in a liquid sample having the composition described in experiment 1, a voltage of 100 mV was applied to the first working electrode 10a and the second working electrode 10b relative to the reference electrode 20 (Ag/AgCl), and the current value between the first working electrode 10a and the counter electrode 60 and the current value of the second working electrode 10b were continuously measured for a specific length of time. The measurement was performed at N = 2.

The measurement results for the current values of the first working electrode 10a and the second working electrode 10b are shown in A and B of Fig. 61, respectively. The current value of the first working electrode 10a shown in Fig. 61A corresponds to the glucose concentration. The current value of the second working electrode 10b shown in Fig. 61B corresponds to the lactic acid concentration. The sensor of experiment 3-2, in which the working electrode conductive layers 11a and 11b and the counter electrode 60 contain 1 wt% platinum particles relative to the carbon, did not show a significant decrease in the current response value as compared to the sensor of experiment 3-1, in which the working electrode conductive layers 11a and 11b and the counter electrode 60 do not contain platinum particles. On the other hand, the sensor of experiment 3-3, in which the working electrode conductive layers 11a and 11b and the counter electrode 60 contain 5 wt% platinum particles relative to the carbon, showed a clear decrease in the current response value as compared to the sensor of experiment 3-1, in which the working electrode conductive layers 11a and 11b and the counter electrode 60 do not contain platinum particles. This suggests that the presence of platinum in the working electrode conductive layers 11a and 11b reduces the responsiveness of the sensor.

### Supplemental Notes Regarding Third Disclosure

Sensors comprising a working electrode and a counter electrode for electrochemically measuring an analyte in a cell culture medium or other such liquid sample were known in the past.

A sensor such as this is sometimes used in continuous monitoring applications in which the sensor is immersed in a liquid sample and continuously measures an analyte over an extended period of time, such as several days. In this case, the sensor preferably has good detection sensitivity for the analyte and also has minimal negative effects (such as cytotoxicity) on cultured cells contained in the liquid sample.

It was found that when an analyte in a liquid sample is electrochemically measured using a sensor comprising a working electrode including a reagent layer containing a reagent that participates in the redox reaction of the analyte, such as an oxidoreductase or a mediator, and a counter electrode, hydrogen peroxide may be generated in the course of extended measurement. If the hydrogen peroxide concentration in the liquid sample goes over a certain level, this can be toxic to cells.

Therefore, it is an object of the third disclosure to provide a sensor comprising a working electrode and a counter electrode, with which the generation of hydrogen peroxide during measurement can be suppressed, or the amount of hydrogen peroxide generated can be reduced, without causing a significant decrease in sensitivity.

When the sensor according to the third disclosure is used to measure an analyte in a liquid sample such as a cell culture medium, the generation of hydrogen peroxide during measurement can be suppressed, or the amount of hydrogen peroxide generated can be reduced, without causing a significant decrease in sensitivity, so it is less likely that there will be an increase in the hydrogen peroxide concentration in the liquid sample. Accordingly, the sensor according to the third disclosure has minimal negative effects on cells in the liquid sample and can be favorably used for continuous monitoring over an extended period.

In view of this, this specification discloses, as a third disclosure, the sensor described in one or more of the supplementary notes 3-1 to 3-16 below.

### Supplementary Note 3-1

A sensor for measuring an analyte in a liquid sample, comprising:
a working electrode including a working electrode conductive layer and a reagent layer that is disposed on the working electrode conductive layer and contains a reagent that will participate in an oxidation-reduction reaction of the analyte; and
a counter electrode,
wherein when the sensor is immersed in the liquid sample and the analyte in the liquid sample is electrochemically measured, the hydrogen peroxide concentration in the liquid sample is maintained at less than 15 µM during measurement.

### Supplementary Note 3-2

The sensor according to supplementary note 3-1, wherein the counter electrode contains a catalyst that breaks down hydrogen peroxide in the portion that comes into contact with the liquid sample.

### Supplementary Note 3-3

The sensor according to supplementary note 3-2, wherein the working electrode conductive layer does not contain a catalyst that breaks down hydrogen peroxide in the portion that comes into contact with the liquid sample.

### Supplementary Note 3-4

The sensor according to supplementary note 3-2 or 3-3, wherein the catalyst contains a metal.

### Supplementary Note 3-5

The sensor according to supplementary note 3-4, wherein the metal includes one or more selected from among platinum, nickel, palladium, iron, manganese, and tungsten.

### Supplementary Note 3-6

The sensor according to supplementary note 3-4, wherein the metal includes platinum. Supplementary Note 3-7

The sensor according to supplementary note 3-6, wherein the platinum is platinum particles.

### Supplementary Note 3-8

The sensor according to supplementary note 3-7, wherein the portion of the counter electrode that comes into contact with the liquid sample includes carbon and at least 1 wt% platinum particles relative to the carbon.

### Supplementary Note 3-9

The sensor according to supplementary note 3-8, wherein the portion of the counter electrode that comes into contact with the liquid sample includes the carbon and the 50 wt% or less platinum particles relative to the carbon.

### Supplementary Note 3-10

The sensor according to any of supplementary notes 3-1 to 3-9, wherein the working electrode conductive layer satisfies the condition that the current value measured when a potential in the range of -0.2 V to 0.3 V (versus the silver-silver chloride (saturated KCl) electrode) is applied to the working electrode conductive layer in phosphate buffered saline using a three-electrode electrolytic cell comprising the working electrode conductive layer as a working electrode, a silver-silver chloride (saturated KCl) electrode as a reference electrode, and a platinum electrode as a counter electrode is ±5.5 nA/mm² or less.

### Supplementary Note 3-11

The sensor according to supplementary note 3-10, wherein the working electrode conductive layer is a carbon conductive layer.

### Supplementary Note 3-12

The sensor according to any of supplementary notes 3-1 to 3-11, wherein the projected area of the counter electrode is no more than 250% of the projected area of the working electrode conductive layer.

### Supplementary Note 3-13

The sensor according to supplementary note 3-12, wherein the projected area of the counter electrode is no more than 100% of the projected area of the working electrode conductive layer.

### Supplementary Note 3-14

The sensor according to supplementary note 3-12 or 3-13, comprising a plurality of the working electrodes.

### Supplementary Note 3-15

The sensor according to any of supplementary notes 3-1 to 3-14, further comprising a protective film that covers the counter electrode.

### Supplementary Note 3-16

The sensor according to supplementary note 3-15, wherein the protective film includes a polymer compound having a cation exchange functional group.

### Embodiment of Fourth Disclosure

An embodiment of the sensor according to the fourth disclosure will now be described. In this embodiment, some unnecessarily detailed description may be omitted. For example, detailed description of already known facts or redundant description of components that are substantially the same may be omitted. This is to avoid unnecessary repetition in the following description, and facilitate an understanding on the part of a person skilled in the art. The components in the sensor according to the fourth disclosure that are the same as in the sensor according to the first disclosure will be numbered the same. See the description of the corresponding components in the sensor according to the first disclosure.

Also, the applicant has provided the appended drawings and the following description so that a person skilled in the art might fully understand this fourth disclosure, but does not intend for these to limit what is discussed in the following "Supplementary Notes Regarding Fourth Disclosure."

### Materials

Examples of materials that can be used in the sensor of the fourth disclosure in this specification are described below.

There are no particular limitations on the material of the insulating substrate used in the sensor of the fourth disclosure of this specification, but for example, the same material as the insulating substrate of the sensor in the first disclosure of this specification can be used.

The conductive layer of the working electrode, reference electrode, and counter electrode of the sensor of the fourth disclosure of this specification is a layer containing a conductive material such as carbon, gold, platinum, or palladium. The conductive layer can be produced by forming a layer of this conductive material on the surface of the substrate by sputtering, vapor deposition, screen printing, or another such method. The conductive layer can be worked into a specific pattern using laser trimming as necessary. In the fourth disclosure, the conductive layer of the working electrode will sometimes be referred to as the working electrode conductive layer, and the conductive layer of the reference electrode as the reference electrode conductive layer. In the fourth disclosure, the conductive layer of the counter electrode is simply referred to as the counter electrode because the conductive layer itself constitutes the counter electrode.

The wiring of the sensor of the fourth disclosure of this specification can also be made of the same conductive material as that of the conductive layer.

The sensor of the fourth disclosure of this specification is used to detect a specific analyte in a liquid sample by being immersing in the liquid sample. The liquid sample is preferably one that contains water as a solvent. Examples of the liquid sample include a cell culture medium and a liquid sample prepared using blood collected from a living organism. Examples of the analyte are as described with respect to the analyte detected with the sensor in the first disclosure of this specification, and it is particularly favorable for this substance to be glucose and/or lactic acid.

The working electrode of the sensor of the fourth disclosure of this specification comprises a reagent layer that is disposed on the working electrode conductive layer and contains a reagent that participates in a redox reaction of the analyte. This reagent can be appropriately selected to suit the analyte. This reagent can be a combination of an oxidoreductase and a mediator (electron transfer substance), or include an oxidoreductase. The oxidoreductase can include a coenzyme.

The oxidoreductase may be an oxidase or a dehydrogenase. Specific examples of the oxidoreductase are as described for the sensor in the first disclosure of this specification, and it is particularly favorable for the oxidoreductase to be glucose oxidase, lactate oxidase, glucose dehydrogenase, or lactate dehydrogenase.

Also, there are no particular limitations on the mediator, but examples thereof are as described for the sensor in the first disclosure of this specification.

The reagent layer in the working electrode of the sensor of the fourth disclosure of this specification may further contain, in addition to the reagent, a buffer, a hydrophilic polymer compound, a conductive carbon filler, a crosslinking agent, or other such components. Examples of the hydrophilic polymer compound and the conductive carbon filler are as described for the sensor in the first disclosure of this specification.

The working electrode of the sensor of the fourth disclosure of this specification further comprises a first protective film disposed on the reagent layer and a second protective film disposed on the first protective film. The first protective film and the second protective film will sometimes be collectively referred to as a protective film. The protective film on the reagent layer can be any film that prevents or suppresses leakage of the reagent contained in the reagent layer to the outside of the protective film and allows the analyte present outside of the protective film to permeate. Preferred embodiments of the material constituting the first protective film and the second protective film on the reagent layer having such properties will be described below.

The reference electrode of the sensor disclosed in the fourth specification may have a protective film. The protective film of the reference electrode may contain a polymer compound. Examples of this polymer compound include the polymer compounds discussed below that are contained in the first protective film or the second protective film disposed on the reagent layer of the working electrode, and the polymer compound contained in the second protective film is particularly favorable.

In the sensor of the fourth disclosure of this specification, the electrodes including the working electrode and the counter electrode disposed on the insulating substrate are preferably covered with an insulating layer, except for the portions in contact with the liquid sample. The insulating layer may contain an insulating resin. The insulating layer may have a multilayer structure consisting of two or more layers. The insulating layer of the multilayer structure may contain, for example, a first insulating layer that is disposed on the insulating substrate and the conductive layer, and a second insulating layer that is disposed on the first insulating layer. Preferred embodiments of the materials constituting the first insulating layer and the second insulating layer are as given for the sensor according to the first disclosure.

### Overview of Sensor 400

A sensor 400 according to an embodiment of the fourth disclosure will be described through reference to the drawings.

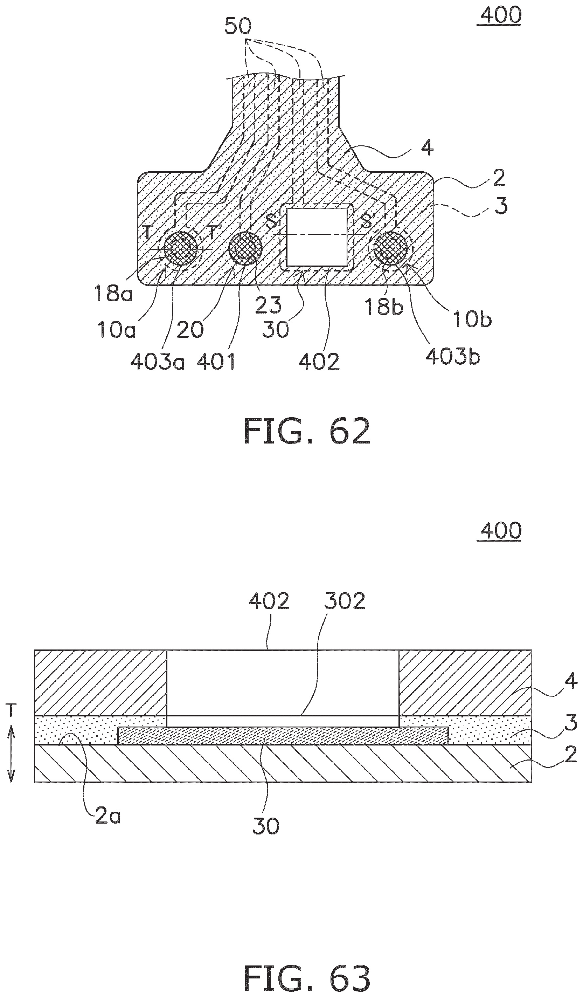

As shown in Figs. 62 to 64, the sensor 400 in this embodiment comprises the insulating substrate 2, the first working electrode 10a, the second working electrode 10b, the reference electrode 20, the counter electrode 30 disposed on a first surface 2a of the substrate 2, and the wiring 50 electrically connected to each of the first working electrode 10a, the second working electrode 10b, the reference electrode 20, and the counter electrode 30. The sensor 400 of this embodiment includes two working electrodes, but in another embodiment (not shown), the number of working electrodes may be only one, or may be three or more. In the following description, when the first working electrode 10a and the second working electrode 10b are not distinguished from one another, they will sometimes be referred to as the working electrodes 10a and 10b. Also, the first working electrode 10a, the second working electrode 10b, the reference electrode 20, and the counter electrode 30 may be collectively referred to as the electrodes. Also, the sensor 400 in this embodiment is a three-electrode sensor including a working electrode, a reference electrode, and a counter electrode as electrodes, but may be a two-electrode sensor including only a working electrode and a counter electrode, without having a reference electrode. Although not depicted in the drawings, the reference electrode and/or the counter electrode may be provided on a substrate separate from the substrate on which the working electrode is disposed.

Just as shown in Fig. 21 for the sensor 1 of the first disclosure, the sensor 400 is immersed in a liquid sample X and used to detect a specific analyte in the liquid sample X. Specific examples of the liquid sample and the analyte are as described in the Materials section.

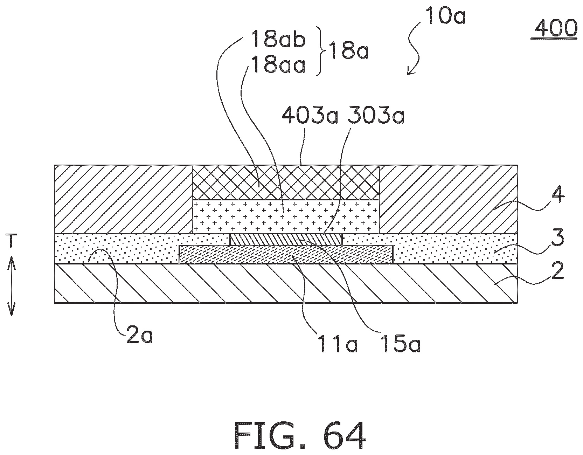

As shown in Fig. 64, the first working electrode 10a of the sensor 400 comprises the working electrode conductive layer 11a, the reagent layer 15a containing a reagent that participates in the oxidation-reduction reaction of the analyte in the liquid sample, a first protective film 18aa that is disposed on the reagent layer 15a, and a second protective film 18ab that is disposed on the first protective film 18aa. Preferred embodiments of the reagent are as described in the Materials section. Although not shown in the drawings, the cross-sectional structure of the second working electrode 10b of the sensor 400 is substantially the same as the cross-sectional structure of the first working electrode 10a shown in Fig. 64, and in Fig. 64, the first working electrode 10a is replaced by the second working electrode 10b, the working electrode conductive layer 11a is replaced by the working electrode conductive layer 11, the reagent layer 15a is replaced by the reagent layer 15b, the first protective film 18aa is replaced by the first protective film 18ba, the second protective film 18ab is replaced by the second protective film 18bb, and the protective film 18a is replaced by the protective film 18b.

When the reagent layers 15a and 15b of the working electrodes 10a and 10b of the sensor 400 contain a reagent that oxidizes the analyte in the liquid sample, electrons move from the analyte to the working electrode conductive layers 11a and 11b under conditions in which a specific voltage is applied to the electrodes of the sensor 400. Similarly, when the reagent layers 15a and 15b contain a reagent that reduces the analyte in the liquid sample, electrons move from the working electrode conductive layers 11a and 11b to the analyte. Since the amount of electrons that move depends on the concentration of the analyte, the concentration, or a change in the concentration, of the analyte in the liquid sample can be measured on the basis of the value of the current, or a change in the current, flowing through the working electrodes 10a and 10b of the sensor 400.

The sensor 400 can constitute an analysis device for analyzing an analyte in a liquid sample. An example of the analysis device is one that comprises the sensor 400, an analyzer 102, and a controller 104, in which the sensor 1 according to the first disclosure in the analysis device 100 shown in Fig. 24 is replaced with the sensor 400 according to the fourth disclosure. The function of this analysis device is as described for the analysis device 100 shown in Fig. 24.

### Structure of Sensor 400

The structure of the sensor 400 according to the fourth disclosure will be described through reference to Fig. 62 and to Figs. 63 and 64, which are cross-sectional views of Fig. 62. The structure of the reference electrode 20 of the sensor 400 according to the fourth disclosure is the same as that of the reference electrode 20 of the sensor 1 according to the first disclosure. Therefore, the description of the reference electrode 20 of the sensor 1 according to the first disclosure with reference to Fig. 22 is cited as a description of the reference electrode 20 of the sensor 400 according to the fourth disclosure.

The sensor 400 comprises then insulating substrate 2, and the working electrode conductive layers 11a and 11b, the counter electrode 30, the reference electrode conductive layer 21, and the wiring 50 that are disposed on the first surface 2a of the substrate 2. In the illustrated embodiment of the sensor 400 according to the fourth disclosure, the counter electrode 30 consists entirely of a conductive layer. In order to specifically refer to the conductive layer portion of the counter electrode, it may also be referred to as the "counter electrode conductive layer" or a "conductive layer of the counter electrode." A first insulating layer 3 is further disposed on the first surface 2a of the substrate 2, and a second insulating layer 4 is further disposed on the first insulating layer 3. The height of the upper surface of the first insulating layer 3 from the first surface 2a of the substrate 2 is greater than the height of the working electrode conductive layers 11a and 11b, the counter electrode 30, the reference electrode conductive layer 21, and the wiring 50 from the first surface 2a of the substrate 2. Therefore, part of the first insulating layer 3 is disposed on the working electrode conductive layers 11a and 11b, the counter electrode 30, the reference electrode conductive layer 21, and the wiring 50.

As shown in Fig. 63, the first insulating layer 3 comprises a counter electrode first opening 302 that is formed at a position overlapping a part of the counter electrode 30 and goes through in the thickness direction T of the substrate 2. Furthermore, the second insulating layer 4 comprises a counter electrode second opening 402 that is formed at a position overlapping the entire counter electrode first opening 302 of the first insulating layer 3 and goes through in the thickness direction T of the substrate 2. The counter electrode 30 is exposed to the outside through the counter electrode first opening 302 and the counter electrode second opening 402. When the sensor 400 is immersed in the liquid sample, the portion of the counter electrode 30 located on the bottom surface of the recess formed by the counter electrode first opening 302 comes into contact with the liquid sample.

As shown in Fig. 64, the working electrodes 10a and 10b include the working electrode conductive layers 11a and 11b and the reagent layers 15a and 15b that are disposed on the working electrode conductive layers 11a and 11b and contain a reagent that will participate in the oxidation-reduction reaction of the analyte. Preferred embodiments of the reagent in the reagent layers 15a and 15b are as described in the Materials section.

As in the first disclosure, in order to distinguish between the "reagent layer 15a" of the first working electrode 10a and the "reagent layer 15b" of the second working electrode 10b, the former will sometimes be called the "first reagent layer 15a" of the first working electrode 10a, and the latter the "second reagent layer 15b" of the second working electrode 10b. Also, the reagent contained in the reagent layer 15a of the first working electrode 10a will sometimes be called the "first reagent," and the reagent contained in the second reagent layer 15b of the second working electrode 10b will sometimes be called the "second reagent." In a preferred embodiment, the sensor 400 comprises a plurality of working electrodes 10a and 10b, and the first reagent contained in the first reagent layer 15a of the first working electrode 10a (out of the plurality of working electrodes 10a and 10b) is different from the second reagent contained in the second reagent layer 15b of the second working electrode 10b, which is different from the first working electrode 10a. The sensor 400 according to this preferred embodiment can be used to detect a plurality of analytes, including a first analyte and a second analyte, in a liquid sample, since the first reagent participates in an oxidation-reduction reaction of a first analyte in the liquid sample, and the second reagent participates in an oxidation-reduction reaction of a second analyte that is different from the first analyte in the liquid sample.

As shown in Fig. 64, the working electrodes 10a and 10b comprise the protective films 18a and 18b, which consist of the first protective films 18aa and 18ba disposed on the reagent layers 15a and 15b, and the second protective films 18ab and 18bb disposed on the first protective films 18aa and 18ba.

As shown in Fig. 64, the first insulating layer 3 comprises working electrode first openings 303a and 303b that are formed at positions overlapping parts of the working electrode conductive layers 11a and 11b, respectively, and that go through in the thickness direction T of the substrate 2. Furthermore, the second insulating layer 4 comprises working electrode second openings 403a and 403b that are formed at positions overlapping parts of the first insulating layer 3 that entirely encompass the working electrode first openings 303a and 303b, and that go through in the thickness direction T of the substrate 2. The reagent layers 15a and 15b are encompassed by the working electrode first openings 303a and 303b, and the outer peripheral edges of the reagent layers 15a and 15b are defined by the inner peripheral edges of the working electrode first openings 303a and 303b. The first protective films 18aa and 18ba and the second protective films 18ab and 18bb are encompassed by the working electrode second openings 403a and 403b, and the outer peripheral edges of the first protective films 18aa and 18ba and the second protective films 18ab and 18bb are defined by the inner peripheral edges of the working electrode second openings 403a and 403b.

### Features of First and Second Protective Films of Sensor 400

The sensor 400 according to the fourth disclosure is characterized in that the first protective films 18aa and 18ba contain a first polymer compound containing a cation exchange functional group, and the second protective films 18ab and 18bb contain a second polymer compound containing a cationic functional group. When a sensor having these characteristics is used for continuous monitoring applications in which an analyte is continuously measured over an extended period of time of several days or more in a state of being immersed in a liquid sample, the first protective film and the second protective film will still not peel off, allowing for stable measurement.

There are known sensors including a protective film having a two-layer structure, in which a first protective film containing a polymer compound containing a cation exchange functional group (such as a copolymer compound of tetrafluoroethylene and perfluoro-2-(2-fluorosulfonylethoxy)propyl vinyl ether) for transporting protons or other such cations between the reagent layer and the liquid sample is provided on the reagent layer, and a second protective film containing a polymer compound containing 4-vinylpyridine as a structural unit (such as P4VP-tBuMA (a block copolymer compound of poly-4-vinylpyridine and poly-tert-butyl methacrylate)) is further provided on the first protective film. The inventors found that when this sensor is immersed in a liquid sample to perform continuous electrochemical measurements of an analyte, after a long period of time of more than three days has elapsed, an abnormal value is detected in which the detected current value changes markedly, making it impossible to perform accurate measurement. It was found that in a sensor indicating an abnormal value, peeling had occurred between the first protective film and the second protective film.

As a result of diligent investigation, the inventors found that the above problem can be solved by the sensor according to the fourth disclosure, which comprises the first and second protective films having the above characteristics. It is believed that because the first polymer compound of the first protective film contains a cation exchange functional group (that is, an anionic functional group), while the second polymer compound of the second protective film contains a cationic functional group, the first and second protective films are bonded together by electrostatic bonding at their interface, making peeling less likely to occur.

In the fourth disclosure, the first polymer compound contained in the first protective film may have a cation exchange functional group, and preferably has a cation exchange functional group in a side chain. This cation exchange functional group is preferably a sulfonic acid group. The first polymer compound having a sulfonic acid group in a side chain is preferably a polymer compound containing as a structural unit a perfluoro compound having a sulfonic acid group in a side chain, more preferably a copolymer compound containing as a structural unit a perfluoro compound having a sulfonic acid group in a side chain and a perfluoro compound not having an ionic functional group in a side chain, with a copolymer compound of tetrafluoroethylene and perfluoro-2-(2-fluorosulfonylethoxy)propyl vinyl ether being especially favorable, and with Nafion (registered trademark) being the most favorable.

In the fourth disclosure, the second polymer compound contained in the second protective film may contain a cationic functional group, and preferably has a cationic functional group in a side chain. Since the pH of the liquid sample to be analyzed may fluctuate during measurement over an extended period, the cationic functional group in the second polymer compound is preferably a pH-independent cationic functional group, with a functional group containing a quaternary ammonium cation being especially favorable. The quaternary ammonium cation preferably originates in a reaction between a tertiary amine and an epoxy group. Examples of tertiary amines include a pyridyl group, with a 4-pyridyl group being particularly favorable.

The second polymer compound preferably includes a first unit containing a cationic functional group, and a second unit containing a hydrophobic functional group, and the ratio of the second unit to the total amount of the units constituting the second polymer compound is at least 50 mol%. Since this second polymer compound is highly hydrophobic, when the sensor according to the fourth disclosure is immersed in an aqueous liquid sample such as a cell culture medium and measured for an extended period of time, the second protective film will still not swell with water, making peeling at the interface with the first protective film less likely to occur. There are no particular limitations on the upper limit of the content of the second unit containing a hydrophobic functional group in the second polymer compound, but for example, the ratio of the second unit to the total amount of the units constituting the second polymer compound can be 70 mol% or less. Examples of hydrophobic functional groups include a saturated hydrocarbon group having 1 to 10 carbon atoms. For example, a second unit derived from an ester of a methacrylic acid or acrylic acid with a monohydric alcohol containing a saturated hydrocarbon group having 1 to 10 carbon atoms is preferred, and more specifically, tert-butyl methacrylate.

The second polymer compound containing a first unit containing a cationic functional group, and a second unit containing a hydrophobic functional group can be obtained, for example, by allowing an uncrosslinked polymer compound containing a third unit containing a tertiary amine and the second unit containing a hydrophobic functional group to react for a certain period of time with a crosslinking compound containing two or more epoxy groups, thereby reacting the tertiary amine of the third unit with the epoxy group of the crosslinking compound to convert the third unit into the first unit containing a quaternary ammonium cation. In this case, the reaction preferably uses the epoxy groups of the crosslinking compound in an amount of at least 6.8 mol%, and more preferably no more than 23 mol%, with respect to the tertiary amine in the third unit (for example, the epoxy groups of the crosslinking compound will account of at least 6.8 mol% and no more than 23 mol% with respect to the tertiary amine in the third unit), so that the obtained second polymer compound has a high density of quaternary ammonium cations, which is preferable because the electrostatic bond between the first protective film and the second protective film will be stronger. Here, in calculating the ratio (mol%) of the epoxy groups of the crosslinking compound to the tertiary amines of the third unit in the uncrosslinked polymer compound, the number of moles of the tertiary amine can be calculated on the basis of the molecular weight of the monomer of the third unit, and the number of moles of the epoxy group can be calculated on the basis of the molecular weight of the crosslinking compound (if the crosslinking compound is a polymer compound, its number average molecular weight is considered to be a uniform molecular weight).

In the uncrosslinked polymer compound, the third unit containing a tertiary amine is preferably a structural unit derived from 4-vinylpyridine.

A preferred example of the uncrosslinked polymer compound is a copolymer compound of 4-vinylpyridine and tert-butyl methacrylate. More preferably, this copolymer compound contains the second unit derived from tert-butyl methacrylate in an amount of at least 50 mol%, and more preferably no more than 70 mol% with respect to the total amount of the third unit derived from 4-vinylpyridine and the second unit derived from tert-butyl methacrylate (the ratio of the second unit to the total amount of the third unit and the second unit is, for example, at least 50 mol% and no more than 70 mol%). It is especially favorable for the copolymer compound of 4-vinylpyridine and tert-butyl methacrylate to be a block copolymer compound. There are no particular limitations on the molecular weight of the uncrosslinked polymer compound, but this may be, for example, a number average molecular weight (Mn) of 150,000 to 500,000.

The crosslinking compound containing two or more epoxy groups is, for example, a linear compound having an epoxy group at each of its two ends, and is preferably a polyalkylene glycol compound in which both ends are modified with a glycidyl group, and more specifically, poly(ethylene glycol) diglycidyl ether. There are no particular limitations on the molecular weight of the poly(ethylene glycol) diglycidyl ether, but this may be a number average molecular weight (Mn) of 200 to 2,000, for example.

### Method for Manufacturing Sensor 400

A preferred method for manufacturing the sensor 400 according to the third disclosure, in which the second protective films 18ab and 18bb contain a second polymer compound containing a first unit containing a quaternary ammonium cation derived from a reaction between a tertiary amine and an epoxy group, and a second unit containing a hydrophobic functional group, comprises:
a first step of obtaining a composition for forming a second protective film, in which a mixture, in alcohol, of an uncrosslinked polymer compound containing a third unit including a tertiary amine and a second unit including a hydrophobic functional group, and a crosslinking compound including two or more epoxy groups is held for at least 6 hours at a temperature of at least 20°C to react the tertiary amines of the third unit with the epoxy groups of the crosslinking compound, convert the third unit into the first unit including the quaternary ammonium cation, and obtain a composition for forming a second protective film containing the second polymer compound including the first unit and the second unit in alcohol; and
a second step of covering first protective films 18aa and 18ba of a sensor component comprising:
   a substrate 2;
   working electrode conductive layers 11a and 11b disposed on the substrate 2;
   reagent layers 15a and 15b disposed on the working electrode conductive layers 11a and 11b; and
   the first protective films 18aa and 18ba disposed on the reagent layers 15a and 15b with the composition for forming a second protective film,
   and drying this coating to form second protective films 18ab and 18bb containing the second polymer compound.

With this method, in the first step, the crosslinking reaction between the tertiary amines of the third unit in the uncrosslinked polymer compound and the epoxy groups of the crosslinking compound proceeds thoroughly enough for a composition for forming a second protective film, which contains a second polymer compound having a high density of quaternary ammonium cations, to be obtained. The temperature in the first step is preferably at least 30°C, and more preferably at least 40°C, with the upper limit preferably being below the boiling point of the alcohol. For example, when ethanol is used as the alcohol, the temperature is preferably below 78°C. The reaction time in the first step is preferably at least 12 hours, and while there are no particular limitations on the upper limit, this period lasts, for example, 48 hours or less (the reaction time in the first step is, for example, 12 hours or more and 48 hours or less). The alcohol is preferably ethanol, methanol, or propanol, with ethanol being especially favorable. Preferred embodiments of the uncrosslinked polymer compound and the crosslinking compound are as described above.

In the second step, the composition for forming a second protective film is applied or dropped onto the first protective films 18aa and 18ba to cover them, and this coating is then dried by evaporating the alcohol solvent to form the second protective films 18ab and 18bb.

With this method, since crosslinking is performed in advance in the first step, there is no need to perform any additional crosslinking treatment after the second protective films 18ab and 18bb have been formed in the second step. This reduces the risk that the reagent layers 15a and 15b containing reagents such as enzymes will be damaged by heat during the crosslinking treatment.

### Working Example of Fourth Disclosure

In experiments 8 to 10 of the sensor according to the fourth disclosure, the sensor 400 having the configuration shown in Fig. 62 that does not include the second working electrode 10b and only includes the first working electrode 10a (hereinafter referred to as "working electrode 10a") was used as a working example and a comparative example of the sensor according to the fourth disclosure.

The characteristics of the sensors used in these experiments will be described individually in experiments 8 to 10. First, an overview of the materials and manufacturing methods common to all the sensors used the experiments will be described.

### Substrate

Just as in the working example of the first disclosure, a substrate having a thickness of 188 µm and made of polyethylene terephthalate with the shape shown in Fig. 62 was used as the insulating substrate 2.

### Conductive Layer

A carbon paste was applied onto the first surface 2a of the insulating substrate 2, and this was heated at 140°C for 1 hour to form a working electrode conductive layer 11a, a reference electrode conductive layer 21, and a counter electrode (counter electrode conductive layer) 30 having the same shapes as those shown in Fig. 1 related to the first disclosure, as well as wiring 50 electrically connected to each of these, using a carbon conductive layer with a thickness of 5 µm.

### First Insulating Layer

A first insulating layer 3 that was made of a fluororesin containing a copolymer containing vinylidene fluoride and hexafluoropropylene was then laminated on the first surface 2a of the substrate 2, on which the carbon conductive layer was disposed, to cover the first surface 2a of the substrate 2 and the carbon conductive layer. The first insulating layer 3 had a thickness of 5 µm on the carbon conductive layer. The method for manufacturing the first insulating layer 3 was as described in the working example of the first disclosure.

The working electrode first opening 303a on the working electrode conductive layer 11a of the first insulating layer 3 was a circle with a diameter of 1.2 mm. The reference electrode first opening 301 was a circle with a diameter of 1.1 mm, and the counter electrode first opening 302 was a rectangle that measured 1.8 × 2.1 mm.

### Second Insulating Layer

The first insulating layer 3 was then coated with a composition containing a polyester resin and a fluorine-based surface modification additive containing a perfluoroalkyl group in a solvent, and this coating was heated at 140°C for 1 hour to form a second insulating layer 4 having a thickness of 40 µm.

The working electrode second opening 403a of the second insulating layer 4 on the working electrode conductive layer 11a was a circle with a diameter of 2 mm. The reference electrode second opening 401 was a circle with a diameter of 2 mm, and the counter electrode second opening 402 was a rectangle that measured 1.8 mm × 2.1 mm.

### Working Electrode Reagent Layer

In the working electrode first opening 303a of the first insulating layer 3, the working electrode conductive layer 11a was coated with 0.4 mg of a glucose measurement reagent solution containing a sodium phosphate buffer solution (pH 7.4) with a final concentration of 3 mM, a carbon black dispersion with a final concentration of 14 mg/mL (carbon black concentration), a polymer-bound mediator, an FAD-dependent glucose dehydrogenase with a final concentration of 3200 U/mL, and a crosslinking agent with a final concentration of 0.1 mM (concentration of binding sites), and this coating was dried to form a reagent layer 15a containing glucose dehydrogenase and a mediator.

### Working Electrode Protective Film

In the working electrode second opening 403a of the second insulating layer 4, the first protective film 18aa was formed on the reagent layer 15a, and the second protective film 18ab was formed on the first protective film 18aa to form the protective film 18a having a two-layer structure consisting of the first protective film 18aa and the second protective film 18ab. The specific manufacturing methods for the first protective film 18aa and the second protective film 18ab are described in experiments 8 to 10.

### Reference Electrode

The cross-sectional structure of the reference electrode 20 is the same as that of the sensor of the first disclosure in Fig. 22, so an overview of the method for manufacturing the reference electrode 20 will be given below with reference to Fig. 22. A silver-silver chloride paste was applied onto the reference electrode conductive layer 21 in the reference electrode first opening 301 of the first insulating layer 3, and this paste was heated at 140°C for 1 hour to form a silver-silver chloride layer 22. A reference electrode protective film 23 was then disposed on the silver-silver chloride layer 22 in the reference electrode second opening 401 of the second insulating layer 4 to form the reference electrode 20.

### Fourth Disclosure / Experiment 8

### Working Electrode Protective Film

6472.79 mg of ethanol (manufactured by FUJIFILM Wako Pure Chemical Industries) and 1,226.46 mg of 5 mol/L sodium hydroxide aqueous solution (manufactured by FUJIFILM Wako Pure Chemical Industries) were added to 23,093.67 mg of a 21.5 wt% Nafion (registered trademark) dispersion (manufactured by Sigma-Aldrich), the pH of the dispersion was adjusted (neutralization of cation exchange groups), and the precipitate was dissolved with a vortex mixer to prepare 30,792.92 mg of a 16.12 wt% Nafion (registered trademark) dispersion. The reagent layer 15a in the working electrode second opening 403a of the second insulating layer 4 was coated with 0.6 mg of the 16.12 wt% Nafion (registered trademark) dispersion thus obtained, and this coating was dried to form a first protective film 18aa containing Nafion (registered trademark) as a polymer compound containing a cation exchange functional group.

Two types of composition for forming a second protective film were then prepared by mixing the following two components in ethanol to achieve the final concentrations given below, and allowing this mixture to react at room temperature (25°C ±3°C) for about 1 hour. P4VP-tBuMA is a block copolymer of poly-4-vinylpyridine (P4VP) of a specific molecular weight and poly-tert-butyl methacrylate (tBuMA) of a specific molecular weight.

Composition for forming second protective film in experiment 8-1:
P4VP-tBuMA (poly-4-vinylpyridine Mn: 74,000, poly-tert-butyl methacrylate Mn: 87,000, Mw/Mn: 1.16, manufactured by Polymer Source), final concentration 5.72 wt%
PEGDGE (poly(ethylene glycol) diglycidyl ether, Mn: approx.. 1000, manufactured by Sigma-Aldrich), final concentration 0.91 wt%

Composition for forming second protective film in experiment 8-2:
P4VP-tBuMA (poly-4-vinylpyridine Mn: 120,000, poly-tert-butyl methacrylate Mn: 270,000, Mw/Mn: 1.15, manufactured by Polymer Source), final concentration 5.72 wt%
PEGDGE (poly(ethylene glycol) diglycidyl ether, Mn: approx.. 1000, manufactured by Sigma-Aldrich), final concentration 2.0 wt%

The first protective film 18aa in the working electrode second opening 403a of the second insulating layer 4 was coated with 0.65 mg of the composition for forming a second protective film of experiment 8-1 or experiment 8-2, and this coating was dried to form the second protective film 18ab of experiment 8-1 or experiment 8-2.

The sensor 400 having the second protective film 18ab of experiment 8-1 was used as the sensor in experiment 8-1.

The sensor 400 having the second protective film 18ab of experiment 8-2 was used as the sensor in experiment 8-2.

As a liquid sample containing an analyte, RPMI-1640 Medium (R1383, manufactured by Sigma-Aldrich), glucose ( FUJIFILM Wako Pure Chemical Industries), and sodium lactic acid (manufactured by Sigma-Aldrich) were used to prepare an RPMI medium containing final concentrations of 30 mM glucose and 15 mM lactic acid, with the pH adjusted to 6.5.

The sensor of experiment 8-1 or 8-2 was immersed in the liquid sample, a voltage of 100 mV was applied to the working electrode 10a relative to the reference electrode 20 (Ag/AgCl), and the current value between the working electrode 10a and the counter electrode 30 was measured continuously for about 14 days. Measurement was performed at N = 12.

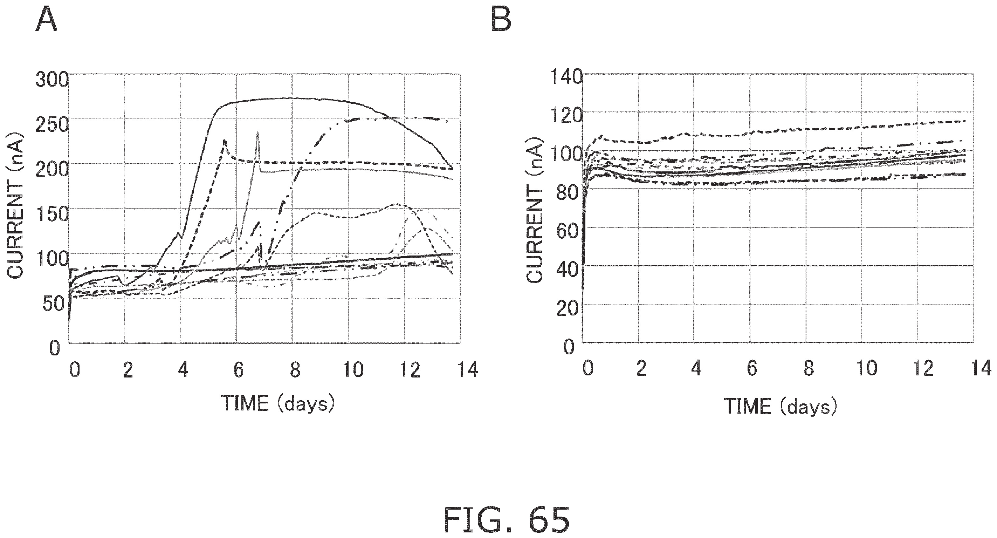

The measurement results for the current value are shown in Fig. 65. Fig. 65A shows the measurement results using the sensor of experiment 8-1, and Fig. 65B shows the measurement results using the sensor of experiment 8-2.

With some samples of the sensor in experiment 8-1, which was equipped with a second protective film 18ab formed from a composition for forming a second protective film containing P4VP-tBuMA having P4VP with Mn: 74,000 and tBuMA with Mn: 87,000, and containing PEGDGE at a final concentration of 0.91 wt%, abnormally high current values were detected after four or more days had passed since the start of measurement.

Meanwhile, with the sensor of experiment 8-2, which was equipped with a second protective film 18ab formed from a composition for forming a second protective film containing P4VP-tBuMA having P4VP with Mn: 120,000 and tBuMA with Mn: 270,000, and containing PEGDGE at a final concentration of 2.0 wt%, no abnormally high current values were detected even after four or more days had passed since the start of measurement.

This result suggests that the occurrence of abnormally high current values during long-term measurement can be suppressed by raising the ratio of tBuMA-derived units containing hydrophobic functional groups and the concentration of the crosslinking compound in the second protective film 18ab.

### Fourth Disclosure / Experiment 9

Two types of composition containing P4VP-tBuMA and PEGDGE in the following final concentrations in ethanol were designed as the composition for forming the second protective film. In the experiment shown below in relation to the fourth disclosure, the number of moles of pyridine derived from P4VP calculated on the basis of the molecular weight of the P4VP monomer and the number of moles of epoxy groups derived from PEGDGE calculated on the basis of the number average molecular weight of PEGDGE (regarded as a uniform molecular weight of PEGDGE) were used in the calculation of the ratio (mol%) of epoxy groups derived from PEGDGE to pyridine derived from P4VP.

Composition A (10.17 mol% of epoxy groups derived from PEGDGE relative to pyridine derived from 4-vinylpyridine):
- P4VP-tBuMA (poly-4-vinylpyridine Mn: 120,000, poly-tert-butyl methacrylate Mn: 270,000, Mw/Mn: 1.15, manufactured by Polymer Source), final concentration 5.72 wt%
- PEGDGE (poly(ethylene glycol) diglycidyl ether, Mn: 1000, manufactured by Sigma-Aldrich), final concentration 0.9 wt%

Composition B (6.77 mol% of epoxy groups derived from PEGDGE relative to pyridine derived from 4-vinylpyridine):
- P4VP-tBuMA (poly-4-vinylpyridine Mn: 120,000, poly-tert-butyl methacrylate Mn: 270,000, Mw/Mn: 1.15, manufactured by Polymer Source), final concentration 5.72 wt%
- PEGDGE (poly(ethylene glycol) diglycidyl ether, Mn: 1000, manufactured by Sigma-Aldrich), final concentration 0.6 wt%

A mixture containing the components of composition A in ethanol was kept at 40°C for 16 to perform pre-crosslinking and thereby prepare the second composition for forming a protective film of experiment 9-1.

A mixture containing the components of composition B in ethanol was kept at 40°C for 16 hours to perform pre-crosslinking and thereby prepare the second composition for forming a protective film of experiment 9-2.

A mixture containing the components of composition B in ethanol was kept at 53°C for 16 hours to perform pre-crosslinking and thereby prepare the second composition for forming a protective film of experiment 9-3.

The sensors of experiments 9-1, 9-2, and 9-3 were prepared by the same procedure as in experiment 8, except that the second protective film 18ab of the working electrode 10a was formed using the compositions for forming a second protective film of experiments 9-1, 9-2, and 9-3 as the compositions for forming a second protective film.

The sensor of experiment 9-1, 9-2, or 9-3 was immersed in the same liquid sample as that used in experiment 8, a voltage of 100 mV was applied to the working electrode 10a relative to the reference electrode 20 (Ag/AgCl), and the current value between the working electrode 10a and the counter electrode 30 was measured continuously for about 14 days. The measurement was performed at N = 8 in experiment 9-1, N = 12 in experiment 9-2, and N = 4 in experiment 9-3.

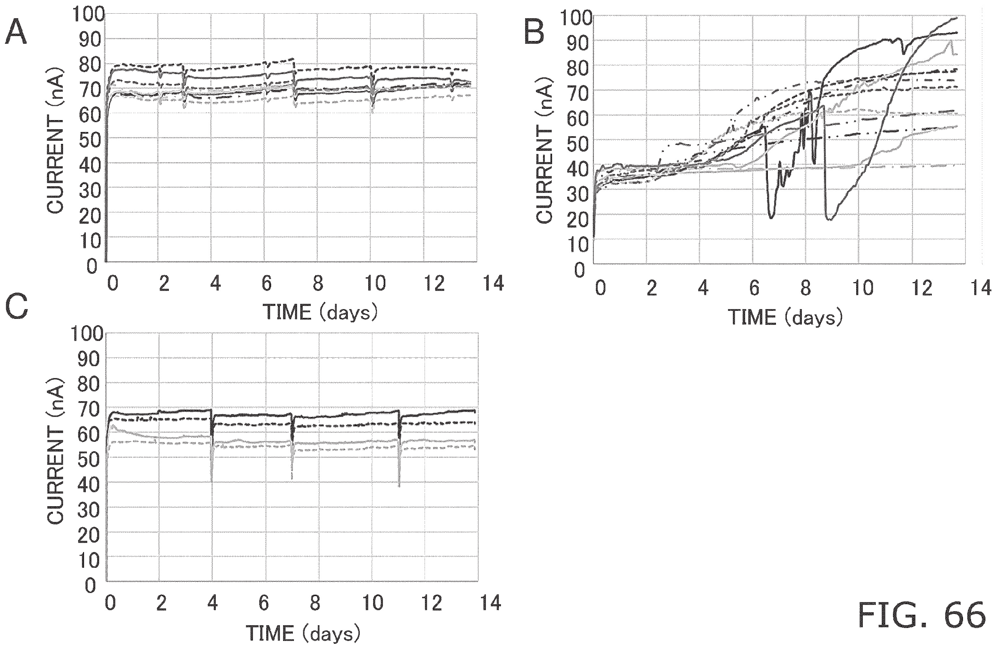

The measurement results for the current values are shown in Fig. 66. Fig. 66A shows the measurement results using the sensor of experiment 9-1, Fig. 66B shows the measurement results using the sensor of experiment 9-2, and Fig. 66C shows the measurement results using the sensor of experiment 9-3.

It was suggested that the occurrence of abnormally high current values during long-term measurement could be suppressed with a sensor in which the second protective film 18ab formed using a composition for forming a second protective film that had been pre-crosslinked by mixing P4VP-tBuMA and PEGDGE and allowing them to react for a certain length of time, was provided on the first protective film 18aa containing Nafion (registered trademark) containing a cation exchange functional group. It was also suggested that the occurrence of abnormally high current values during long-term measurement could be further suppressed by raising the temperature during pre-crosslinking.

### Fourth Disclosure / Experiment 10

Three types of composition were designed containing P4VP-tBuMA and PEGDGE in the following final concentrations in ethanol, as the composition for forming the second protective film.

Composition C (22.67 mol% of epoxy groups derived from PEGDGE relative to pyridine derived from 4-vinylpyridine):
- P4VP-tBuMA (poly-4-vinylpyridine Mn: 120,000, poly-tert-butyl methacrylate Mn: 270,000, Mw/Mn: 1.15, manufactured by Polymer Source), final concentration 5.72 wt%
- PEGDGE (poly(ethylene glycol) diglycidyl ether, Mn: 1000, manufactured by Sigma-Aldrich), final concentration 2.0 wt%

Composition D (10.17 mol% of epoxy groups derived from PEGDGE relative to pyridine derived from 4-vinylpyridine):
- P4VP-tBuMA (poly-4-vinylpyridine Mn: 120,000, poly-tert-butyl methacrylate Mn: 270,000, Mw/Mn: 1.15, manufactured by Polymer Source), final concentration 5.72 wt%
- PEGDGE (poly(ethylene glycol) diglycidyl ether, Mn: 1000, manufactured by Sigma-Aldrich), final concentration 0.9 wt%

Composition E (6.77 mol% of epoxy groups derived from PEGDGE relative to pyridine derived from 4-vinylpyridine):
- P4VP-tBuMA (poly-4-vinylpyridine Mn: 120,000, poly-tert-butyl methacrylate Mn: 270,000, Mw/Mn: 1.15, manufactured by Polymer Source), final concentration 5.72 wt%
- PEGDGE (poly(ethylene glycol) diglycidyl ether, Mn: 1000, manufactured by Sigma-Aldrich), final concentration 0.6 wt%

A mixture containing the components of composition C in ethanol was kept at room temperature (25°C ±3°C) for about 1 hour to perform pre-crosslinking and thereby prepare the second composition for forming a protective film of experiment 10-1.

A mixture containing the components of composition D in ethanol was kept at 40°C for 16 hours to perform pre-crosslinking and thereby prepare the second composition for forming a protective film of experiment 10-2.

A mixture containing the components of composition E in ethanol was kept at 53°C for 16 hours to perform pre-crosslinking and thereby prepare the second composition for forming a protective film of experiment 10-3.

The sensors of experiments 10-1, 10-2, and 10-3 were prepared by the same procedure as in experiment 8, except that the second protective film 18ab of the working electrode 10a was formed using the compositions for forming a second protective film of experiments 10-1, 10-2, and 10-3 as the composition for forming a second protective film.

Next, the sensors of experiments 10-1, 10-2, and 10-3 thus produced were each stored in the presence of a desiccant at 60°C for 14 days in order to accelerate deterioration.

After storage at 60°C for 14 days, each sensor was immersed in the same liquid sample as that used in experiment 8, and a voltage of 100 mV was applied to the working electrode 10a relative to the reference electrode 20 (Ag/AgCl), and the current value between the working electrode 10a and the counter electrode 30 was measured for 10 days in a row. The measurement was performed at N = 4. The current value 24 hours after the start of the measurement (average of N = 4) was set to 100%, and the current value measured at a later time point (average of N = 4) was expressed as a relative value.

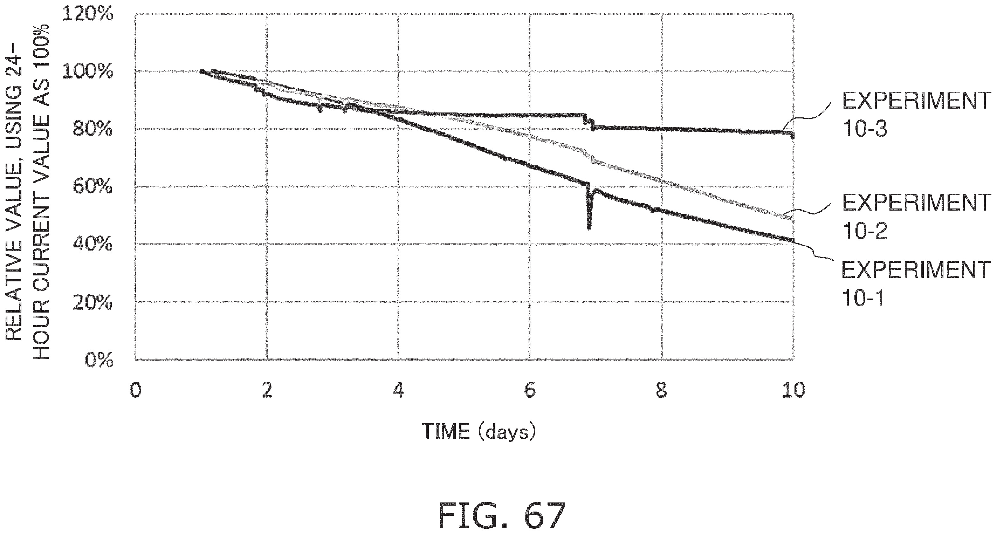

The measurement results for the current value are shown in Fig. 67.

It was confirmed that the sensors of experiments 10-2 and 10-3, in which the second protective film 18ab formed using a composition for forming a second protective film that had been pre-crosslinked by mixing P4VP-tBuMA and PEGDGE and allowing these to react for at least 6 hours, was provided on the first protective film 18aa containing Nafion (registered trademark) containing a cation exchange functional group, had less fluctuation in current value after a temperature-accelerated test than the sensor of experiment 10-1. It was also confirmed that the fluctuation in current value after a temperature-accelerated test can be further reduced by raising the temperature during pre-crosslinking. These results suggest that a sensor with excellent long-term stability can be obtained by forming the second protective film 18ab on the first protective film 18aa containing Nafion (registered trademark), using a composition for forming a second protective film that has been pre-crosslinked by mixing P4VP-tBuMA and PEGDGE and allowing these to react for at least 6 hours at a temperature of 20°C or more.

### Supplemental Note Regarding Fourth Disclosure

Sensors for electrochemically measuring an analyte in a cell culture medium or other such liquid sample, which were provided with a working electrode including a reagent layer containing a reagent that participates in an oxidation-reduction reaction, are conventionally known.

These sensors are sometimes used in applications of continuous monitoring, in which the analyte is continuously measured over an extended period of time (several days or more) while immersed in a liquid sample, and in this case there needs to be little change in responsiveness in order to allow for stable measurement during the measurement period.

In view of this, it is an object of the fourth disclosure of this specification to provide a sensor with which stable measurement with little change in responsiveness even when the sensor is immersed in a liquid sample for an extended period of time, as well as a method for manufacturing this sensor. The sensor according to the fourth disclosure can be used favorably in applications involving long-term continuous monitoring.

More specifically, this specification discloses, as a fourth disclosure, the sensor and the manufacturing method thereof described in any one or more of Supplementary Notes 4-1 to 4-10 below.

### Supplementary Note 4-1

A sensor comprising an insulating substrate and a working electrode that is disposed on the substrate,
wherein the working electrode comprises:
a working electrode conductive layer that is disposed on the substrate;
a reagent layer that is disposed on the working electrode conductive layer and includes a reagent that will participate in an oxidation-reduction reaction;
a first protective film that is disposed on the reagent layer and includes a first polymer compound having a cation exchange functional group; and
a second protective film that is disposed on the first protective film and includes a second polymer compound containing a cationic functional group.

### Supplementary Note 4-2

The sensor described in supplementary note 4-1, wherein the second polymer compound includes the first unit containing a cationic functional group and the second unit containing a hydrophobic functional group, and the ratio of the second unit to the total amount of structural units of the second polymer compound is 50 mol% or more.

### Supplementary Note 4-3

The sensor described in supplementary note 4-1 or 4-2, wherein the cationic functional group is pH independent.

### Supplementary Note 4-4

The sensor described in supplementary note 4-3, wherein the cationic functional group includes a quaternary ammonium cation derived from a reaction between a tertiary amine and an epoxy group.

### Supplementary Note 4-5

The sensor described in supplementary note 4-3, wherein the reaction is between the tertiary amine and at least 6.8 mol% of the epoxy groups relative to the tertiary amine.

### Supplementary Note 4-6

The sensor described in any of supplementary notes 4-1 to 4-5, wherein the second polymer compound is a copolymer compound of 4-vinylpyridine and tert-butyl methacrylate crosslinked by a crosslinking compound containing two or more epoxy groups.

### Supplementary Note 4-7

A method for manufacturing a sensor comprising an insulating substrate and a working electrode disposed on the substrate,
the working electrode comprising:
a working electrode conductive layer that is disposed on the substrate;
a reagent layer that disposed on the working electrode conductive layer and includes a reagent that will participate in an oxidation-reduction reaction;
a first protective film that is disposed on the reagent layer and includes a first polymer compound containing a cation exchange functional group; and
a second protective film that is disposed on the first protective film and contains a second polymer compound that includes a first unit including a quaternary ammonium cation derived from a reaction between a tertiary amine and an epoxy group, and a second unit including a hydrophobic functional group,
said method comprising:
   a step of obtaining a second composition for forming a protective film, containing the second polymer compound containing the first unit and the second unit in alcohol, by holding a mixture of an uncrosslinked polymer compound containing a third unit containing a tertiary amine and the second unit containing a hydrophobic functional group, and a crosslinking compound containing two or more epoxy groups, in alcohol at a temperature of at least 20°C for 6 hours or more, thereby allowing the tertiary amine of the third unit to react with the epoxy group of the crosslinking compound and converting the third unit into the first unit containing the quaternary ammonium cation; and
   a step of forming the second protective film containing the second polymer compound by covering the first protective film of a sensor part comprising:
      the second protective film forming composition;
      the substrate;
      the working electrode conductive layer disposed on the substrate;
      the reagent layer disposed on the working electrode conductive layer; and the first protective film disposed on the reagent layer
      with the second composition for forming a protective film, and then drying this composition.

### Supplementary Note 4-8

The method described in supplementary note 4-7, wherein the uncrosslinked polymer compound contains the third unit and the second unit such that the ratio of the second unit to the combined amount of the third unit and the second unit is at least 50 mol%.

### Supplementary Note 4-9

The method described in supplementary note 4-7 or 4-8, wherein the mixture contains the uncrosslinked polymer compound and the crosslinking compound such that the amount of epoxy groups of the crosslinking compound is at least 6.8 mol% relative to the tertiary amine of the third unit.

### Supplementary Note 4-10

The method described in any of supplementary notes 4-7 to 4-9, wherein the uncrosslinked polymer compound is a copolymer compound of 4-vinylpyridine and tert-butyl methacrylate.

### REFERENCE SIGNS LIST

1 sensor
2 substrate
3, 3a and 3b first insulating layer
3a1, 3b1 first opening
3a10, 3b10 inner peripheral edge of first opening
3a2, 3b2 water-repellent surface
3a3, 3b3 portion of first insulating layer that entirely encompasses first opening
4, 4a and 4b second insulating layer
4a1 and 4b1 second opening
4a10, 4b10 inner peripheral edge of second opening
4b2, 4b2 alcohol-repellent surface
10a and 10b working electrode
10a first working electrode
10b second working electrode
11a and 11b working electrode conductive layer
11a conductive layer of first working electrode
11b conductive layer of second working electrode
15a and 15b reagent layer
15a10, 15b10 outer peripheral edge of reagent layer
15a first reagent layer of first working electrode
15b second reagent layer of second working electrode
16a, 16b protective film
16a10, 16b10 outer peripheral edge of protective film
16a first protective film
16ba second protective film
16bb third protective film
20 reference electrode
30 counter electrode
50 wiring
C cells
T thickness direction of substrate
X liquid sample
A liquid composition containing reagent participating in oxidation-reduction reaction in water
B liquid composition containing protective film components in alcohol
1101, 1101 ', 1101" sensor
1200 insulating substrate
1201 distal end portion of substrate
1202 first surface of substrate
1210 main body portion
1211 first end portion of main body portion
1220 connecting portion
1222 second end of connecting portion
1223 third end of connecting portion
1225 bending part
1230 proximal end portion
1234 fourth end of proximal end portion
1300 detection electrode
1310 working electrode
1311 working electrode conductive layer
1315 reagent layer
1316 working electrode protective film
1320 reference electrode
1321 reference electrode conductive layer
1322 silver/silver chloride layer
1326 reference electrode protective film
1330 counter electrode
1331 upper surface of counter electrode
1501 working electrode opening
1502 reference electrode opening
1503 counter electrode opening
1050 insulating layer
1051 first insulating layer
1052, 1052a, 1052b, 1052c second insulating layer
1055 non-insulating region
1061 distal end opening
1062 flow path
1062A extension
1005 wiring
1006 terminal
900 sensor unit
957 lower support plate (support member)
957d first engagement portion
959 upper support plate (support member)
959a second engagement portion
T1: thickness direction of substrate
X1 liquid sample
300, 400 sensor
60 counter electrode
60a Portion of counter electrode into contact with liquid sample
11a, 11b working electrode conductive layer
11a1, 11b1 Portion of working electrode conductive layer in contact with liquid sample
18a protective film
18aa first protective film
18ab second protective film

It follows a list of further embodiments of the application:
Embodiment 1. A sensor comprising an insulating substrate and a working electrode disposed on the substrate,
   wherein the working electrode comprises:
   a conductive layer that is disposed on the substrate;
   a first insulating layer that is at least partially disposed on the conductive layer and that has a water-repellent surface and a first opening formed at a position overlapping part of the conductive layer in a plan view in the thickness direction of the substrate, the first opening passing through in the thickness direction;
   a second insulating layer that is disposed on the first insulating layer and that has a surface that is liquid-repellent to alcohol, and a second opening formed at a position overlapping the part of the first insulating layer that entirely encompasses the first opening in a plan view in the thickness direction, the second opening passing through in the thickness direction;
   a reagent layer that is disposed within the first opening of the first insulating layer, and includes an outer peripheral edge defined by the inner peripheral edge of the first opening of the first insulating layer, and a reagent that participates in an oxidation-reduction reaction; and
   a protective film that is disposed within the second opening of the second insulating layer, and includes an outer peripheral edge defined by the inner peripheral edge of the second opening of the second insulating layer.
Embodiment 2. The sensor with the features of embodiment 1,
   wherein the surface of the first insulating layer contains a fluororesin.
Embodiment 3. The sensor with the features of embodiment 1 or 2,
   wherein the surface of the second insulating layer contains a compound containing a perfluoroalkyl group.
Embodiment 4. The sensor with the features of any of embodiment 1 to 3,
   wherein a plurality of the working electrodes are provided, and
   a first reagent contained in a first reagent layer of a first working electrode included in the plurality of working electrodes, is different from a second reagent contained in a second reagent layer of a second working electrode different from the first working electrode.
Embodiment 5. The sensor with the features of embodiment 4,
   wherein the first reagent contains glucose dehydrogenase or glucose oxidase, and
   the second reagent contains lactate oxidase or lactate dehydrogenase.
Embodiment 6. The sensor with the features of any of embodiment 1 to 5,
   wherein the protective film includes a first protective film containing a polymer compound containing 4-vinylpyridine as a structural unit.
Embodiment 7. The sensor with the features of any of embodiment 1 to 6,
   wherein the protective film includes a second protective film disposed on the reagent layer and containing a polymer compound having a cation exchange functional group, and a third protective film disposed on the second protective film and containing a polymer compound having 4-vinylpyridine as a structural unit.
Embodiment 8. The sensor with the features of any of embodiment 1 to 7,
   further comprising a counter electrode and a reference electrode disposed on the substrate.
Embodiment 9. A method for manufacturing the sensor with the features of any of embodiment 1 to 8, said method comprising the steps of:
   forming a droplet of a liquid composition A containing the reagent in water in the first opening of the first insulating layer of the substrate on which the conductive layer, the first insulating layer, and the second insulating layer are disposed, and then drying this droplet to form the reagent layer; and
   after the reagent layer is formed, forming a droplet of a liquid composition B containing a protective film component in alcohol in the second opening of the second insulating layer, and then drying this droplet to form the protective film.
Embodiment 10. The method with the features of embodiment 9,
   wherein the surface of the first insulating layer contains a fluororesin.
Embodiment 11. The method with the features of embodiment 9 or 10,
   wherein the surface of the second insulating layer contains a compound containing a perfluoroalkyl group.
Embodiment 12. The method with the features of any of embodiment 9 to 11,
   wherein the sensor comprises a plurality of the working electrodes, and
   the formation of the reagent layer includes:
      forming a first reagent layer of a first working electrode included in the plurality of working electrodes by forming a droplet of a first liquid composition A containing a first reagent in water in the first opening of the first insulating layer of the first working electrode, and then drying this droplet, and
      forming a second reagent layer of a second working electrode, which is different from the first working electrode included in the plurality of working electrodes, in the first opening of the first insulating layer of the second working electrode by forming a droplet of a second liquid composition A containing a second reagent different from the first reagent in water and then drying this droplet.
Embodiment 13. The method with the features of embodiment 12,
   wherein the first reagent contains glucose dehydrogenase or glucose oxidase, and
   the second reagent contains lactate oxidase or lactate dehydrogenase.
Embodiment 14. The method with the features of any of embodiment 9 to 13,
   wherein the protective film includes a first protective film disposed on the reagent layer and containing a polymer compound containing 4-vinylpyridine as a structural unit, and
   the formation of the protective film includes:
      after the reagent layer is formed, forming the first protective film in the second opening of the second insulating layer by forming a droplet of a first liquid composition B containing a polymer compound containing 4-vinylpyridine as a structural unit in alcohol, and then drying this droplet.
Embodiment 15. The method with the features of any of embodiment 9 to 14,
   wherein the protective film includes a second protective film disposed on the reagent layer and including a polymer compound containing a cation exchange functional group, and a third protective film disposed on the second protective film and containing a polymer compound containing 4-vinylpyridine as a structural unit, and
   the formation of the protective film includes:
      after the reagent layer is formed, forming the second protective film in the second opening of the second insulating layer by forming a droplet of a second liquid composition B containing a polymer compound containing the cation exchange functional group in alcohol, and then drying this droplet, and
      after the second protective film is formed, forming the third protective film in the second opening of the second insulating layer of the second working electrode by forming a droplet of a third liquid composition B containing a polymer compound containing 4-vinylpyridine as a structural unit in alcohol, and then drying this droplet.

## Claims

1. A sensor that is used in a state of being immersed in a liquid sample, comprising:
an insulating substrate including a distal end portion that is immersed in the liquid sample;
detection electrodes disposed near the distal end portion on a first surface of the substrate, the detection electrodes including a working electrode, a counter electrode, and a reference electrode;
wiring that is disposed on the first surface of the substrate and is connected to the detection electrodes;
an insulating layer that is disposed on the first surface of the substrate and is formed so as to cover at least part of the detection electrodes and the wiring;
a distal end opening that is formed in the insulating layer, communicates with an upper surface of the counter electrode, and opens to the side of the substrate with the distal end portion; and
a flow path that is formed in the insulating layer, connects the distal end opening and the upper surface of the counter electrode, and guides the liquid sample from the distal end opening to the upper surface of the counter electrode.

2. The sensor according to claim 1, wherein:
the insulating layer comprises a counter electrode opening that is formed at a position overlapping the counter electrode in plan view from the thickness direction of the substrate, and goes through in the thickness direction, and
the upper surface of the counter electrode is exposed through the counter electrode opening.

3. The sensor according to claim 1 or 2, wherein:
the insulating layer comprises, in plan view from the thickness direction of the substrate:
a working electrode opening that is formed at a position overlapping the working electrode and goes through in the thickness direction; and
a reference electrode opening that is formed at a position overlapping the reference electrode and goes through in the thickness direction,
the working electrode includes a working electrode protective film disposed in the working electrode opening, and
the reference electrode includes a reference electrode protective film disposed in the reference electrode opening.

4. The sensor according to any of claims 1 to 3, wherein:
the insulating layer includes:
a first insulating layer that is disposed on the first surface of the substrate and contains an insulating cured resin; and
a second insulating layer that is disposed on the first insulating layer and includes an insulating sheet, and
the distal end opening and the flow path are formed in the second insulating layer.

5. The sensor according to claim 4,
wherein the distal end opening and the flow path are further formed in the first insulating layer.

6. The sensor according to any of claims 1 to 5, wherein the flow path has an expansion section where the width of the flow path expands toward the distal end opening.

7. The sensor according to any of claims 1 to 6, wherein:
the substrate includes:
a main body portion that includes the distal end portion and a first end portion on the opposite side from the distal end portion, and on which the detection electrodes are disposed;
a connecting portion that includes a second end connected to the first end of the main body portion, and a third end on the opposite side from the second end, and that extends from the second end to the third end; and
a proximal end portion that includes a fourth end connected to the third end of the connecting portion, and
the connecting portion of the substrate includes a bending portion near the third end portion, at which |the substrate is bent so that the first surface side protrudes.

8. The sensor according to claim 7,
wherein the insulating layer includes a first insulating sheet that is disposed above the region of the first surface of the substrate having the portions of the main body portion and the connecting portion that are closer to the second end portion than the bending portion.

9. The sensor according to claim 8,
wherein the insulating layer has a non-insulating region that does not include an insulating sheet, above the region of the first surface of the substrate having the bending portion of the connecting portion.

10. The sensor according to claim 8 or 9, wherein:
the insulating layer further includes a second insulating sheet that is disposed above the region of the first surface of the substrate having the proximal end portion, and
the first insulating sheet and the second insulating sheet are separated from each other above the region of the first surface of the substrate having the bending portion of the connecting portion.

11. A sensor unit, comprising:
the sensor according to any of claims 1 to 10; and
a support member equipped with an engagement portion that engages with the substrate of the sensor and supports the sensor so that the distal end portion of the substrate is the lower end.
